# EUROPEAN PATENT APPLICATION

(11) **EP 3 607 971 A1**
(43) Date of publication of application: **12.02.2020**
(21) Application number: 18781089.0
(22) Date of filing: 16.02.2018
(51) Int. Cl.: A61K 45/00, A61K 31/7088, A61K 31/7105, A61K 31/713, A61K 35/76, A61K 38/16, A61K 48/00, A61P 25/00, A61P 35/00, A61P 43/00

(54) **FUNCTION INHIBITOR OF SWI/SNF COMPLEXES**

(30) Priority: 05.04.2017 JP 2017075506; 17.04.2017 JP 2017081615
(71) Applicant: National University Corporation Chiba University, Chiba-shi, Chiba 263-8522 (JP)
(72) Inventor: IBA Hideo, Chiba-shi Chiba 260-8673 (JP); KOBAYASHI Kazuyoshi, Chiba-shi Chiba 260-8673 (JP)
(74) Representative: Vossius & Partner Patentanwälte Rechtsanwälte mbB
(86) International application number: PCT/JP2018/005527
(87) International publication number: WO 2018/186032

(57) **Abstract**

The present invention provides an anticancer agent based on a novel mechanism. Specifically, the present invention relates to an inhibitor that inhibits gene expression regulation by SWI/SNF complex-dependent NF-κB or a corepressor complex that participate in chromatin remodeling. More specifically, the present invention provides an inhibitor that reduces the interaction (such as binding and transcription regulation) of endogenous SWI/SNF complexes with NF-κB or a corepressor complex, or an inhibitor that inhibits the adapter function of d4 family proteins. The inhibitor of the present invention can be utilized in the treatment of cancers involving SWI/SNF complexes (such as SWI/SNF-mediated cancers).

## Description

### Technical Field

The present invention relates to an inhibitor which inhibits the regulation of gene expression by NF-κB or corepressor complex depending on SWI/SNF complex involved in chromatin remodeling. The inhibitor of the present invention can be utilized in the treatment of cancers involving the SWI/SNF complex (SWI/SNF-mediated cancers, etc.).

### Background Art

The genomic DNA of eukaryotic multicellular organisms is wrapped around a histone octamer to construct a compact chromatin structure. The transcription, replication, repair, etc. of genomic gene requires loosening up this compact chromatin structure so that factors such as transcription factors can directly bind to the genomic DNA. One of the mechanisms responsible for such epigenetic regulation is the conversion of the chromatin structure. SWI/SNF complex has been found as an evolutionarily conserved representative factor having such activity. In recent years, mutations or deletions have been found in genes of subunits constituting this SWI/SNF complex in human cancers, and the involvement of the SWI/SNF complex in tumorigenesis has received attention (Patent Literatures 1 and 2). However, the SWI/SNF complex is responsible for the transcriptional regulation of a very wide range of gene groups with respect to each type of cell and therefore has the limited possibility of application in the treatment of particular diseases such as cancers.

A transcription factor NF-κB has been found as a component involved in the transcription of an immunoglobulin κ chain in B cells. This NF-κB is now known to regulate a large number of vital phenomena including development, differentiation, cellular growth and apoptosis, etc. through the activation of the expression of cytokine growth factors, adhesion molecules and the like in a wide range of cells. Not only is the constitutive or excessive activity of NF-κB found in inflammatory diseases or autoimmune diseases, but constitutive activation is also observed in many tumor cells or leukemia cells. Therefore, inhibitors thereof have also been prepared (Patent Literatures 3 to 5). However, the NF-κB inhibitors have the limited possibility of application in the treatment of diseases from the viewpoint of adverse reactions because NF-κB has a very wide range of biological effects. The present inventors have previously reported that d4 family proteins (DPF1, DPF2 and DPF3a/b) function as adaptor proteins linking NF-κB with SWI/SNF complex (Non Patent Literatures 2 and 3). However, a target gene group that undergoes transcriptional activation by a larger complex formed by NF-κB and SWI/SNF complex, or a mechanism underlying this activation, etc. has not yet been elucidated.

Tailless (also called TLX or NR2E1) belonging to the nuclear receptor family is a transcriptional regulator that is expressed in the ends of fruit-fly embryos or mouse forebrains during the process of development. TLX is expressed in neural stem cells present in the "subventricular zone", etc. in the brain of human adults. TLX has also been shown to play an important role in neural development while participating in brain tumor derived from neural stem cells. Knockout mice of the TLX gene have been shown to have phenotypes related to the central nervous system, such as the defective limbic system. The modification of TLX functions may influence diseases of the central nervous system such as depression, anxiety, aggressive diseases, stroke, multiple sclerosis, Alzheimer's disease, Parkinson's disease, neuropathic pain, central nervous system inflammatory diseases and other neurodegenerative diseases, as well as eye defects and the development of the central nervous system (Patent Literature 6).

Glioma initiating cells (GICs) are considered to cause the refractoriness of glioblastoma, lethal primary brain tumor, in human adults and tumor recurrence. The stem cell-like properties of GICs, for example, the ability to self-renewal and tumorigenicity, are epigenetically regulated. However, the details of the involvement of factors such as SWI/SNF complex, which is a major human chromatin remodeling complex, the role of a transcriptional regulator related thereto, etc. have not been elucidated for these cells (Non Patent Literature 4).

### Citation List

### Patent Literature

Patent Literature 1: Japanese Patent Laid-Open No. 2003-299486
Patent Literature 2: National Publication of International Patent Application No. 2015-536308
Patent Literature 3: National Publication of International Patent Application No. 2008-538285
Patent Literature 4: National Publication of International Patent Application No. 2003-521214
Patent Literature 5: International Publication No. WO 2006/132204
Patent Literature 6: National Publication of International Patent Application No. 2000-506397

### Non Patent Literature

Non Patent Literature 1: Ishizaka, A. et al., JOURNAL OF BIOLOGICAL CHEMISTRY, VOL. 287, NO. 15, pp. 11924-11933, 2012
Non Patent Literature 2: Nakamura, S. et al., "The 38th Annual Meeting of the Molecular Biology Society of Japan, The 88th Annual Meeting of the Japanese Biochemical Society, 2LBA-050, Kobe, December 2, 2015 (the proceedings were published on line on November 16, 2015) "Suppression of cancers by functional inhibition of adaptor proteins linking the SWI/SNF complex with NF-κB"
Non Patent Literature 3: Kobayashi, K. et al, "The 74th Annual Meeting of the Japanese Cancer Association, J-1183, Nagoya, October 9, 2015 (the abstracts were published (including on line publication) on September 18, 2015)" "Suppression of adaptor proteins linking the SWI/SNF complex with NF-κB dimers efficiently suppresses cancer development"
Non Patent Literature 4: Hiramatsu H. et al., "The 38th Annual Meeting of the Molecular Biology Society of Japan, The 88th Annual Meeting of the Japanese Biochemical Society, 1P-1053, Kobe, December 1, 2015 (the proceedings were published on line on November 16, 2015)" "Role of the SWI/SNF chromatin remodeling complex in the maintenance of glioma stemness".

### Summary of Invention

### Technical Problem

An object of the present invention is to provide an anticancer agent based on a novel mechanism.

### Solution to Problem

The present inventors have found that an inhibitor which inhibits the SWI/SNF complex-dependent regulation of gene expression by NF-κB or corepressor complex has anticancer activity.

More specifically, the present inventors have found that siRNA and shRNA which knock down the expression of a gene encoding a d4 family protein, particularly, the expression of a gene encoding DPF2 or DPF3a/3b, essential for the expression of a target gene of SWI/SNF complex-dependent NF-κB in epithelial cancer in which the SWI/SNF complex functions, exhibit strong anticancer activity against the epithelial cancer. The present inventors have further found that a region (designated as CT1 peptide) consisting of N-terminal 84 amino acid residues highly conserved among DPF1, DPF2 and DPF3a/3b exhibits stronger anticancer activity against epithelial cancer than that of the siRNA or the shRNA.

The present inventors have also found that the SWI/SNF complex plays an essential role in the maintenance of GIC stemness via a d4 family protein, particularly, DPF1 or DPF3a. More specifically, the present inventors have found that siRNA or shRNA which knocks down the expression of a gene encoding the d4 family, particularly, the expression of a gene encoding DPF1 or DPF3a, exhibits strong anticancer activity by suppressing the SWI/SNF complex-dependent activity of corepressor complex comprising a nuclear receptor TLX and LSD1/RCOR2.

The present invention is based on the findings described above and provides the following inhibitor, pharmaceutical composition, etc.
[1] An inhibitor which (a) inhibits the SWI/SNF complex-dependent regulation of gene expression by NF-κB or corepressor complex, or (b) inhibits an adaptor function of a d4 family protein.
[2] The inhibitor according to [1], wherein the inhibiting is the competitive inhibition of the function of the d4 family protein, or the suppression of the expression of the d4 family protein.
[3] The inhibitor according to [2], wherein the d4 family protein is at least one selected from DPF1, DPF2, DPF3a and DPF3b.
[4] The inhibitor according to any of [1] to [3], which is a dominant negative mutant to the d4 family protein.
[5] The inhibitor according to [4], wherein the dominant negative mutant is a polypeptide comprising an amino acid sequence represented by any of SEQ ID NOs: 1 to 7, or a polypeptide which comprises an amino acid sequence having 90% or higher identity to the amino acid sequence represented by any of SEQ ID NOs: 1 to 7, and has competitive inhibitory activity against the d4 family.
[6] The inhibitor according to any of [1] to [5], which binds to the SWI/SNF complex.
[7] The inhibitor according to any of [4] to [6], which binds to the NF-κB and/or the corepressor complex.
[8] The inhibitor according to any of [1] to [7], which reduces the expression of IL-6 and/or IL-8.
[9] The inhibitor according to any of [1] to [3], wherein the inhibitor is an expression vector comprising a polynucleotide encoding an amino acid sequence represented by any of SEQ ID NOs: 1 to 7, or an amino acid sequence having 90% or higher identity to the amino acid sequence represented by any of SEQ ID NOs: 1 to 7.
[10] The inhibitor according to any of [1] to [3], wherein the inhibitor is a polynucleotide that suppresses the expression of the d4 family gene.
[11] The inhibitor according to [10], wherein the polynucleotide is RNA that exerts RNAi, or an antisense oligonucleotide, the RNA or the antisense oligonucleotide that suppresses the expression of the d4 family gene.
[12] The inhibitor according to [11], wherein the RNA that suppresses the expression of the d4 family gene and exerts RNAi is siRNA or shRNA that is specific for any d4 family gene of SEQ ID NOs: 74 to 77 and suppresses the expression of the d4 family gene.
[13] The inhibitor according to [11] or [12], wherein the polynucleotide that suppresses the expression of the d4 family gene is siRNA or shRNA selected from double-stranded RNA consisting of a sequence selected from the group consisting of SEQ ID NOs: 41 to 73 and a complementary sequence of the sequence, and double-stranded RNA comprising a sequence comprising deletion, substitution, insertion and/or addition of one to several nucleotides for the sequence and a complementary sequence thereof.
[14] The inhibitor according to any of [11] to [13], wherein the RNA that inhibits the expression of the d4 family gene is siRNA comprising annealed polynucleotides of a set selected from a combination of the sequence of SEQ ID NO: 41 and the sequence of SEQ ID NO: 42, a combination of the sequence of SEQ ID NO: 43 and the sequence of SEQ ID NO: 44, a combination of the sequence of SEQ ID NO: 45 and the sequence of SEQ ID NO: 46, a combination of the sequence of SEQ ID NO: 47 and the sequence of SEQ ID NO: 48, a combination of the sequence of SEQ ID NO: 49 and the sequence of SEQ ID NO: 50, a combination of the sequence of SEQ ID NO: 51 and the sequence of SEQ ID NO: 52, a combination of the sequence of SEQ ID NO: 60 and the sequence of SEQ ID NO: 61, a combination of the sequence of SEQ ID NO: 62 and the sequence of SEQ ID NO: 63, a combination of the sequence of SEQ ID NO: 64 and the sequence of SEQ ID NO: 65, a combination of the sequence of SEQ ID NO: 66 and the sequence of SEQ ID NO: 67, and a combination of the sequence of SEQ ID NO: 68 and the sequence of SEQ ID NO: 69 or shRNA comprising the sequence of the siRNA and a loop sequence.
[15] The inhibitor according to [11], wherein the inhibitor is shRNA comprising a polynucleotide having any sequence selected from SEQ ID NOs: 16 to 33.
[16] A pharmaceutical composition comprising an inhibitor according to any of [1] to [15].
[17] The pharmaceutical composition according to [16], which is an anticancer agent.
[18] The pharmaceutical composition according to [16], which is a therapeutic agent for epithelial cancer.
[19] The pharmaceutical composition according to [16], which is a therapeutic agent for glioblastoma.
[20] The pharmaceutical composition according to [19], which comprises a polynucleotide that suppresses the expression of DPF1 gene as the inhibitor.

### Effects of Invention

The present invention can provide an anticancer agent based on a novel mechanism by inhibiting/controlling a particular function depending on SWI/SNF complex responsible for chromatin remodeling. More specifically, the present invention can provide a pharmaceutical composition comprising a d4 family protein-derived dominant negative mutant (CT1, etc.), or a polynucleotide inhibitor (siRNA, shRNA, etc.) that suppresses the expression of a gene encoding a d4 family protein, for epithelial cancer in which the SWI/SNF complex functions. The present invention can also provide a pharmaceutical composition comprising a polynucleotide inhibitor (siRNA, shRNA, etc.) that suppresses the expression of a gene encoding a d4 family protein, for glioma.

### Brief Description of Drawings

[Figure 1] Figure 1 shows the effect of the knockdown of respective genes encoding d4 family proteins, Brm and BRG1 on the anchorage-independent growth of HeLaS3 (A) and A549 (B).
[Figure 2-1] Figure 2 shows the structures and amino acid sequences of d4 family proteins and their dominant negative mutants. Figure 2(A) shows a schematic diagram of the structures of the d4 family proteins and their C-terminally truncated mutants CT1. Figure 2(B) shows the alignment of the amino acid sequences corresponding to the N-terminal regions CT1s of DPF1, DPF2 and DPF3. The box depicts nuclear localization signal sequences.
[Figure 2-2] Figures 2(C) and 2(D) show the effect of the ectopic expression of Halo-DPF2-CT1 and Halo-DPF3-CT1 on the cell growth of A549 cells and HeLaS3 cells in monolayer culture (C) and in soft agar (D). Figure 2(E) shows results of analyzing the expression of a Halo-tagged protein introduced in A549 cells in monolayer culture by Western blot using an anti-Halo antibody.
[Figure 3-1] Figure 3(A) shows the results of measuring, by RT-PCR, the effect of the ectopic expression of Halo-DPF2-CT1 and Halo-DPF3-CT1 on the mRNA expression levels of NF-κB target genes, IL-6, IL-8, TNF-α and ICAM1, induced by TNF-α stimulation for 1 hour in A549 cells and HeLaS3 cells. Figure 3(B) shows the results of performing TNF-α stimulation for 30 minutes, 60 minutes, 90 minutes and 120 minutes in the same way as in Figure 3(A).
[Figure 3-2] Figure 3(C) shows the results of extracting RNA from cultures maintained under TNF-α-untreated conditions, and measuring the mRNA levels of the four genes described above in the same way as in Figure 3(A).
[Figure 4-1] Figures 4(A) and 4(B) show the results of functionally analyzing a HA-tagged dominant negative mutant HA-DPF3-CT1. The figures show the effect of ectopically expressed HA-DPF3-CT1 on the cell growth of A549 cells and HeLaS3 cells in monolayer culture (A) and in soft agar (B).
[Figure 4-2] Figures 4(C) and 4(D) show the expression of HA-DPF3-CT1 under the same conditions (C) and the results of co-immunoprecipitation experiments of catalytic subunits of SWI/SNF complex with HA-DPF3-CT1, HA-DPF3a and HA-DPF3b (D).
[Figure 5] Figure 5 shows the ectopic expressivity and biological activity of HA-tagged truncation mutants derived from DPF3-CT1 in A549 cells.
[Figure 6] Figure 6 shows the effect of the ectopic expression of HA-DPF3-CT1 on tumorigenicity in mouse xenograft models using A549 cells.
[Figure 7] Figure 7 shows a schematic diagram of the interaction among SWI/SNF complex, d4 family proteins, and NF-κB.
[Figure 8-1] Figure 8(A) shows the results of measuring, by RT-PCR, change in the expression of mRNA encoding subunits of SWI/SNF complex and proteins strongly binding to the complex in 3 types of GICs derived from different patients and corresponding differentiated cells induced therefrom.
[Figure 8-2] Figure 8(B) shows the bar graphs of the results for DPF1, DPF2, DPF3a, DPF3b, BRG1 and Brm mRNAs among the results of Figure 8(A).
[Figure 9-1] Figure 9(A) shows the effect of the knockdown of d4 family proteins, BRG1 and Brm on the sphere forming activity of TGS-01 cells.
[Figure 9-2] Figure 9(B) shows that the effect of the gene knockdown on the sphere forming activity in Figure 9(A) was rescued by the cDNA coexpression of BRG1, DPF1 and DPF3a.
[Figure 9-3] Figure 9(C) shows that TGS-01 cells ectopically expressing DPF3a were sensitive to DPF1 knockdown, whereas TGS-01 cells ectopically expressing DPF1 was resistant to DPF3 knockdown.
[Figure 10-1] Figures 10(A) and 10(B) show the effect of the knockdown of BRG1 or Brm on the sphere forming activities of TGS-04 cells (A) and TGS-05 cells (B).
[Figure 10-2] Figure 10(C) shows that the effect of the knockdown of BRG in TGS-01 cells was partially rescued by the cDNA coexpression of Brm.
[Figure 11] Figure 11 shows Kaplan-Meier survival curves of nude mice orthotopically transplanted with TGS-01 cells.
[Figure 12-1] Figures 12(A) and 12(B) show the results of detecting larger SWI/SNF complex comprising TLX and LSD1/RCOR2 in TGS-01 cells by the Western blot of immunoprecipitates.
[Figure 12-2] Figures 12(C) and 12(D) show the results of detecting larger SWI/SNF complex by the Western blot of immunoprecipitates using lysates of TGS-01 cells ectopically expressing FLAG-tagged DPF1, DPF2 or DPF3a.
[Figure 13] Figure 13 shows the results of detecting proximal localization among SWI/SNF complex, TLX and LSD1/RCOR2 in TGS-01 cells by PLA method.
[Figure 14] Figure 14 shows the results of detecting, by PLA, change in signals (the number of dots) of SWI/SNF complex and TLX (or LSD1/RCOR2) proximally located in TGS-01 cells by DPF1 knockdown.
[Figure 15] Figure 15 shows a schematic diagram of the interaction among SWI/SNF complex, d4 family proteins, and corepressor complex comprising TLX in GICs.
[Figure 16] Figure 16 shows results of studying shRNA against d4 family genes that is suitable for the loss of GIC stemness.

### Description of Embodiments

Hereinafter, each embodiment and each term of the present invention will be described in detail. However, the embodiments described below are given for merely illustrating the present invention and are not intended to limit the present invention only to such embodiments. Specifically, the present invention can be carried out in various modes without departing from the spirit of the present invention.

The present invention provides an inhibitor which inhibits the regulation of gene expression by NF-κB, or corepressor complex comprising TLX, in a SWI/SNF complex-dependent manner. Preferably, the inhibitor of the present invention inhibits the SWI/SNF complex-dependent regulation of gene expression by NF-κB or corepressor complex comprising TLX, both *in vitro* and *in vivo.*

### (1. SWI/SNF complex)

In the present specification, the "SWI/SNF complex" refers to a complex that has a molecular weight of approximately 2 MDa and is constituted by multiple proteins and. In the present specification, specific examples of the SWI/SNF complex typically mean a complex comprising human or non-human mammalian Brm (GenBank accession No. X72889) or BRG1 (GenBank accession No. U29175) as a DNA-dependent ATPase subunit. The SWI/SNF complex may also include other human or mammalian homologs of yeast SWI/SNF or Drosophila Brahma.

The SWI/SNF complex in a mammal such as a human is broadly classified into two types: one comprising Brm and one comprising BRG1. In the case of a mammal, examples of other constituents of the SWI/SNF complex include, but are not limited to, INI1, BAF250, BAF60a, BAF60b, BAF60c, BAF170, BAF57, BAF155, BAF53 and β-actin (Vignali, M. et al., (2000) Mol. Cell. Biol. 20, 1899-1910). One example of factors constituting the SWI/SNF complex will be shown below.

### [Table 1]

**Table 1 Components of SWI/SNF Complex**

| | organism | | | | | |
|---|---|---|---|---|---|---|
| | *Saccharomyces cerevisiae* | | *Dorsophila melanogaster* | | *Homo sapiens* | |
| **Complex** | **Swi/SNF** | **RSC** | **BAP** | **PBAP** | **BAF** | **PBAF** |
| **ATPase domain** | Swi2/Snf2 | SthI | **BRM, Bhrama** | | BRG1 or hBRM | BRG1 |
| **Core** | Swi3 | Rsc8/Swh3 | **MOIRA, BAP155** | | **BAF155, BAF170** | |
| **Subunit** | Snf5 | SfhI | **BAP45(SNR1)** | | **SNF5 (INI1/BAF47)** | |
| | SwiI/Adr6 | | OSA | | BAF250a,b | |
| | | Rsc9 | | BAP170 | | BAF200 |
| | | RscI,2,4 | | Polybromo | | BAF180 |
| | Swp73 | Rsc6 | BAP60 | | BAF60 a,b,c | |
| | | | **BAP111** | | | **BAF57** |
| | | | | | | **BAF45** |
| | | | | | **a,b,c,d** | |
| | Arp7,9 | | **Actin** | | **beta Actin** | |
| **Accessory Subunit** | | | **BAP55/BAP47** | | **BAF53 a,b** | |
| | | | | | | BRD7 |
| | Swp82 | | | | | |
| | Snf6 | | | | | |
| | SnfII | | | | | |
| | TafI4 | | | | | |
| | | Rsc3-5,7 | | | | |
| | | Rsc10,30 | | | | |
| | | HtlI | | | | |
| | | Ldb7 | | | | |
| | | RttIO2 | | | | |

(excerpts from "Journal of Kyoto Prefectural University of Medicine 124 (12), 825-838, 2015" with partial modifications)

The SWI/SNF complex has a chromatin remodeling function via DNA-dependent ATPase. The chromatin remodeling refers to the process of changing the chromatin structure of DNA. The chromatin structure typically refers to a structure that is composed mainly of DNA and histone and has, as a basic unit structure, folded and compressed nucleosome composed of 165-bp (approximately two turns) DNA wrapping around a histone octamer formed by 4 types of histone molecules (H2A, H2B, H3 and H4). This highly folded DNA interferes with transcription, replication, repair, recombination, and the like. Accordingly, the SWI/SNF complex has the function of changing the chromatin structure by transferring and/or removing the nucleosome through the use of the energy of ATP hydrolysis. In this respect, the SWI/SNF complex plays an important role in gene expression and/or regulation together with histone modifying enzymes (histone acetylase, deacetylase, kinase, methylase, etc.). For example, when DNA is transcribed into mRNA, the SWI/SNF complex changes the chromatin structure by the chromatin remodeling described above and thereby exposes the target DNA sequence so that the DNA becomes accessible to a transcriptional regulator or the like.

In the present specification, the "SWI/SNF complex-dependent regulation of transcription by NF-κB or corepressor complex" mean that, in one embodiment, the SWI/SNF complex, a d4 family protein, and NF-κB (usually constituted by a dimer of NF-κB family protein subunits) or corepressor complex comprising TLX in cells form a large complex and thereby exert transcriptional regulation activity.

It is known in the art that the SWI/SNF complex can bind to various transcriptional regulators via core units or subunits. For example, it is known that: glucocorticoid receptor and estrogen receptor bind to BRG1 (Peterson, C.L. et al., (2000) Curr. Opin. Gen. Dev. 10, 187-192); c-Myc binds to Ini1 (Yudkovsky, N. et al., (1999). Genes Dev. 13, 2369-2374); and c-Fos and c-Jun bind to BAF60a (Ito, T. et al., (2001) J. Biol. Chem. 276: 2852-2857). Also, d4 family proteins such as DPF3a/b and DPF2 have been reported to function as adaptors and bind to NF-κB (Rel A/p50 and Rel B/p52) (Tando, T et al., J. Biol. Chem. 285, 21951-21960 (2010); and Non Patent Literatures 1 to 3). The present invention has newly revealed that the SWI/SNF complex is bound to corepressor complex comprising TLX with a d4 family protein as an adaptor. The SWI/SNF complex-dependent regulation of transcription according to the present invention is particularly directed to the regulation of transcription by a large complex consisting of a d4 family protein, and one transcriptional regulator of NF-κB and corepressor complex comprising TLX.

Example approaches of detecting change in the interaction between the SWI/SNF complex and a transcriptional regulator of interest include, but are not limited to, an approach of detecting the binding between the SWI/SNF complex and the transcriptional regulator of interest, an approach of detecting the proximal location between the SWI/SNF complex and the transcriptional regulator of interest, and an approach of detecting the transcriptional activity (upregulation or downregulation) of the transcriptional regulator of interest in the presence of the SWI/SNF complex. Alternatively, such change in the interaction may be detected using various other approaches known to those skilled in the art. Specific examples of the detection approach include methods such as immunoprecipitation, density gradient centrifugation, gel shift assay, ChIP (chromatin immunoprecipitation) assay, Far-Western blot and yeast two-hybrid. Examples of the approach of detecting proximal location between the SWI/SNF complex and the transcriptional regulator of interest include Proximity Ligation Assay (PLA) (method described in Examples of the present specification), fluorescence resonance energy transfer (FRET) and bioluminescence resonance energy transfer (BRET). Examples of the approach of detecting the transcriptional activity (upregulation or downregulation) of the intended transcriptional regulator in the presence of the SWI/SNF complex include, but are not limited to, reporter assay using a reporter (luciferase, green fluorescence protein, alkaline phosphatase, etc.), quantitative RT-PCR (qRT-PCR), microarrays and Northern hybridization.

In the present specification, the suppression of the SWI/SNF complex-dependent regulation of gene expression by a transcriptional regulator also means that cells exhibit an abnormality such as growth, differentiation, anchorage independency or change in cell surface molecule, or change at various cells levels, such as growth arrest or delay, dormancy and apoptosis of host cells, are caused, as a result of change such as the coexistence of the SWI/SNF complex with the transcriptional regulator, increase or decrease in the expression level or activity of one of them, or a mutation. As described above, the SWI/SNF complex is capable of causing change in host cells via the intracellular interaction with various transcriptional regulators or the like. Mutations in subunits of the SWI/SNF complex have been found in various cancers, and the relation of the SWI/SNF complex to cancers has received attention (Wang X et al., Clin Cancer Res 2014, 20, 21-27; Biegel J.A. et al., Am J Med Genet C Semin Med Genet 2014, 166C, 350-66; Helming K.C. et al., Cancer Cell 2014, 26, 309-317; and Wilson, B.G. et al., Nat. Rev. Cancer 2011, 11: 481-492).

### (2. NF-κB)

NF-κB, also called nuclear factor κB, has been found as a transcription factor binding to an enhancer region necessary for the mature-B cell-specific expression of immunoglobulin κ light chain gene. NF-κB is constituted by a homodimer or a heterodimer formed among 5 types of Rel family proteins, RelA (p65), RelB, c-Rel, NF-κB1 (p105/p50) and NF-KB2 (p100/p52). NF-κB induces intracellular functions such as immunity, inflammation, development, cellular growth and apoptosis, and further induces the expression of many exogenous viral genomic genes. It is required for correctly maintaining these diverse vital phenomena that NF-κB should selectively activate a promoter of a target gene group. However, many of detailed mechanisms underlying this process still remains to be clarified. Accordingly, it is important to clarify, for example, a molecular mechanism that imparts promoter selectivity to each NF-κB dimer in the most downstream signal transduction. The regulation of target gene expression by NF-κB is considered to be also largely influenced by its neighboring chromatin environment.

NF-κB is related to a large number of diseases. Examples of the disease include acute and chronic inflammatory responses, Crohn disease, rheumatoid arthritis, cancers, septic shock, viral diseases caused by human immunodeficiency virus (HIV), cytomegalovirus, and the like, chronic obstructive pulmonary disease (COPD), cystic fibrosis, and neurodegenerative diseases.

In induction with inflammatory stimulation such as TNF-α or LPS, NF-κB is translocated into the cellular nucleus through the phosphorylation of IκBα by IκB kinase (IKK) complex, etc. NF-κB induces the transcription of many cytokines and growth factors (e.g., IL-2, IL-8, IFN-β, M-CSF, G-CSF and VEGF) as well as various transcription factors and signal regulators (e.g., IκBα, IRF-1 and IRF-2). In the present invention, as an index for the function of NF-κB in epithelial cancer, the ability to express or produce a protein selected from growth factors IL-6, IL-8 and TNF-α, and a cell adhesion molecule ICAM1 is used. However, other NF-κB-dependent proteins known in the art may be used as indexes. Other examples of the index include, but are not limited to, the confirmation of the ability of cells to grow, and the ability to induce apoptosis.

### (3. Corepressor complex)

In one embodiment, the present invention provides an inhibitor which inhibits the regulation of gene expression by corepressor complex. In the present invention, the corepressor complex refers to a factor belonging to cofactors. The cofactor refers to a factor that works as a bridge between a transcriptional regulator and a general transcription factor to enhance or suppress transcription, and includes coactivator complex and corepressor complex. For example, various subunits constituting the coactivator complex may include a subunit having the function of modifying histone and enhancing transcription (e.g., histone acetyltransferase: HAT), while subunits constituting the corepressor complex may include a subunit having the function of removing the modification from histone and suppressing transcription (e.g., histone deacetylase (HDAC) and histone demethylase) (Patent Literatures 1 and 2; Japanese Patent Laid-Open No. 2015-147794; Karin Meier & Alexander Brehm (2014), Epigenetics, 9:11, 1485-1495, DOI: 10.4161/15592294.2014.971580; etc.). Some corepressor complexes may comprise at least one molecule selected from TLX, RCOR2 and LSD1 (Yang et al., Stem Cells. 2011 29:791-801). Such coactivator complex or corepressor complex are capable of forming a larger complex together with the SWI/SNF complex described above.

The corepressor complex may be detected through the use of an approach known to those skilled in the art, for example, a detection approach such as Western blot, immunoprecipitation or ChIP assay using an antibody against a constituting subunit (anti-HDAC antibody, etc.), or an approach of detecting histone deacetylase activity.

The nuclear receptor family generally refers to a family of proteins that are receptors for ligands such as steroid, thyroid hormone, female hormone, male hormone, retinoic acid, retinoid, vitamin D, glucocorticoid and mineralocorticoid, and function as transcription factors in the nucleus upon binding to such ligands (Parker (1993) Curr. Op. Cell. Biol. 5: 499-504; Laudet et al., (1992) EMBO J. 11: 1003-1013; Mangelsdorf et al., 1995 Cell 83: 835-839; Loprs de Silva and Burbach, (1995) Trends Neurosci. 18: 542-548; etc.). The nuclear receptor family proteins have similar domain structures including a DNA binding domain and a ligand binding domain and are responsible for functions such as metabolism, homeostasis, differentiation, growth, development, aging and reproduction in organisms.

TLX, a member of the nuclear receptor family, is also known as tailless (Monaghan et al., 1995, Development 121: 839-853; and Pignoni et al., 1990, Cell 62: 151-163). The TLX has been found in fruit-flies (Pignoni et al., 1990), mice (Tu et al., 1994, Nature 370: 375-379; and Monaghan et al., 1995), African clawed frogs (Holleman et al., 1996, GenBank deposition No. U67886), chickens (Yu et al., 1994), humans (GenBank deposition No. Y13276; and Jackson et al., Royal Free Hospital, London), etc. TLX is an orphan receptor whose ligand has not yet been elucidated. TLX is expressed in the ends of developing fruit-fly embryos (Pignoni et al, 1990) and the developing forebrains of chickens and mice (Yu et al., 1994; and Monaghan et al., 1995). Knockout mice of the TLX gene have been shown to have phenotypes such as the defective limbic system (Monaghan et al., 1997, Nature 390: 515-517). Homozygous mutant mice are viable at birth, but have small sizes of the rhinencephalic and limbic structures including the olfactory, infrarhinal and entorhinal cortices, amygdala and dentate gyrus. The loss of TLX functions incurs the absence of all neuroblasts in the archencephalon (Younossi-Hartenstein et al., 1997, Developmental Biology 182: 270-283). Abnormal functions of TLX may cause problems with the eyes and/or the central nervous system, such as diseases of the central nervous system including depression, anxiety, aggressive diseases, stroke, multiple sclerosis, Alzheimer's disease, Parkinson's disease, neuropathic pain, central nervous system inflammatory diseases and other neurodegenerative diseases.

TLX has been reported to form a complex with a corepressor atrophin 1 (ATN1) (Zhang, CL. et al., 2006, Genes and Dev. 20: 1308-1320), to recruit histone deacetylase complex 1 (HDAC1) (Sun, GQ. et al., PNAS, 2007, 104, 39, pp. 15282-15287), and to act together with LSD1 and BCL11A (Corso-Diaz, X et al., BMC Genomics, 2016, 17: 832), for example. However, its interaction with or dependency on SWI/SNF complex has not yet been elucidated.

### (4. d4 family protein)

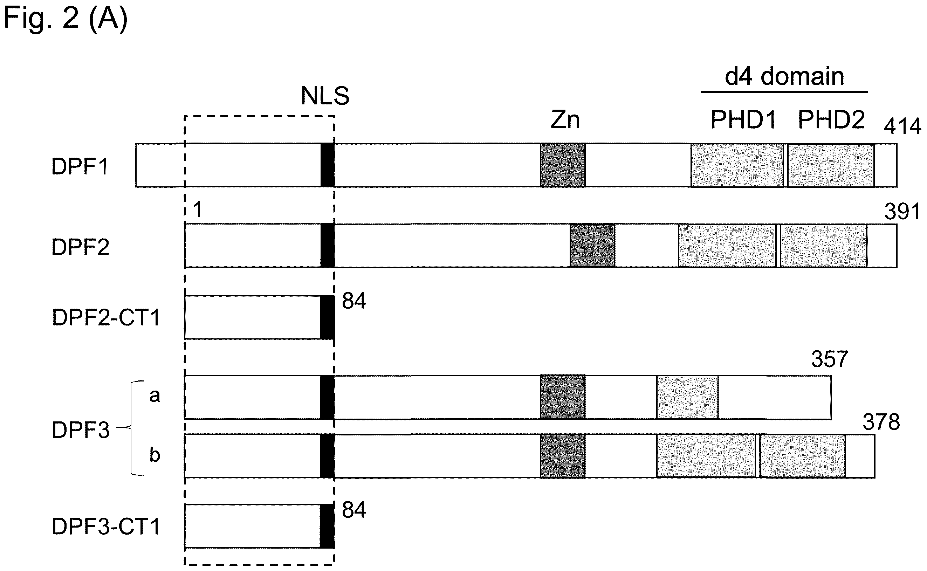

d4 family proteins are known to function as transcription cofactors. The d4 family proteins include DPF1, DPF2, DPF3a and DPF3b. These family proteins are characterized by having an N-terminal region comprising a nuclear localization signal, a central C2H2-type Kruppel-like zinc finger motif, and a C-terminal d4 domain comprising one or two PHD zinc finger motifs (PHD1 and PHD2) (Figure 2(A); Buchman, V.L. et al., 1992, Nucleic Acids Res. 20, 5579-5585).

DPF1 (also called Neud4 or BAF45b) has been reported to have human isoforms a, b, d, and the like, and the human isoform a has 414 amino acids (GenBank accession No: NP_001128627; Chestkov AV et al., Genomics 36 (1), 174-177 (1996)). A cDNA sequence corresponding thereto is Homo sapiens double PHD fingers 1 (DPF1), transcript variant 1, mRNA registered under NM_001135155 (SEQ ID NO: 74). The DPF1 and DPF3a/b described below have been reported to bind to SWI/SNF complex in a manner specific for differentiation in neurons (Lessard, J. et al., 2007, Neuron 55, 201-215).

DPF2 (also called requiem or BAF45d) has been reported to have human isoforms 1, 2, and the like, and the human isoform 1 has 391 amino acids (GenBank accession No: NP_006259.1). A cDNA sequence corresponding thereto is Homo sapiens double PHD fingers 2 (DPF2), transcript variant 1, mRNA registered under NM_006268.4 (SEQ ID NO: 75). The DPF2 and DPF3a/b described below have been reported to function as an adaptor molecule between NF-κB and SWI/SNF complex and to play an important role in the transcriptional activation of NF-κB in a great majority of downstream parts of the nonstandard pathway of NF-κB (Tando et al., 2010, J. BIOL. CHEM., 285, 29, pp. 21951-21960; and Non Patent Literature 1).

DPF3 (also called Cerd4 or BAF45c) has two splicing variants (DPF3a and DPF3b), and their products differ in C terminal side. Human DPF3a and DPF3b have 357 amino acids and 378 amino acids, respectively (GenBank accession No: NP_036206.3; Liu, H. et al., Int J Clin Exp Pathol 7 (7), 3966-3974 (2014), and GenBank accession No: Q92784.3; Lange, M. et al., 2008, Genes Dev. 22, 2370-2384). Corresponding cDNA sequences are Homo sapiens D4, zinc and double PHD fingers, family 3 (DPF3), transcript variant 1, mRNA registered under NM_012074.4 (SEQ ID NO: 76) for DPF3a, and Homo sapiens D4, zinc and double PHD fingers, family 3 (DPF3), transcript variant 2, mRNA registered under NM_001280542.1 (SEQ ID NO: 77) for DPF3b. The DPF3b protein has all the features of the other d4 family members, such as activity of binding to either methylated residues or acetylated residues of histones H3 and H4 via two PHD fingers, whereas DPF3a has one truncated PHD finger lacking the terminus of the d4 domain in the PHD domain, and thus has no function of binding to the modified histones.

### (a. Adaptor function)

In the present invention, the adaptor function means a function responsible for conferring new activity related to the interaction (binding) between SWI/SNF complex and a transcription regulation factor, or transcriptional regulation, for example. Heretofore, decrease in intracellular functional SWI/SNF complex level, such as the decreased or deleted expression of component(s) (one or more components) of SWI/SNF complex has been practiced as an approach of changing the interaction between SWI/SNF complex and a transcription regulation factor (Patent Literatures 1 and 2). However, gene groups targeted by the above-described various transcription regulation factors known to interact with SWI/SNF complex are not always regulated in a manner dependent on SWI/SNF complex, and as is known, are rather regulated by these transcription regulation factors without the SWI/SNF complex.

Thus, the present invention provides a method for inhibiting a particular function of a SWI/SNF complex-dependent particular transcription regulation factor (e.g., NF-κB and corepressor complex comprising TLX) by reducing new activity related to the interaction or transcriptional regulation of the transcription regulation factor. Examples of such a method include a method of reducing the effect of a SWI/SNF complex-dependent transcription regulation factor by inhibiting an adaptor function of a substance (protein) that functions as an adaptor between SWI/SNF complex and various transcription regulation factors. This inhibition of the adaptor function can be appropriately detected and quantified by use of an approach of detecting change in the interaction described above or particular transcriptional regulatory activity.

In the present invention, the approach of inhibiting an adaptor function between SWI/SNF complex and a particular transcription regulation factor also includes an inhibitory protein that inhibits the function. In one embodiment, the present invention provides an approach of inhibiting an adaptor function between SWI/SNF complex and a particular transcription regulation factor using a dominant negative mutant of the adaptor protein described above. In another embodiment, the present invention provides, as an approach of reducing an adaptor function, an approach of inhibiting an adaptor function between SWI/SNF complex and a particular transcription regulation factor by inhibiting the gene expression of the adaptor protein described above.

### (b. Dominant negative mutant of present invention)

The "dominant negative mutant to a d4 family protein" of the present invention is capable of competing with the binding between the d4 family protein and SWI/SNF complex and/or between the d4 family protein and a transcriptional regulator in cells, and reducing a function (transcriptional regulation, etc.) exerted by a large complex comprising the wild-type d4 family protein, the SWI/SNF complex and the transcriptional regulator. The d4 family protein has an adaptor function between SWI/SNF complex and a transcriptional regulator (NF-κB or corepressor complex) (Non Patent Literature 1, etc.). It is possible that the dominant negative mutant to the d4 family protein can be prepared, for example, by removing a site other than the SWI/SNF complex binding site of the d4 family protein and/or the transcriptional regulator binding site of the d4 family protein.

The dominant negative mutant of the present invention which "binds to SWI/SNF complex" means that this mutant, when present in the cellular nucleus, can bind to endogenous SWI/SNF complex to form a complex. The dominant negative mutant of the present invention which "binds to NF-κB and/or corepressor complex" means that this mutant, when present in the cytoplasm or the cellular nucleus, preferably in the cellular nucleus, can bind to endogenous NF-κB or corepressor complex to form a complex.

The dominant negative mutant of the present invention forms a complex with SWI/SNF complex and/or NF-κB or corepressor complex in cells, and as a result, can inhibit the SWI/SNF complex-dependent transcriptional regulation of the NF-κB or the corepressor complex. For example, in response to an external signal (TNF-α) for NF-κB activation, the transcription of a gene that is transcriptionally regulated by NF-κB in a SWI/SNF complex-dependent manner, for example, IL-6 gene and IL-8 gene, is significantly inhibited by the dominant negative mutant of the present invention, whereas the transcription of a gene that is transcriptionally regulated by NF-κB in a SWI/SNF complex-independent manner, for example TNF-α gene and ICAM1 gene, is not significantly influenced by the dominant negative mutant of the present invention.

The dominant negative mutant to the d4 family protein according to the present invention is, for example, an N-terminal partial polypeptide of the d4 family protein and comprises a nuclear localization signal (NLS) of the d4 family protein. As a more specific example, the dominant negative mutant of the present invention is a polypeptide comprising an amino acid sequence of any of SEQ ID NOs: 1 to 7 provided in the present specification, or a polypeptide comprising an amino acid sequence having 85% or higher, 90% or higher, 91% or higher, 92% or higher, 93% or higher, 94% or higher, 95% or higher, 96% or higher, 97% or higher, or 98% or higher identity to the amino acid sequence of any of SEQ ID NOs: 1 to 7, or the deletion, substitution and/or addition of one to several amino acid residues in the amino acid sequence of any of SEQ ID NOs: 1 to 7, has the ability to bind to SWI/SNF complex or a transcriptional regulator such as NF-κB or corepressor complex, and has a function that can be detected and quantified by the aforementioned approach of measuring change in the interaction, for example, a function of competing with the binding of the d4 family protein to endogenous SWI/SNF complex and/or a transcriptional regulator such as NF-κB or corepressor complex, and/or a function of changing (preferably reducing or inhibiting) the transcriptional activity of the transcriptional regulator. A higher percentage of the identity described above is more preferred. A polypeptide mutant having each identity described above may have 10% or higher, preferably 20% or higher, 30% or higher, 40% or higher, 50% or higher, 60% or higher, 70% or higher, 80% or higher, or 90% or higher specific activity of at least one of the functions described above, as compared with the original polypeptide mutant.

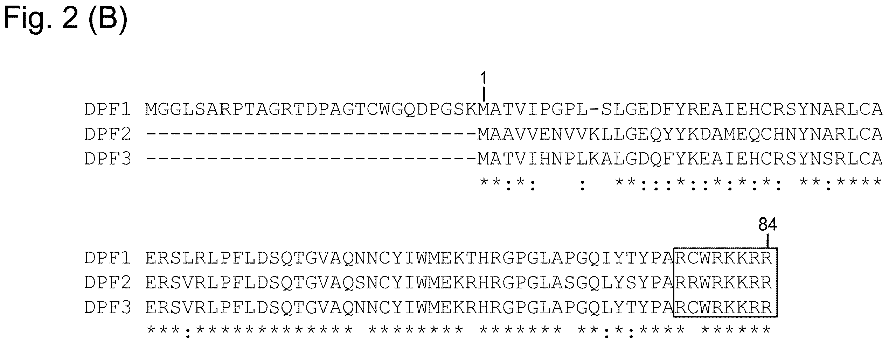

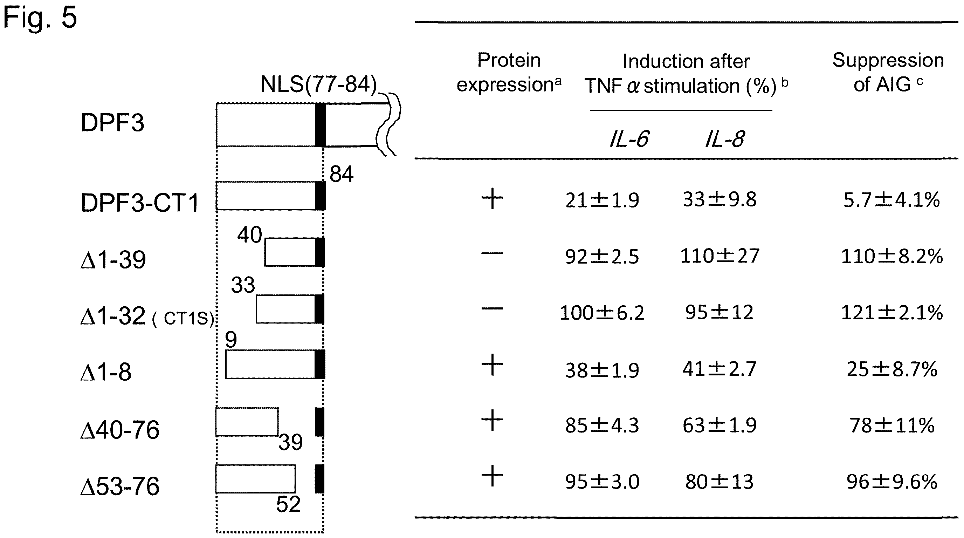

In a further embodiment of the present invention, the dominant negative mutant to the d4 family protein of the present invention may comprise only a further limited polypeptide of a functional moiety of the d4 family protein. The dominant negative mutant of the present invention may comprise, for example, a peptide in SEQ ID NO: 3, comprising a further limited moiety of the amino acid sequences of SEQ ID NOs: 1 to 3, for example, a sequence from amino acid residues 9 to 84 (SEQ ID NO: 4), a sequence from amino acid residues 33 to 84 (SEQ ID NO: 5), a sequence from amino acid residues 40 to 84 (SEQ ID NO: 6), or a sequence from amino acid residues 53 to 84 (SEQ ID NO: 7), or a peptide which comprises a SEQ ID NO: 1- or 2-derived amino acid sequence corresponding to any of these amino acid sequences, a peptide which inhibits an adaptor function between SWI/SNF complex and NF-κB, and a peptide which reduces the SWI/SNF complex-dependent transcriptional regulation function of NF-κB (see Figure 5). The corresponding portions among the amino acid sequences of SEQ ID NOs: 1 to 3 can be determined on the basis of the amino acid sequence alignment (Figure 2B, etc.) of the d4 family proteins. The dominant negative mutant of the present invention may further comprise a mutant which is a polypeptide comprising an amino acid sequence having 85% or higher, 90% or higher, 91% or higher, 92% or higher, 93% or higher, 94% or higher, 95% or higher, 96% or higher, 97% or higher, or 98% or higher identity to the amino acid sequence of the limited moiety described above, possesses an adaptor function between SWI/SNF complex and a transcriptional regulator such as NF-κB, and changes (preferably reduces or inhibits) the transcriptional activity of the transcriptional regulator.

Examples of mutually substitutable amino acid residues in the protein (polypeptide) of the present invention will be shown below. Amino acid residues included in the same group are mutually substitutable.
A Group: leucine, isoleucine, norleucine, valine, norvaline, alanine, 2-aminobutanoic acid, methionine, o-methylserine, t-butylglycine, t-butylalanine and cyclohexylalanine;
B Group: aspartic acid, glutamic acid, isoaspartic acid, isoglutamic acid, 2-aminoadipic acid and 2-aminosuberic acid;
C Group: asparagine and glutamine;
D Group: lysine, arginine, ornithine, 2,4-diaminobutanoic acid and 2,3-diaminopropionic acid;
E Group: proline, 3-hydroxyproline and 4-hydroxyproline;
F Group: serine, threonine and homoserine; and
G Group: phenylalanine and tyrosine.

In the present specification, the "protein" and the "polypeptide" are used interchangeably with each other and are meant to be a polymer of amino acids. The polypeptide used in the present specification is described such that the N terminus (amino terminus) is placed on the left and the C terminus (carboxyl terminus) is placed on the right according to the conventional peptide notation. A partial peptide of the polypeptide of the present invention (in the present specification, also referred to as the partial peptide of the present invention) is a partial peptide of the polypeptide of the present invention described above and preferably has properties similar to the polypeptide of the present invention described above.

In another embodiment, the dominant negative mutant to the d4 family protein according to the present invention may be in the form of a polynucleotide that permits intracellular expression of the polypeptide of interest. Examples of such a polynucleotide include a polynucleotide encoding a dominant negative mutant having an amino acid sequence of any of SEQ ID NOs: 1 to 3, an amino acid sequence having the predetermined identity described above to the amino acid sequence of any of SEQ ID NOs: 1 to 3, or an amino acid sequence of a further particular moiety of the amino acid sequence of any of SEQ ID NOs: 1 to 3. Examples of such a polynucleotide of the present invention can include polynucleotides of SEQ ID NOs: 10 to 13, and polynucleotides which have 80% or higher, 81% or higher, 82% or higher, 83% or higher, 84% or higher, 85% or higher, 86% or higher, 87% or higher, 88% or higher, 89% or higher, 90% or higher, 91% or higher, 92% or higher, 93% or higher, 94% or higher, 95% or higher, 96% or higher, 97% or higher, 98% or higher, 99% or higher, 99.1% or higher, 99.2% or higher, 99.3% or higher, 99.4% or higher, 99.5% or higher, 99.6% or higher, 99.7% or higher, 99.8% or higher or 99.9% or higher identity to these polynucleotides and which encode dominant negative mutants to the d4 family proteins. Such a polynucleotide of the present invention can be combined with an expression cassette and an expression vector known in the art and thereby expressible in the cell of interest.

### Nucleotide sequence of CT1 region

DPF1 > NM_001135155.2 Homo sapiens double PHD fingers 1 (DPF1), transcript variant 1, mRNA-derived (110 amino acid residues) 
ATGGGCGGCCTCAGCGCCCGCCCGACCGCTGGAGGACCGACCCGGCGG
GGACCTGCTGGGGGCAGGACCCGGGGAGCAAGATGGCCACTGTCATCCC
TGGCCCCCTGAGCCTAGGCGAGGACTTCTACCGCGAGGCCATCGAGCACT
GCCGCAGTTACAACGCGCGCCTGTGCGCCGAGCGCAGCCTGCGACTGCC
CTTCCTCGACTCGCAGACCGGCGTGGCCCAGAACAACTGCTACATCTGGA
TGGAGAAGACCCACCGCGGGCCGGGTTTGGCCCCGGGACAGATTTACAC
GTACCCCGCCCGCTGTTGGAGGAAGAAACGGAGA (SEQ ID NO: 10)
DPF2 > NM_006268.4 Homo sapiens double PHD fingers 2 (DPF2), transcript variant 1, mRNA-derived (84 amino acid residues) 
ATGGCGGCTGTGGTGGAGAATGTAGTGAAGCTCCTTGGGGAGCAGTACT
ACAAAGATGCCATGGAGCAGTGCCACAATTACAATGCTCGCCTCTGTGCT
GAGCGCAGCGTGCGCCTGCCTTTCTTGGACTCACAGACCGGAGTAGCCCA
GAGCAATTGTTACATCTGGATGGAAAAGCGACACCGGGGTCCAGGATTG
GCCTCCGGACAGCTGTACTCCTACCCTGCCCGGCGCTGGCGGAAAAAGC
GGCGA (SEQ ID NO: 11)
DPF3a > NM_012074.4 Homo sapiens double PHD fingers 3 (DPF3), transcript variant 1, mRNA (84 amino acid residues) 
ATGGCGACTGTCATTCACAACCCCCTGAAAGCGCTCGGGGACCAGTTCTA
CAAGGAAGCCATTGAGCACTGCCGGAGTTACAACTCACGGCTGTGTGCA
GAGCGCAGCGTGCGTCTTCCCTTCCTGGACTCACAGACTGGGGTGGCCCA
 
GAACAACTGCTACATCTGGATGGAGAAGAGGCACCGAGGCCCAGGCCTT
GCCCCGGGCCAGCTGTATACATACCCTGCCCGCTGCTGGCGCAAGAAGA
GACGA (SEQ ID NO: 12)
DPF3b > NM_001280542.1 Homo sapiens double PHD fingers 3 (DPF3), transcript variant 2, mRNA (an N-terminal sequence comprising the CT1 region is the same sequence as that of DPF3a) (84 amino acid residues)

ATGGCGACTGTCATTCACAACCCCCTGAAAGCGCTCGGGGACCAGTTCTA
CAAGGAAGCCATTGAGCACTGCCGGAGTTACAACTCACGGCTGTGTGCA
GAGCGCAGCGTGCGTCTTCCCTTCCTGGACTCACAGACTGGGGTGGCCCA
GAACAACTGCTACATCTGGATGGAGAAGAGGCACCGAGGCCCAGGCCTT
GCCCCGGGCCAGCTGTATACATACCCTGCCCGCTGCTGGCGCAAGAAGA
GACGA (SEQ ID NO: 13)

[Chemical 1]

Conserved region NLS

DPF1 MGGLSARPTAGRTDPAGTCWGQDPGSKMATVIPGPL-SLGEDFYREAIEHCRSYNARLCA
DPF2 ---------------------------MAAVVENVVKLLGEQYYKDAMEQCHNYNARLCA
DPF3b ---------------------------MATVIHNPLKALGDQFYKEAIEHCRSYNSRLCA
**:*:  .  :   **::*::*:*:*:.**:****

DPF1 ERSLRLPFLDSQTGVAQNNCYIWMEKTHRGPGLAPGQIYTYPARCWRKKRRLNILEDPRL
DPF2 ERSVRLPFLDSQTGVAQSNCYIWMEKRHRGPGLASGQLYSYPARRWRKKRRAHPPEDPRL
DPF3b ERSVRLPFLDSQTGVAQNNCYIWMEKRHRGPGLAPGQLYTYPARCWRKKRRLHPPEDPKL
***:*************.********  ******  **:*:****  ******  :   ***:*

A nucleotide sequence encoding a region from the N terminus of each d4 family protein to the last arginine (R) residue of NLS is shown above (SEQ ID NOs: 10 to 13). DPF1 is longer by 26 amino acid residues (78 nucleotides: the underlined nucleotides of SEQ ID NO: 10) which are not included in the conserved region. The DPF1-derived CT1 peptide may or may not comprise the 26 amino acid residues.

The present invention also encompasses a polynucleotide which hybridizes under stringent conditions to a polynucleotide consisting of a sequence complementary to the polynucleotide sequence of any of SEQ ID NOs: 10 to 13, and encodes a dominant negative mutant to the d4 family protein. In this context, the "polynucleotide which hybridizes under a stringent condition" refers to a polynucleotide (e.g., DNA) that is obtained by use of colony hybridization, plaque hybridization or Southern hybridization, etc. with the whole or a portion of the polynucleotide consisting of a sequence complementary to the polynucleotide sequence of any of SEQ ID NOs: 10 to 13 as a probe. As such a hybridization method, a method described in, for example, Molecular Cloning 3rd Ed., Current Protocols in Molecular Biology, John Wiley & Sons 1987-1997 can be utilized. The "stringent condition" may be any of low stringent conditions, moderately stringent conditions and highly stringent conditions. The "low stringent condition" is a condition involving, for example, 5 × SSC, 5 × Denhardt's solution, 0.5% SDS, 50% formamide and 32°C. The "moderately stringent condition" is a condition involving, for example, 5 × SSC, 5 × Denhardt's solution, 0.5% SDS, 50% formamide and 42°C. The "highly stringent condition" is a condition involving, for example, 5 × SSC, 5 × Denhardt's solution, 0.5% SDS, 50% formamide and 50°C. Under these conditions, it can be expected that a polynucleotide (e.g., DNA) having higher homology is efficiently obtained as the temperature is elevated. However, as a factor that influences the stringency of hybridization, a plurality of factors such as temperature, probe concentration, probe length, ionic strength, time and salt concentration are considered. Those skilled in the art can achieve similar stringency by appropriately selecting these factors.

Examples of such a polynucleotide capable of hybridizing include a polynucleotide which has a sequence having 80% or higher, 81% or higher, 82% or higher, 83% or higher, 84% or higher, 85% or higher, 86% or higher, 87% or higher, 88% or higher, 89% or higher, 90% or higher, 91% or higher, 92% or higher, 93% or higher, 94% or higher, 95% or higher, 96% or higher, 97% or higher, 98% or higher, 99% or higher, 99.1% or higher, 99.2% or higher, 99.3% or higher, 99.4% or higher, 99.5% or higher, 99.6% or higher, 99.7% or higher, 99.8% or higher or 99.9% or higher identity to the polynucleotide sequence of any of SEQ ID NOs: 10 to 13 when the identity is calculated with homology search software such as FASTA or BLAST using default parameters, and which encodes the above-described polypeptide that functions as the dominant negative mutant of the present invention.

The amino acid substitution or the nucleotide substitution appropriately utilized in the present invention can be generated by use of various genetic engineering techniques known to those skilled in the art. For such genetic engineering procedures, see, for example, Molecular Cloning 3rd Edition, J. Sambrook et al., Cold Spring Harbor Lab. Press. 2001 and Current Protocols in Molecular Biology, John Wiley & Sons 1987-1997.

The identity or homology of the amino acid sequence or the polynucleotide sequence described above can be determined using the algorithm BLAST of Karlin and Altschul (Proc. Natl. Acad. Sci. USA 872264-2268, 1990; and Proc. Natl. Acad. Sci USA 90: 5873, 1993). A program, called BLASTN or BLASTX, based on the algorithm of BLAST, has been developed (Altschul SF, et al: J Mol Biol 215: 403,1990). In a case of analyzing a nucleotide sequence using BLASTN in the invention of the present application, score = 100 and word length = 12 are used as parameters. In a case of analyzing an amino acid sequence using BLASTX, score = 50 and word length = 3 are used as parameters. In a case of using BLAST and Gapped BLAST programs, default parameters of each program are used.

In the present specification, higher sequence identity or homology described above to a particular amino acid sequence or polynucleotide sequence is more preferred. In the present specification, a polypeptide or a polynucleotide having particular identity or homology to a particular amino acid sequence or polynucleotide sequence is meant to be able to function equivalently to a polypeptide or a polynucleotide having the original sequence. In the present specification, the polypeptide or the polynucleotide that is "able to function equivalently" is meant to be a polypeptide or a polynucleotide that exhibits 10% or higher, preferably 20% or higher, 30% or higher, 40% or higher, 50% or higher, 60% or higher, 70% or higher, 80% or higher or 90% or higher specific activity of the function, as compared with the original sequence.

The term "polynucleotide" used in the present specification is used interchangeably with "nucleic acid", " gene" or "nucleic acid molecule" and is meant to be a polymer of nucleotides. The term "nucleotide sequence" used in the present specification is used interchangeably with "nucleic acid sequence" or "base sequence" and is indicated as the sequence of deoxyribonucleotides (abbreviated to A, G, C and T). For example, the "polynucleotide comprising the nucleotide sequence of SEQ ID NO: 1, or a fragment thereof" is meant to be a polynucleotide comprising a sequence represented by each deoxynucleotide A, G, C and/or T of SEQ ID NO: 1, or a fragment moiety thereof.

Each "polynucleotide" according to the present invention may exist in the form of DNA (e.g., cDNA or genomic DNA) and may be in the form of RNA (e.g., mRNA) in some cases. Each polynucleotide used in the present specification may be double-stranded or single-stranded DNA. Such single-stranded DNA or RNA may be a coding strand (also known as a sense strand) or may be a noncoding strand (also known as an antisense strand).

### (c. Polynucleotide that suppresses expression of d4 family gene according to present invention)

The polynucleotide that suppresses the expression of a gene encoding a d4 family protein (d4 family gene) according to the present invention is not particularly limited and can be appropriately selected according to a purpose from among nucleotides known in the art for use in the suppression of gene expression. Examples thereof include siRNA, shRNA, miRNA and antisense oligonucleotides that can suppress the expression of a d4 family gene. Among others, siRNA or shRNA is desirable because of its high suppressive effect on the expression of a d4 family gene. These polynucleotides can suppress the expression of a d4 family protein when introduced in the form of a nucleotide or in a form integrated into a vector known in the art in a target cell.

The "expression of a gene" used in the present specification includes a process of expressing mRNA under the regulation of a promoter sequence with genomic gene as a template (transcription), and/or a process of synthesizing a protein with this mRNA as a template (translation). Accordingly, the "inhibition of the expression of the d4 family gene" of the present invention means the inhibition of the transcription process or the translation process of a d4 family gene (preferably endogenous one).

In the present specification, the "antisense oligonucleotide that suppresses the expression of a gene" refers to an oligonucleotide having the nucleotide sequence of an antisense strand against the sense strand of the gene. The antisense technique has been known in the art as a method for suppressing the expression of a particular endogenous gene, and is described in various literatures (see e.g., Hirashima and Inoue: Shin Seikagaku Jikken Koza (New Methods of Biochemistry in English) 2, Kakusan (Nucleic Acid in English) IV, Replication and Expression of Genes (the Japanese Biochemical Society ed., Tokyo Kagaku Dojin Co., Ltd.), pp. 319-347, 1993). The sequence of the antisense oligonucleotide is preferably a sequence complementary to the endogenous gene or a portion thereof, and may not be completely complementary thereto as long as the expression of the gene can be effectively suppressed. In one embodiment, the sequence of the antisense oligonucleotide of the present invention may comprise, for example, the deletion, substitution, insertion and/or addition of one to several nucleotides in the endogenous gene or a portion thereof. The length of the antisense oligonucleotide may be 11 to 20 bases in length, 12 to 19 bases in length, 13 to 18 bases in length, 14 to 17 bases in length or 14 to 16 bases in length. In the present invention, the antisense oligonucleotide may comprise a natural (unmodified) nucleotide (deoxyribonucleotide, ribonucleotide, or both) and/or a non-natural (modified) nucleotide.

In the present specification, the "RNA that exerts RNAi and suppresses the expression of a gene" refers to RNA that suppresses the expression of the gene by RNA interference (RNAi). The "RNAi" refers to a phenomenon in which double-stranded RNA having a sequence identical or similar to a target gene sequence is introduced into cells so that the expression of the target endogenous gene or the introduced foreign gene is suppressed. In this context, examples of the RNA used include siRNA (small interfering RNA), which is double-stranded RNA of 21 to 25 bases in length that exerts RNAi, and shRNA (short hairpin RNA) which comprises a loop sequence connecting the double-stranded RNA of the siRNA, and is cleaved into the siRNA by Dicer (RNase III enzyme) in cells. The loop sequence is not particularly limited, and can be any nucleotide sequence that is usable for expressing the double-stranded RNA that exerts RNAi as single-stranded RNA that forms a hairpin-loop structure. The RNA that exerts RNAi may be locally delivered to the desired site by a delivery system such as a liposome, and can be expressed at the desired site using an expression vector that allows production of the siRNA or the shRNA as described above. A method for preparing such RNA (RNA, siRNA and shRNA), a method for using the RNA, etc. can be designed on the basis of the sequence of the gene of interest through the use of techniques known in the art described in many literatures (see National Publication of International Patent Application No. 2002-516062; U.S. Patent Publication No. 2002/086356A; Nature Genetics, 24 (2), 180-183, 2000 Feb.; Genesis, 26 (4), 240-244, 2000 April; Nature, 407: 6802, 319-20, 2002 Sep. 21; Genes & Dev., Vol. 16, (8), 948-958, 2002 Apr. 15; Proc. Natl. Acad. Sci. USA., 99 (8), 5515-5520, 2002 Apr. 16; Science, 296 (5567), 550-553, 2002 Apr. 19; Proc Natl. Acad. Sci. USA, 99: 9, 6047-6052, 2002 Apr. 30; Nature Biotechnology, Vol. 20 (5), 497-500, 2002 May; Nature Biotechnology, Vol. 20 (5), 500-505, 2002 May; Nucleic Acids Res., 30: 10, e46, 2002 May 15; etc.).

Examples of the "RNA that exerts RNAi and suppresses the expression of the d4 family gene" of the present invention (hereinafter, also referred to as the "RNA of the present invention") include siRNA or shRNA that is specific for a polynucleotide sequence according to any of SEQ ID NOs: 74 to 77 and inhibits the expression of the d4 family gene. Such RNA of the present invention can be designed on the basis of the sequence of a location specific for the d4 family gene through the use of a sequence database such as BLAST. One type of RNA of the present invention may be used alone, or two or more types thereof may be used in combination.

The RNA of the present invention may be, for example, shRNA that is specific for a polynucleotide sequence described in any of SEQ ID NOs: 74 to 77 and inhibits the expression of the d4 family gene. Examples of such RNA include shRNA consisting of at least one polynucleotide sequence selected from the group consisting of SEQ ID NOs: 14 to 40. Such shRNA is converted into siRNA by an intracellular enzyme (Dicer, etc.).

The "RNA that inhibits the expression of the d4 family gene" of the present invention may comprise, for example, any sequence selected from the group consisting of SEQ ID NOs: 41 to 73, or the deletion, substitution, insertion and/or addition of one to several nucleotides in the sequence. Alternatively, siRNA having a sequence having at least 90% or higher identity to the sequence described above may be used. When the siRNA is, for example, a duplex having a cohesive end (e.g., two nucleotides), the siRNA may comprise a moiety complementary to one strand and a noncomplementary moiety of several (e.g., two) nucleotides. Further preferably, the "RNA that inhibits the expression of the d4 family gene" of the present invention may comprise at least one siRNA comprising annealed polynucleotides of a set selected from the combination of the sequence of SEQ ID NO: 41 and the sequence of SEQ ID NO: 42, the combination of the sequence of SEQ ID NO: 43 and the sequence of SEQ ID NO: 44, the combination of the sequence of SEQ ID NO: 45 and the sequence of SEQ ID NO: 46, the combination of the sequence of SEQ ID NO: 47 and the sequence of SEQ ID NO: 48, the combination of the sequence of SEQ ID NO: 49 and the sequence of SEQ ID NO: 50, the combination of the sequence of SEQ ID NO: 51 and the sequence of SEQ ID NO: 52, the combination of the sequence of SEQ ID NO: 60 and the sequence of SEQ ID NO: 61, the combination of the sequence of SEQ ID NO: 62 and the sequence of SEQ ID NO: 63, the combination of the sequence of SEQ ID NO: 64 and the sequence of SEQ ID NO: 65, the combination of the sequence of SEQ ID NO: 66 and the sequence of SEQ ID NO: 67, and the combination of the sequence of SEQ ID NO: 68 and the sequence of SEQ ID NO: 69 or at least one shRNA comprising the sequence of the siRNA and a loop sequence. Each nucleotide sequence described above may comprise the deletion, substitution, insertion and/or addition of one to several nucleotides. Accordingly, the resulting siRNA may comprise one to several nucleotides that form no duplex.

The RNA of the present invention can further comprise, for example, a non-natural nucleotide such as peptide nucleic acid (PNA), locked nucleic acid (LNA) or fluoroarabinonucleic acid (FANA), for the purpose of improving biostability, etc. (National Publication of International Patent Application No. 2010-521973, etc.). The siRNA of the present invention may further comprise a modified nucleotide known in the art in which the nucleotide modification is selected from phosphorothioate, alkyl phosphonate, phosphorodithioate, alkyl phosphonothioate, phosphoramidate, carbamate, carbonate, phosphate triester, acetamidate, carboxy methyl ester, and combinations thereof.

### (5. Pharmaceutical composition according to present invention)

In one embodiment, the present invention provides a pharmaceutical composition for the treatment of a cancer such as epithelial cancer. In the present specification, the "epithelial cancer" refers to neoplastic epithelial cells. The epithelium refers to a cell layer that covers the outer surface of the body of an animal or the inner surface of each of coelomic organs. The epithelium has no vascular vessel, and cells assume densely packed populations. Examples of the epithelium include, but are not limited to, covering epithelium that covers the surface of the skin or the mucosa, glandular epithelium constituting parenchymal cells of the duct of the gland, ciliated epithelium and pigment epithelium. The epithelial cancer according to the present invention includes both malignant and benign ones. Examples of the epithelial cancer include squamous cell cancer, epithelioma (which refers to epithelial tumor and includes papilloma, adenoma, cyst, and the like), neuroepithelioma, epithelial malignant tumor (hepatocellular carcinoma, basal cell carcinoma, skin cancer, etc.) and intraepithelial adenocarcinoma.

In another embodiment, the present invention provides a pharmaceutical composition for the treatment of glial (glia cell) tumor (glioma), particularly, glioblastoma. Glioblastoma initiating cells (GICs) have been considered to contribute to the resistance to therapy or tumor recurrence of glioblastoma, which is a lethal and important brain tumor in human adults (Singh, S.K. et al., Nature 432, 396-401 (2004); Bao, S. et al., Nature 444, 756-760 (2006); and Chen, J. et al., Nature 488, 522-526 (2012)). The modification of a chromatin structure has been shown to be an important determinant for the maintenance of GIC stemness and the induction of differentiation thereof (Natsume, A. et al., Cancer Res 73, 4559-4570 (2013); and Safa, A.R. et al., J Biomed Res 30, (2015)). It has been shown that differentiated glioblastoma cells can be reprogrammed into spheroid-forming stem-like tumor-propagating cells by the ectopic expression of four core transcription factors POU3F2, SALL2, SOX2 and OLIG2 which were found in recent years by using gene expression data from both stem cell-like cell populations and differentiated cell populations (Suva, M. L. et al., Cell 157, 580-594 (2014)). These results have indicated reversible hierarchies as a differentiated state in glioblastoma cell populations, and demonstrated the importance of epigenetic regulation in these biological processes (Gronych, J. et al., Cell 157, 525-527 (2014)).

In one embodiment, the present invention provides a pharmaceutical composition comprising a dominant negative mutant to the d4 family protein or a polynucleotide that inhibits the expression of the d4 family gene. In the present specification, the pharmaceutical composition means a medicament composed of only a principal agent, or a pharmaceutical composition containing a principal agent and an excipient in combination. The principal agent can be combined with, if necessary, for example, an appropriate excipient described below. In the present specification, the pharmaceutical composition refers to various dosage forms. Examples of such forms include, but are not limited to, oral, parenteral (intravenous), intramuscular, oral mucous, rectal, vaginal, transdermal, transnasal, and inhalation-mediated dosage forms. The dose of the pharmaceutical composition of the present invention can be, for example, a dose within a range such as 1 to 5000 mg, preferably 10 to 1000 mg, 10 to 800 mg, 10 to 600 mg, 10 to 500 mg, 10 to 400 mg, 10 to 300 mg, 10 to 200 mg, 10 to 100 mg, 10 to 75 mg or 10 to 50 mg per day in a human adult (e.g., body weight: 60 kg). Such a daily dose may be administered in several divided portions, for example, two or more portions or three or more portions.

In another embodiment, the pharmaceutical composition of the present invention comprises a polynucleotide encoding the dominant negative mutant to the d4 family protein, or a polynucleotide encoding the polynucleotide that inhibits the expression of the d4 family. Such a polynucleotide can be designed such that the polynucleotide can be delivered into a target cell in combination with an appropriate expression cassette containing a promoter and the like, to express the inhibitor of interest. Such an expression cassette can utilize, for example, a suitable viral vector for delivery to the target cell. In general, viral vectors can introduce a gene to target cells with high efficiency and are therefore suitable for the treatment of diseases. Examples of the viral vector that may be utilized in the pharmaceutical composition of the present invention include, but are not limited to, retroviral vectors, adenoviral vectors, adeno-associated viral vectors and herpes simplex viral vectors (Miller, A.D. et al., (1991) J. Virol. 65, 2220-2224; Miyake, S. et al., (1994) Proc. Natl. Acad. Sci. USA, 91, 8802-8806; Samulski, R. J. et al., (1989) J. Virol. 63, 3822-3828; and National Publication of International Patent Application No. 2004-528836). Particularly, the introduction of the foreign gene according to the present invention is preferably performed via a retroviral vector or a herpes viral vector. The retroviral vector also includes a lentiviral vector. Examples of the retroviral vector include, but are not limited to, ecotropic viral vectors (Kitamura, T. et al., (1995) Proc. Natl. Acad. Sci. USA. 92, 9146-9150), amphotropic viral vectors, viral vectors pseudotyped with VSV-G or the like (Arai, T. et al., (1998) J. Virol. 72, 1115-1121), and lentiviral vectors such as HIV vectors (Shimada, T. et al., (1991) J. Clin. Inv. 88, 1043-1047). Examples of the herpes viral vector include neurotropic herpes viral vectors, B lymphocyte-tropic herpes viral vectors, T lymphocyte-tropic herpes viral vectors and herpes simplex virus (HSV) vectors. For example, a herpes simplex viral vector derived from HSV-1 or HSV-2 may be used as the herpes simplex viral vector. Alternatively, a vector derived from any of the following viruses: varicella zoster virus (VZV), herpes simplex virus 6 (HSV-6), Epstein-Barr virus, cytomegalovirus, HHV6, and HHV7 may be used as the viral vector. The herpes viral vector may comprise one or more inactivating mutation of a viral endogenous gene. Examples of the expression regulatory sequence to be combined with the polynucleotide encoding the inhibitor of the invention of the present application include an endogenous promoter of a target cancer cell, and an exogenous promoter and/or enhancer of a virus or the like. Specific examples of the viral promoter include LTR of retrovirus (including viruses such as MuLV, MMTV, RSV, HIV and FIV), SV40 promoter, CMV promoter, and HPV promoter. Examples of the endogenous promoter of a host include EF1α promoter and β-globin promoter. In the case of administering the medicament of the present invention comprising such a vector, the dose can be, for example, 1 to 5000 mg, preferably 10 to 1000 mg, more preferably 10 to 800 mg, 10 to 600 mg, 10 to 500 mg, 10 to 400 mg, 10 to 300 mg, 10 to 200 mg, 10 to 100 mg, 10 to 75 mg or 10 to 50 mg, per day in a human adult (e.g., body weight: 60 kg). Such a daily dose may be administered in several divided portions, for example, two or more portions or three or more portions.

In the case of administering the pharmaceutical composition of the present invention, for example, oral, parenteral (intravenous), intramuscular, oral mucous, rectal, vaginal, transdermal, transnasal, or inhalation-mediated administration can be performed. Preferably, the pharmaceutical composition of the present invention is parenterally administered. Intravenous administration is further preferred. The pharmaceutical composition of the present invention may be formulated using each active ingredient alone or a combination of active ingredients, and can also be provided in the form of a preparation supplemented with a pharmaceutically acceptable carrier or a pharmaceutical additive. In this case, the active ingredient of the present invention can be contained at, for example, 0.1% by weight or more and 99.9% by weight or less, preferably 90% by weight or less, 80% by weight or less, 70% by weight or less, 60% by weight or less, 50% by weight or less, 40% by weight or less, 30% by weight or less, 20% by weight or less or 10% by weight or less in the preparation. The pharmaceutical composition of the present invention may be formulated using each active ingredient alone or a plurality of active ingredients in combination. In this case, the active ingredient of the present invention can be contained at, for example, a total of 0.1% by weight or more and 99.9% by weight or less, preferably, 90% by weight or less, 80% by weight or less, 70% by weight or less, 60% by weight or less, 50% by weight or less, 40% by weight or less, 30% by weight or less, 20% by weight or less or 10% by weight or less, in the preparation.

For example, an excipient, a disintegrant, a disintegration aid, a binder, a lubricant, a coating agent, a dye, a diluent, a solubilizer, a solubilization aid, a tonicity agent, a pH adjuster or a stabilizer can be used as the pharmaceutically acceptable carrier or additive.

Examples of the preparation suitable for oral administration can include powders, tablets, capsules, fine granules, granules, solutions and syrups. For the oral administration, various excipients such as microcrystalline cellulose, sodium citrate, calcium carbonate, dipotassium phosphate and glycine can be used together with various disintegrants such as starch, preferably corn, potato or tapioca starch, alginic acid and a certain type of silicate, and granulation binders such as polyvinylpyrrolidone, sucrose, gelatin and gum arabic. Also, a lubricant such as magnesium stearate, sodium lauryl sulfate or talc is often very effective for tableting. Gelatin capsules may be filled with a homogeneous solid composition and used. Examples of suitable substances in relation thereto can include lactose or milk sugar as well as high-molecular-weight polyethylene glycol. In the case of desiring an aqueous suspension and/or an elixir for oral administration, an active ingredient is combined with various sweeteners or flavors, colorants or dyes, optionally with an emulsifier and/or a suspending agent, if necessary, and can be used together with a diluent including water, ethanol, propylene glycol, glycerin, or the like, or a combination thereof.

Examples of the preparation suitable for parenteral administration can include injections and suppositories. For the parenteral administration, the active ingredient of the present invention is dissolved in either sesame oil or peanut oil or dissolved in an aqueous propylene glycol solution, and the resulting solution can be used. The aqueous solution is appropriately buffered (preferably to pH 8 or higher), in need, and a liquid diluent needs to be first rendered isotonic. Such a liquid diluent can employ, for example, saline. The prepared aqueous solution is suitable for intravenous injection, whereas an oil-based solution is suitable for intraarticular injection, intramuscular injection and subcutaneous injection. The production of all of these solutions in a sterile state can be readily achieved by a standard pharmaceutical technique well known to those skilled in the art. Further,the active ingredient of the present invention may be locally administered to the skin or the like. In this case, it is desirable to locally administer the active ingredient in the form of a cream, jelly, a paste or an ointment according to the standard medical practice.

In the present specification, the term "plasmid" means various genetic elements known in the art, for example, a plasmid, a phage, a transposon, a cosmid and a chromosome. The plasmid can be replicated in a particular host and can transfer a gene sequence between cells. In the present specification, the plasmid comprises various nucleotides (DNA, RNA, etc.) known in the art and may be single-stranded or double-stranded. A double-stranded plasmid is preferred.

In the present invention, the method for introducing the foreign gene into a target cancer cell is not particularly limited and can utilize a transfection method known in the art. Examples of the introduction of an expression plasmid or the like into cells or a tissue include methods known in the art such as calcium phosphate coprecipitation method, lipofection, DEAE dextran method, a method of directly injecting a DNA solution to a tissue using an injection needle or the like, and introduction using a gene gun.

In the present specification, terms that are not particularly defined are intended to refer to the scopes of the meaning of terms usually understood by those skilled in the art.

### Examples

### (Experimental procedures)

### (Culture and growth assay of A549, HeLaS3 and MDA-MB-231 cells)

Human cancer cell lines A549 (non-small cell lung cancer), HeLaS3 (uterine cervical cancer) and MDA-MB-231 (human breast cancer) were all cultured and maintained in Dulbecco's modified Eagle's medium containing 10% fetal calf serum. The A549 and MDA-MB-231 cell lines were purchased from the American Type Culture Collection. The HeLaS3 cell line was obtained from the Cell Resource Center for Biomedical Research, Institute of Development, Aging and Cancer, Tohoku University, Japan. Cell growth in monolayer cultures was measured using Cell-Titer Glo (Promega Corp., Madison, WI) in accordance with the manufacturer's instructions. Anchorage-independent growth assay was carried out as described previously (Tando, T. et al., J Biol Chem 285: 21951-60 (2010)).

### (Cell culture of TGS-01, TGS-04 and TGS-05)

Primary grade IV glioblastoma samples were obtained during surgery from consenting patients, as approved by the Institutional Review Board at the University of Tokyo Hospital. Three independent glioma initiating cells (GICs) termed TGS-01, TGS-04 and TGS-05 were established from the samples. All GICs and experimental procedures using these cells were permitted by the ethics committee of the University of Tokyo Hospital (24-69-250809) and Medical Mycology Research Center (MMRC), Chiba University (#10). GICs were passaged in DMEM/F12 serum-free medium (Thermo Fisher Scientific Inc.) supplemented with B27 (Thermo Fisher Scientific Inc.), 20 ng/ml of EGF, and 20 ng/ml of bFGF (both from PeproTech, Inc.) using ultra low attachment dishes or flasks. For the induction of differentiation of GICs as well as the passages of HEK293FT and PLAT-A cells (human embryonic kidney cells), Dulbecco's modified Eagle's medium containing 10% fetal bovine serum (FBS) was used.

### (Isolation of extracts and immunoprecipitation - (1))

Nuclear extracts were prepared from A549 or HeLaS3 cells using NE-PER Nuclear and Cytoplasmic Extraction Reagents (Pierce Biotechnology, Inc., Rockford, IL) in accordance with the manufacturer's instructions, and were finally diluted 8-fold in a buffer [50 mM Tris-HCl (pH 7.5), 100 mM KCl, 1 mM DTT, 20% glycerol and protease inhibitor cocktail (Nacalai Tesque, Inc.)]. Immunoprecipitation was carried out using Dynabeads Protein G (Invitrogen Corp.) and an anti-HA antibody (C29F4; Cell Signaling Technology, Inc.) in accordance with the manufacturers' instructions.

### (Plasmid preparation for retroviral/lentiviral vectors)

Pairs of oligonucleotides encoding gene-specific short hairpin RNA (shRNA) were synthesized (SEQ ID NOs: 14 to 40) and inserted into the BbsI/EcoRI sites of pmU6 (Haraguchi T. et al., Nucleic Acids Res 37: e434 (2009)).

The sequences of shCre#4 (Haraguchi et al., FEBS Letters; 581: 4949-4954 (2007)) [used as negative control (NC)] and shBrm#4 (Yamamichi et al., Oncogene 24:5471-5481 (2005)) were previously described in the paper (Kobayashi, K. et al., Sci. Rep. 5: 8427 (2015)). These plasmids were doubly digested with BamHI and EcoRI, and the resulting fragments encoding shRNA were inserted into the same sites of pSSSP (Yamamichi et al., Oncogene 24: 5471-5481 (2005)) (EV-1) to generate retroviral vector plasmids. For oligonucleotide pairs encoding IL-6 shRNA, through a procedure similar to the above, inserted pmU6 plasmids were digested with BamHI and EcoRI and inserted between these sites in pLSP (Haraguchi T. et al., Nucleic Acids Res 37: e434 (2009)) (EV-4) to generate lentiviral vector plasmids for IL-6 shRNA expression. For Halo-tagged CT1 expression vectors, the SgfI/PmeI fragment of pFN21K Halo tag CMV Flexi Vector (Promega Corp.) (encoding *E. coli* lethal gene *Barnase*) was substituted with an adaptor oligonucleotide or a DNA fragment encoding DPF2-CT1 containing the terminal sequences of SgfI/PmeI to generate pFN21K-Halo and pFN21K-Halo-DPF2-CT1, respectively. Halo- and Halo-DPF2-CT1-containing fragments were isolated by doubly digesting these plasmids with NheI and BamHI, and substituted with the SpeI/BamHI fragment of pXL001 (26112, Addgene) encoding tTR-KRABi, to generate pXL001-Halo (EV-2) and pXL001-Halo-DPF2-CT1, respectively. For the construction of pXL001-Halo-DPF3-CT1, the DPF3-CT1 fragment (approximately 250 bp) prepared by PCR using a primer pair containing the SgfI or PmeI site was substituted with the SgfI/PmeI digest of pXL001-Halo. For HA-tagged vector construction, synthetic oligonucleotides encoding a HA sequence were inserted into the BamHI/EcoRI site of pcDNA3.1(+) (Invitrogen Corp.) to generate pcDNA3.1(+)-HA-MCS.

DPF3-CT1, DPF3a and DPF3b fragments obtained by PCR using sets of a primer containing the terminal sequence of EcoRI and a primer containing the terminal sequence of BglII-EcoRV were digested with EcoRI and EcoRV and inserted into the EcoRI/EcoRV site of pcDNA3.1(+)-HA-MCS to generate pcDNA3.1(+)-HA-DPF3-CT1, pcDNA3.1(+)-HA-DPF3a and pcDNA3.1(+)-HA-DPF3b, respectively. The DNA fragments encoding HA-tagged protein as well as a HA fragment were obtained by doubly digesting them with NheI and BglII, and inserted into SpeI/BamHI site of pXL001 to generate pXL001-HA-DPF3-CT1, pXL001-HA-DPF3a, pXL001-HA-DPF3b and pXL001-HA (EV-3), respectively.

The Brm and BRG1 expression plasmids (pLE-Brm-IP and pLE-BRG1-IP) were prepared by inserting Brm and BRG1 cDNA fragments, which were obtained from pCAG-Brm and BRG1 expression vectors, into pLE-IP (EV-6).

### (DNA transfection and preparation of retro/lentivirus)

For the transfection of each cell line with plasmids, Lipofectamine 2000 (Thermo Fisher Scientific Inc.) was used in accordance with the manufacturer's instructions. Vesicular stomatitis virus-G (VSV-G) pseudotyped retroviral vectors were prepared with the prepackaging cell line (PLAT-A). VSV-G pseudotyped lentiviral vectors were generated with the prepackaging cell line HEK293FT, using ViraPower Lentiviral Expression System (Thermo Fisher Scientific Inc.) in accordance with the manufacturer's instructions. For viral vectors for use in the infection to epithelial cancer cell lines, the medium was changed to DMEM 3 hours after transfection. The transfection supernatant was collected 24 and 48 hours after transfection and filtered through a 0.45 µm filter. The epithelial cancer cell line was transduced by incubation overnight at 37°C with viral vector stocks and polybrene (final concentration: 8 µg/ml). For viral vectors for use in the infection of GICs, the medium was changed to virus production serum-free medium (VP-SFM; Thermo Fisher Scientific Inc.) containing 4 mM L-glutamine 3 hours after transfection. The transfection supernatant was collected 24 and 48 hours after transfection, filtered through a 0.45 µm filter, and subsequently centrifuged at 6000 × g at 4°C for 16 hours. The obtained pellets were suspended in a culture medium for use in GIC culture. GICs were transduced by incubation at 37°C for 4 hours with viral vector stocks.

### (Quantitative RT-PCR)

Total RNA was extracted using mirVana microRNA Isolation Kit (Ambion, Inc. or Thermo Fisher Scientific Inc.). All RNA samples were then treated with TURBO DNase enzyme (TURBO DNA free Kit; Thermo Fisher Scientific Inc.) in some cases. To detect mRNA of a coding gene, cDNA was synthesized using PrimeScript(TM) RT Reagent Kit (Takara Bio Inc.) or PrimeScript RT Master Mix (Takara Bio Inc.) with cDNA Eraser (Perfect Real Time) in accordance with the manufacturer's instructions. Quantitative real time RT-PCR (qRT-PCR) was carried out using a SYBR(TM) Select Master Mix (Applied Biosystems, Inc. or Thermo Fisher Scientific Inc.). GAPDH mRNA was used as an internal control. PCRs were carried out in triplicate using StepOne Plus real time PCR system (Applied Biosystems, Inc. or Thermo Fisher Scientific Inc.).

### (Western blotting)

Total protein extracts were prepared by boiling the cells in SDS sample buffer at 95°C for 10 minutes. The proteins were then electrophoresed by 10% SDS-PAGE and transferred onto Immobilon-P^{SQ} membranes or Immobilon-P PVDF membranes (Millipore Corp.). Immunoblotting was performed by incubating the membrane overnight at 4°C with primary antibodies against the following proteins: Brm (ab15597; Abcam plc), BRG1 (sc-10768; Santa Cruz Biotechnology, Inc.), Halo-Tag (G928A; Promega Corp.), HA-Tag (C29F4; Cell Signaling Technology, Inc.) and β-actin (sc-47778; Santa Cruz Biotechnology, Inc.). Alternatively, Western blotting was performed by incubating the membrane overnight at 4°C in Can Get Signal Solution I (TOYOBO Co., Ltd.) containing the primary antibody. After three washes with Tris-buffered saline (TBS) containing Tween 20, the membranes were incubated with secondary antibodies (donkey anti-rabbit horseradish peroxidase antibody (AP182P) or donkey anti-mouse horseradish peroxidase antibody (AP192P); Millipore Corp.) at room temperature for 1 hour. Signals were detected on AE-9300H-CP Ez-CaptureMG (ATTO Corp.) using ECL reagent (Promega Corp.) or ImmunoStar LD (Wako Pure Chemical Industries, Ltd.). The amounts of the electrophoresed protein samples were normalized to β-actin.

### (Single-cell sphere formation assay)

Two days after transduction with shRNA expression retroviral vectors (pSSCG) or shRNA/cDNA dual expression lentiviral vectors (pLE-IG), GICs were dissociated using TrypLE express (Thermo Fisher Scientific Inc.), and GFP(+)/7-AAD(-) cells were sorted by FACS ARIA1 or ARIA1 (Becton, Dickinson and Company) at a density of 1 cell per well into ultra low attachment 96-well plates (Corning Inc.) in 100 µl of DMEM/F12 serum-free medium. After 2 weeks, the percentage of wells containing spheres was calculated.

### (Intracranial growth assay)

Two days after transduction with shRNA expression lentiviral vectors (pLE-IG), GICs were collected, and GFP(+)/7-AAD(-) cells were sorted by FACS ARIA1 or ARIA2, centrifuged and resuspended in DMEM/F12 serum-free medium. 3 × 10³ cells (2 µl) were injected stereotactically into the right cerebral hemisphere of 5-week-old female BALB/c nu/nu mice at a depth of 3 mm. All animal experimental protocols were carried out in accordance with the policies of the Animal Ethics Committee of the University of Tokyo.

### (Immunoprecipitation - (2))

Cells were lysed with a buffer containing 50 mM Tris-HCl (pH 7.5), 140 mM NaCl, 1 mM MgCl₂, 0.5 mM DTT, 0.1% Tween 20, protease inhibitor cocktail (Nacalai Tesque, Inc.) and phosphatase inhibitor cocktail (Nacalai Tesque, Inc.). Immunoprecipitation was carried out using Dynabeads Protein G (Thermo Fisher Scientific Inc.) in accordance with the manufacturer's instructions.

### (Immunofluorescent staining and proximity ligation assay)

For immunofluorescent staining, GICs were seeded on 8-Well Lab Tek II chamber slide (Nunc) coated with poly-L-lysine. The cells were fixed with PBS containing 4% paraformaldehyde (Nacalai Tesque, Inc.) at room temperature (RT) for 10 minutes, washed twice with PBS and permeabilized with 0.2% Triton X-100 PBS at RT for 15 minutes. After washing twice with PBS, blocking was carried out using a 1:1 mixture of 5% BSA 0.02% NaN₃ PBS and Blocking one (Nacalai Tesque, Inc.) at 37°C for 1 hour. The samples were then incubated with each primary antibody in a blocking buffer overnight at 4°C. The samples were washed twice and subsequently incubated with Alexa Fluor 546 or 488 conjugated secondary antibodies (Thermo Fisher Scientific Inc., 1:1000) in a blocking buffer in the dark at RT for 1 hour. The samples were mounted in Vectashield Mounting Medium with DAPI (Vector Laboratories Inc.). Fluorescence was detected under a fluorescence microscope (BZ--X710; Keyence Corp.). Images were processed using Adobe Photoshop CS3 software.

For proximity ligation assay (PLA), GICs were seeded, treated and incubated with primary antibodies as described above. PLA was carried out using the Duolink In Situ Starter Set ORANGE (Sigma-Aldrich Co. LLC) in accordance with the manufacturer's instructions. Anti-mouse MINUS and anti-rabbit PLUS PLA probes were used. Fluorescence was detected under a fluorescence microscope (BZ-X710; Keyence Corp.).

### (Statistical analysis)

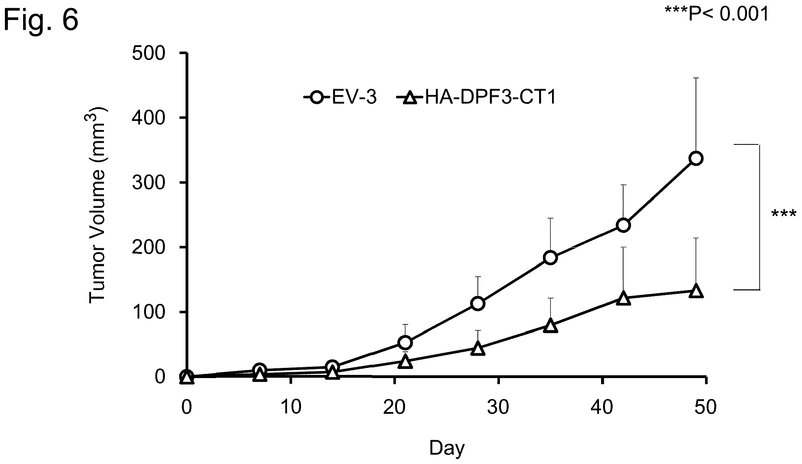

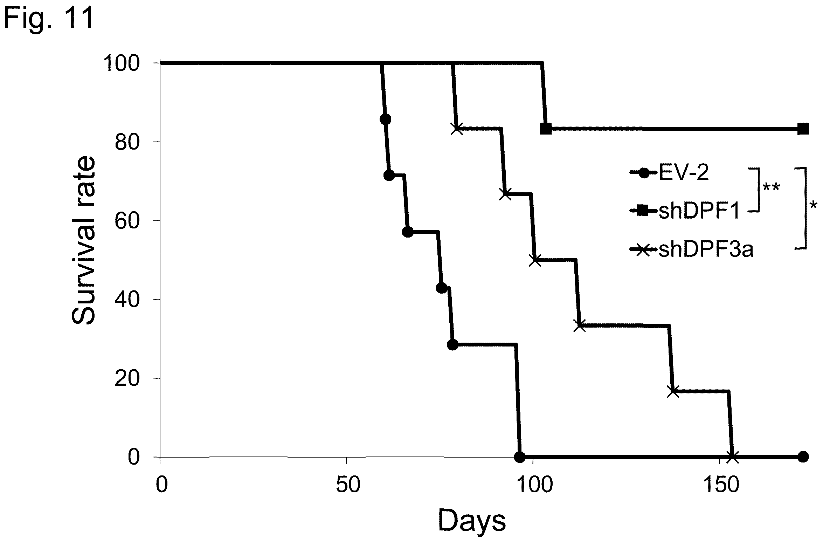

The obtained results are presented as means ± standard deviation (S.D.). Statistical significance for quantitative RT-PCR assay and statistical significance for single-cell sphere formation assay were determined using the two tailed Student's t test. For the survival analysis in Figures 6 and 11, difference in survival rate was evaluated by the log-rank test.

### Example 1

### (Single knockdown of d4 family protein DPF1, DPF2 or DPF3a/b suppresses anchorage-independent growth of tumor cell lines)

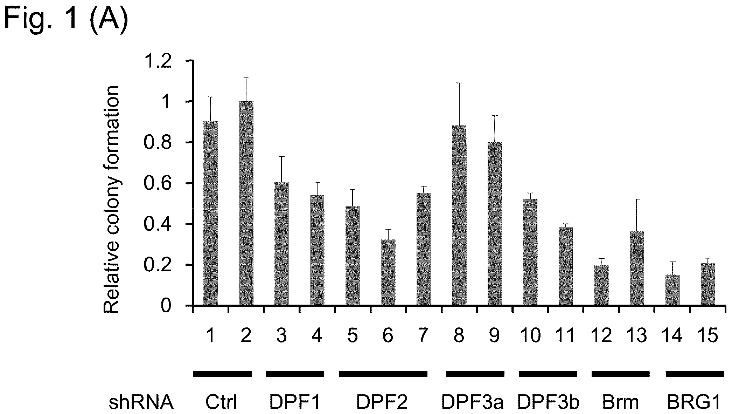

The effect of the knockdown of the expression of each d4 family protein by siRNA on anchorage-independent growth was tested using two human epithelium-derived cancer cell lines (originated from uterine cervical tumors (HeLaS3) and non-small cell lung carcinomas (A549)). The DNA sequences encoding the siRNAs used are shown in Table 2. The cancer cell lines expressed all of the three d4 family proteins, albeit at different expression levels (data not shown). HeLaS3 cells express both Brm and BRG1, whereas A549 cells lack BRG1 expression. Suppressing the expression of any of the d4 family genes in these cells marginally affected cellular growth in monolayer culture (for the A549 cells) (data not shown), but reduced colony formation in soft agar (anchorage-independent growth) to 30 to 60% relative to the control cells (transduced with the shRNA expression vector (shCre#4)) (Figures 1A (for HeLaS3 cells) and 1B (for A549 cells)). This result suggests that the single knockdown of any of the d4 family proteins can partially inhibit the adaptor-mediated links between SWI/SNF complex and NF-κB dimmers, which lead to the suppression of the transforming activity of these cancer cell lines. However, their suppression of anchorage-independent growth was still moderate. This is probably because of the presence of other members of the d4 family proteins with no decrease in their expression even after the single knockdown of the d4 family protein. Since the expression level of each d4 family protein is variable among epithelial tumor cell lines, simultaneous suppression of all the d4 family proteins would be needed for developing effective NF-κB inhibitors independent of the types of epithelial cancer cells.

**[Table 2-1]**

| Used Oligonucleotides | |
|---|---|
| shIL-6-3'UTR#1-sense | 5'- TTTGTATTTATATAATGTATAAATGCTTCCTGTCACATTT ATACATTATATAAATACTTTTTTG-3' |
| shIL-6-3'UTR#1-antisense | 5'- AATTCAAAAAAGTATTTATATAATGTATAAATGTGACA GGAAGCATTTATACATTATATAAATA-3' |
| shDPF1-3'UTR#2-sense | 5'- TTTGTTAATATATACAAAGAGTCCGCTTCCTGTCACG GACTCTTTGTATATATTAACTTTTTTG-3' |
| shDPF1-3'UTR#2-antisense | 5'- AATTCAAAAAAGTTAATATATACAAAGAGTCCGTG A CAGGAAGCGGACTCTTTGTATATATTAA-3' |
| shDPF1-3'UTR#4-sense | 5'- TTTGAATTAACTTGTTCTGTGTATGCTTCCTGTCACAT ACACAGAACAAGTTAATTCTTTTTTG-3' |
| shDPF1-3'UTR#4-antisense | 5'- AATTCAAAAAAGAATTAACTTGTTCTGTGTATGTGAC AGGAAGCATACACAGAACAAGTTAATT-3 |
| shDPF2-3'UTR#3-sense | 5'- TTTGTAGCTTCACCTTGTTATTCCGCTTCCTGTCACGG AATAACAAGGTGAAGCTACTTTTTTG-3' |
| shDPF2-3'UTR#3-antisense | 5'- AATTCAAAAAGTAGCTTCACCTTGTTATTCCGTGAC AGGAAGCGGAATAACAAGGTGAAGCTA-3' |
| shDPF2-3'UTR#4-sense | 5'- TTTGCTCTTAACTGAATTGGGAGCGCTTCCTGTCACG CTCCCAATTCAGTTAAGAGCTTTTTTG-3' |
| shDPF2-3'UTR#4-antisense | 5'- AATTCAAAAAAGCTCTTAACTGAATTGGGAGCGTGA CAGGAAGCGCTCCCAATTCAGTTAAGAG-3' |
| shDPF2-3'UTR#6-sense | 5'- TTTGGTGATCACAGGGTTCAAACAGCTTCCTGTCACT GTTTGAACCCTGTGATCACCTTTTTTG-3' |
| shDPF2-3'UTR#6-antisense | 5'- AATTCAAAAAAGGTGATCACAGGGTTCAAACAGTGA CAGGAAGCTGTTTGAACCCTGTGATCAC-3' |
| shDPF3a-CDS#2-sense | 5'- TTTGCTTTGATGAGGACGATTTGGGCTTCCTGTCACC CAAATCGTCCTCATCAAAGCTTTTTTG-3' |
| shDPF3a-CDS#2-antisens | 5'- AATTCAAAAAAGCTTTGATGAGGACGATTTGGGTGA CAGGAAGCCCAAATCGTCCTCATCAAAG-3' |

**[Table 2-2]**

| | |
|---|---|
| shDPF3a-3'UTR#4-sense | 5'- TTTGAAATCGAAGCAATATCCTGTGCTTCCTGTCACA CAGGATATTGCTTCGATTTCTTTTTTG-3' |
| shDPF3a-3'UTR#4-antisense | 5'- AATTCAAAAAAGAAATCGAAGCAATATCCTGTGTGA CAGGAAGCACAGGATATTGCTTCGATTT-3' |
| shDPF3b-3'CDS#6-sense | 5'- TTTGGGAACTGCTCAAAGAGAAAGGCTTCCTGTCACC TTTCTCTTTGAGCAGTTCCCTTTTTTG-3' |
| shDPF3b-3'CDS#6-antisense | 5'- AATTCAAAAAAGGGAACTGCTCAAAGAGAAAGGTGA CAGGAAGCCTTTCTCTTTGAGCAGTTCC-3' |
| shDPF3b-3'CDS#7-sense | 5'- TTTGATGACCAGCTACTCTTCTGCGCTTCCTGTCACGC AGAAGAGTAGCTGGTCATCTTTTTTG-3' |
| shDPF3b-3'CDS#7-antisense | 5'- AATTCAAAAAAGATGACCAGCTACTCTTCTGCGTGAC AGGAAGCGCAGAAGAGTAGCTGGTCAT-3' |
| shBrm#4-sense | 5'- TTTGAATGTGGTGTTGGTGCTTTCGCTTCCTGTCACG AAAGCACCAACACCACATTCTTTTTTG-3' |
| shBrm#4-antisense | 5'- AATTCAAAAAAGAATGTGGTGTTGGTGCTTTCGTGA CAGGAAGCGAAAGCACCAACACCACATT-3' |
| shBrm#8-sense | 5'- TTTGTGATAAACTACAAAGATAGGGCTTCCTGTCACCCT ATCTTTGTAGTTTATCACTTTTTTG-3' |
| shBrm#8-antisense | 5'- AATTCAAAAAAGTGATAAACTACAAAGATAGGGTGACA GGAAGCCCTATCTTTGTAGTTTATCA |
| shBRG1-CDS#2-sense | 5'- TTTGTTGGAAGTACATGATTGTGGCTTCCTGTCACC CACAATCATGTACTTCCAACTTTTTTG-3' |
| shBRG1-CDS#2-antisense | 5'- AATTCAAAAAAGTTGGAAGTACATGATTGTGGGTGA CAGGAAGCCCACAATCATGTACTTCCAA-3' |
| shBRG1-CDS#4-sense | 5'- TTTGCGTATCGCGGCTTTAAATACGCTTCCTGTCACG TATTTAAAGCCGCGATACGCTTTTTTG-3' |

(These nucleotide sequences are described in SEQ ID NOs: 14 to 40, respectively)

### Example 2

### (Design of d4 family protein truncation mutants that function as dominant mutants)

The d4 family proteins harbor a conserved N-terminal domain, a central C2H2-type Kruppel zinc finger motif and C-terminal paired plant homeobox domain (PHD) fingers (which is considered to mediate interactions with proteins including modified histone) (Lange, M. et al., Genes Dev 22: 2370-84 (2008), Figure 2A). The present inventors previously showed that GST-DPF2 fusion protein can directly bind to SWI/SNF complex subunits Brm, BRG1, Ini1 and BAF60a as well as a NF-κB subunit p52 (Tando, T. et al., J Biol Chem 285: 21951-60 (2010)).

The present inventors constructed a series of truncation mutants of DPF2 protein and tested the binding activity of the mutants to the SWI/SNF complex subunits or the p52 protein, consequently finding that a limited region of the N-terminal region (amino acid residues 1 to 84; designated as CT1 peptide hereinafter, SEQ ID NO: 2) of DPF2 is sufficient for the binding to all of these proteins of interest (Figure 2A). When the amino acid sequences of all of the d4 family proteins are aligned and compared, it was found that the CT1 region is highly conserved among four d4 family proteins (DPF3a and DPF3b have the same N-terminal region) as shown in Figure 2B. Importantly, the C terminal eight amino acid residues of CT1 are composed of a representative nuclear localization signal and would facilitate the CT1 transport to cellular nuclei. Accordingly, the present inventors hypothesized that the CT1 peptide originated from either of d4 family proteins might compete with the role of the d4 family protein as an adaptor molecule between SWI/SNF complex subunits and NF-κB subunits and be thereby capable of functioning as a dominant negative mutant to the entire d4 family protein, when expressed at high levels.

### Example 3

### (Ectopic expression of CT1 peptide inhibits anchorage-independent growth of tumor cell lines)

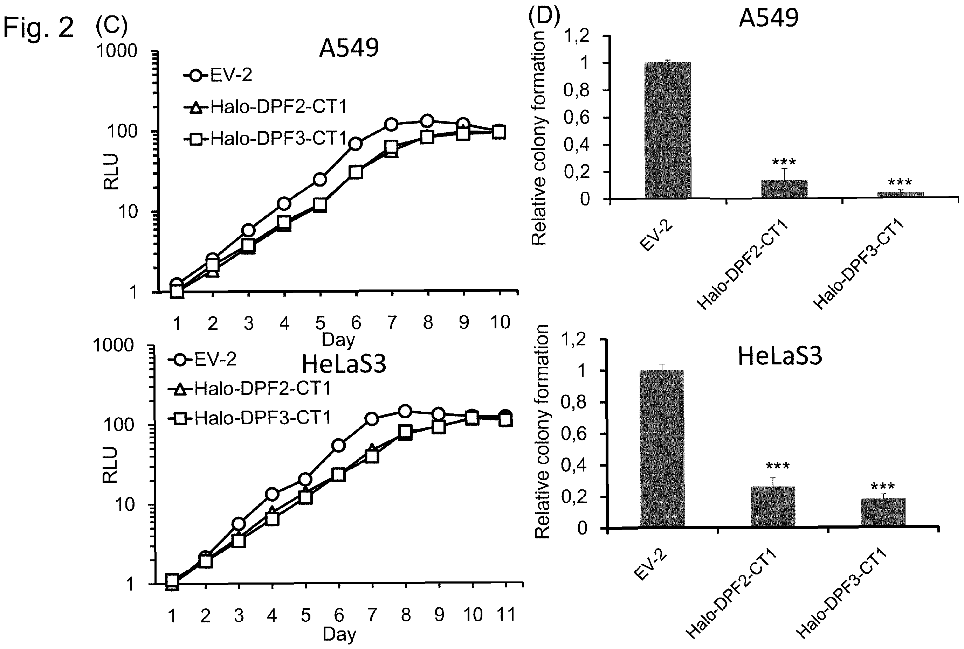

CT1 derived from DPF2 or DPF3 was fused to the C or N terminus of Halo tag (which often stabilizes short peptides fused thereto). When the N-terminally Halo tag-fused CT1 peptide of DPF2 (hereinafter, designated as "Halo-DPF2-CT1") was ectopically expressed in HeLaS3 and A549 cells, the fusion proteins were shown to localize in cellular nuclei by staining with an anti-Halo tag antibody (data not shown). The cells ectopically express Halo-DPF2-CT1 merely provided slightly reduced growth rates in monolayer culture (Figure 2C), whereas the ability of the cells to form colonies was strongly inhibited in an anchorage-independent state in soft agar (Figure 2D); and the colony numbers were remarkably reduced when compared with the cells transduced with an empty vector (EV-2). It was also observed that the effect of Halo-DPF3-CT1 derived from the CT1 region of DPF3 has a high similarity to the effect of Halo-DPF2-CT1 in anchorage-independent growth and growth in monolayers (Figures 2C and 2D).

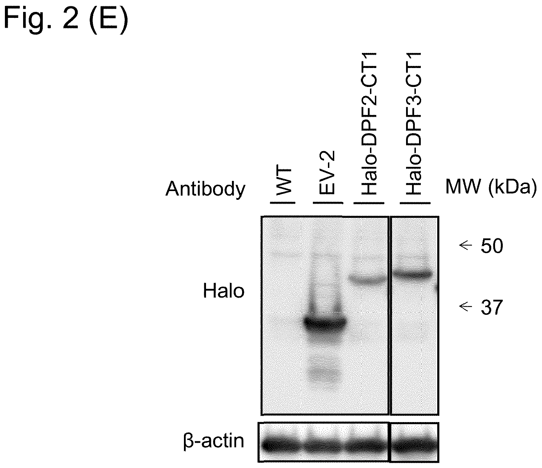

These results suggest that the CT1 peptide derived from any d4 family protein functions as a dominant negative mutant to all the wild-type d4 family proteins. The results also revealed that colony formation in soft agar which reflects a tumor property for epithelial cancer cells requires much higher levels of SWI/SNF complex-dependent NF-κB activity than the growth in monolayer culture. Halo-DPF3-CT1 inhibited anchorage-independent growth more efficiently than Halo-DPF2-CT1 did in both HeLaS3 and A549 cells. This might reflect the expression of Halo-DPF3-CT1 at higher levels than Halo-DPF2-CT1, when expressed by the same expression vector (Figure 2E).

### Example 4

### (A particular subset of NF-κB target genes requires SWI/SNF complex for gene expression thereof)

The present inventors previously reported that shRNA against proteins belonging to the d4 family as well as shRNA against Brm and BRG1 suppressed the luciferase activity of a NF-κB reporter integrated into cellular chromosome (Ishizuka A. et al., J. Biol. Chem. 287: 11924-33 (2012)). Several groups including the present inventors further reported that a particular subset of endogenous NF-κB target genes requires chromatin remodeling by SWI/SNF complex (Ramirez-Carrozzi V.R. et al., Cell 138: 114-28 (2009)).

Accordingly, the present inventors then tested the SWI/SNF complex dependency of representative NF-κB target genes. Four genes, IL-6 (Libermann, T.A. et al., Mol Cell Biol 10: 2327-34 (1990); and Ray A. et al., Mol Cell Biol 10: 5736-46 (1990)), IL-8 (Kunsch C. et al., Mol Cell Biol 13: 6137-46 (1993)), TNF-α (Collart M.A, et al., Mol Cell Biol 10: 1498-506 (1990); and Shakhov A.N. et al., Gene 95: 215-21 (1990)) and ICAM1 (van de Stolpe A. et al., J Biol Chem 269: 6185-92 (1994)), were selected as the test genes. It was known that all of these genes are expressionally induced by TNF-α stimulation via NF-κB binding site(s) present in promoters of the genes in several epithelial cancer cell lines. In A549 cells, the knockdown of a SWI/SNF complex subunit Brm drastically reduced the induction of IL-6 and IL-8 by TNF-α stimulation, but had no influence on the induction of TNF-α and ICAM1. This clear difference in the Brm dependency of these four genes expressionally induced by TNF-α stimulation can be seen even in basal level expression without TNF-α stimulation (data not shown).

When SW13 cells, which express neither Brm nor BRG1, were caused to express Brm or BRG1 by transfection, the basal level expression of IL-6 and IL-8 genes was dramatically elevated (37-fold or more), as compared with SW13 cells transduced with an empty vector. Compared with these two genes, the basal level expression of TNF-α and ICAM1 genes was not significantly affected by the expression of genetically introduced Brm (data not shown).

These loss-of-function and gain-of-function experiments indicate NF-κB target genes can be categorized into two subtypes according to their SWI/SNF complex dependency; and, in these epithelial cancer cell lines, the activation of IL-6 and IL-8 genes through NF-κB requires SWI/SNF complex, whereas the activation of TNF-α and ICAM1 genes do not require the complex.

### Example 5

### (CT1 inhibits induction of a particular subset of NF-κB target genes)

The gene promoters containing NF-κB binding sites would show a wide variety of inducibility even after the TNF-α treatment for NF-κB activation. This inducibility would also be affected by several factors including the chromatin contexts of the promoters in the cell type used and other transcription factors that would be secondarily induced by NF-κB after the TNF-α treatment.

On the basis of a list of NF-κB target genes that were confirmed by their promoter analysis (https;//www.bu.edu/nf-kb/, 594 probes for NF-κB target genes), the present inventors conducted a microarray analysis on the CT1 sensitivity of the NF-κB target gene expression induced by TNF-α treatment in these two cell lines, A549 and HeLaS3 cells (data not shown). By 1-hour TNF-α treatment, 54 genes in A549 cells and 50 genes in HeLaS3 cells were induced more than 1.5 times. In either A549 or HeLaS3 cells, 71 genes that were induced by TNF-α included 19 cytokine genes. Among these cytokine genes, 6 genes were expressionally suppressed by CT1 to less than 66% in ectopically CT1-expressing cells as compared with control cells transduced with the EV-2 vector. IL-6 and IL-8 genes which were expressed in a SWI/SNF complex-dependent manner were detected in these induced genes as expected.

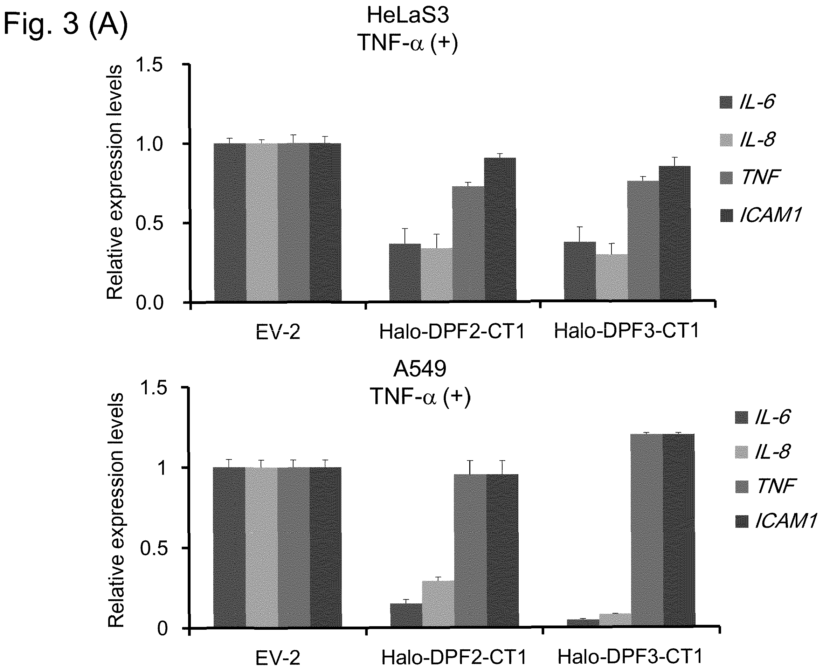

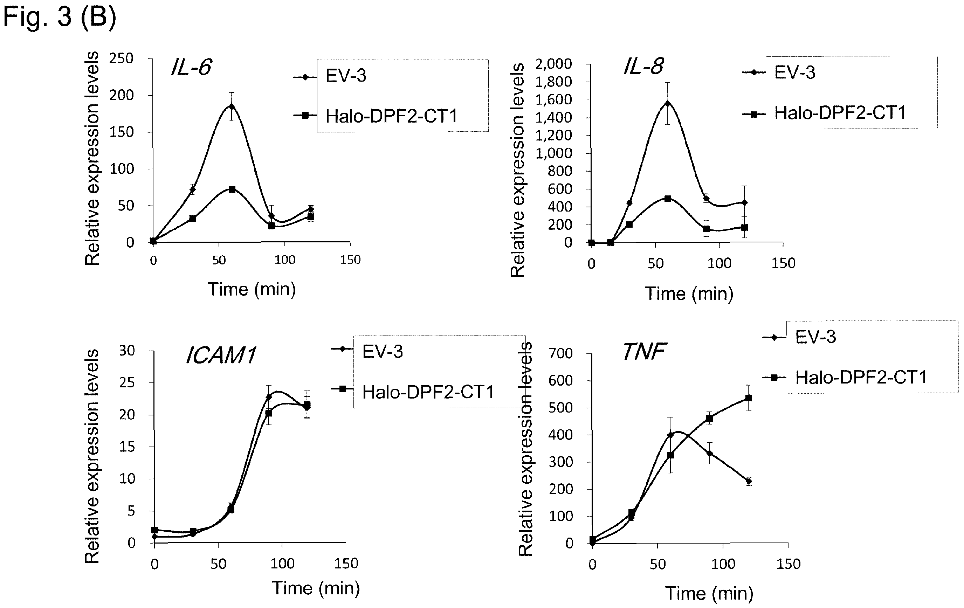

When the RNA samples used in the microarray analysis were also analyzed by quantitative reverse-transcription PCR (qRT-PCR), results similar to those obtained by the microarray analysis were obtained (Figure 3A). The expression of CT1 was confirmed to reduce the expression levels of IL-6 and IL-8 without affecting induction profiles themselves at least within 100 minutes after TNF-α stimulation (Figure 3B).

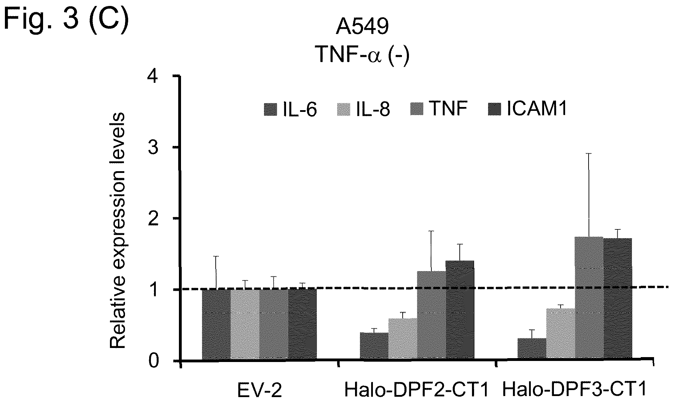

When HeLaS3 and A549 cells were maintained in normal medium, these four genes were expressed at low basal levels. The basal level expression of these four genes in A549 cells was measured by qRT-PCR. Importantly, the basal level expression showed dynamics similar to CT1 expression after stimulation with TNF-α; and that IL-6 and IL-8 genes were sensitive to the ectopic expression of CT1, whereas TNF-α and ICAM1 genes were rather insensitive to the ectopic expression (Figure 3C). This indicates that the SWI/SNF complex-dependent expression of these four genes as determined by CT1 sensitivity has been unchanged between basal level expression and expression after TNF-α stimulation.

### Example 6

### (HA-tagged CT1 also functions as an effective dominant negative mutant and an adaptor linking SWI/SNF with NF-κB)

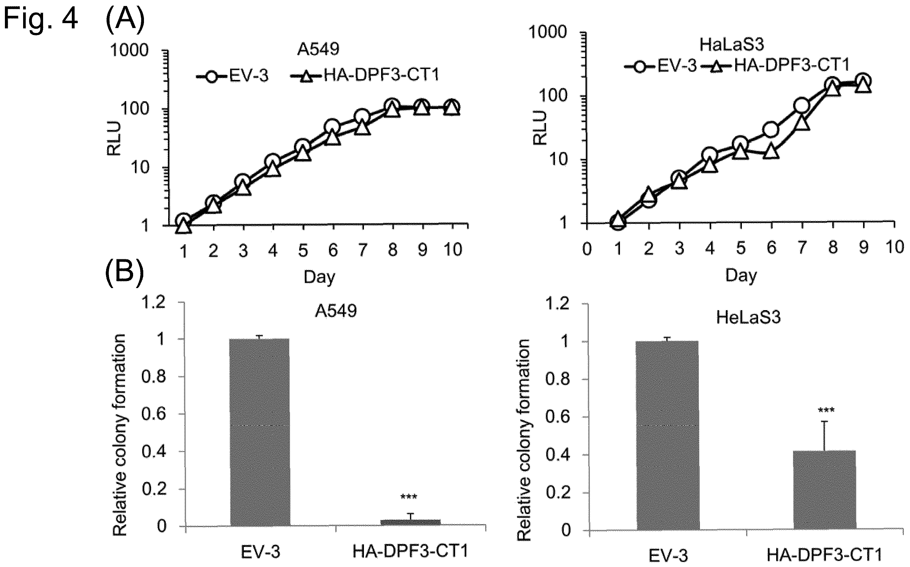

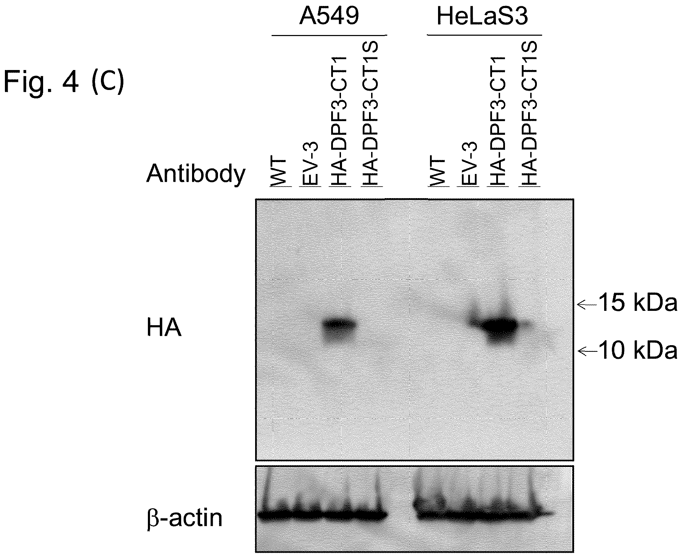

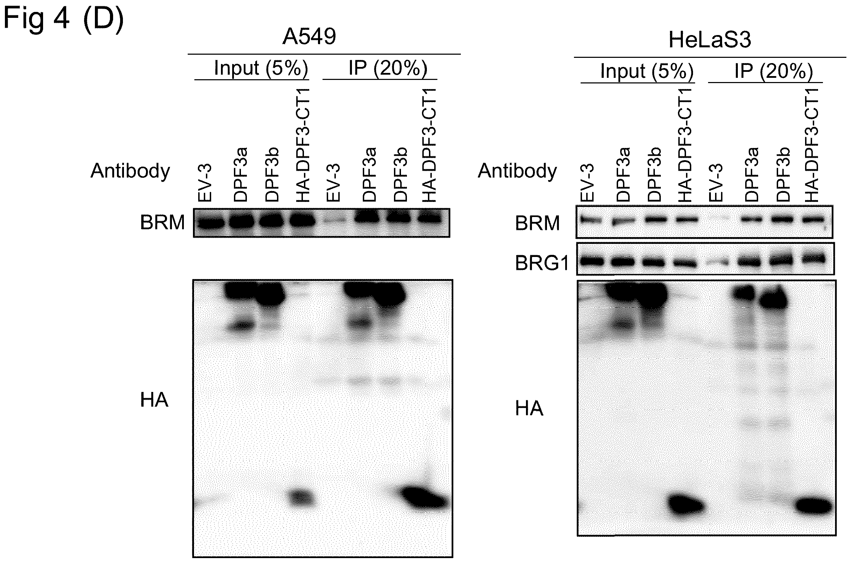

Halo-CT1 was very stable in cells, while HA-CT1 having N-terminally added HA tag, which was shorter than Halo tag, could also be expressed stably in cells. HA-CT1 had a dominant negative function similar to that of Halo-CT1 and reduced anchorage-independent growth without affecting cellular growth in monolayer culture when ectopically expressed in A549 or HeLaS3 cells (Figures 4A and 4B). To test whether HA-CT1 could bind to Brm or BRG1 in cells, total lysates of A549 and HeLaS3 cells caused to ectopically express HA-CT1 (Figure 4C) were immunoprecipitated with an anti-HA antibody. Either Brm or BRG1 was co-immunoprecipitated in the HeLaS3 cells, in a similar efficiency as full-length HA-DPF3a or HA-DPF3b did (Figures 4C and 4D). In the case of A549 cells which lack BRG1 expression, Brm was co-immunoprecipitated with HA-CT1. To narrow down the region required for the dominant negative function of CT1, several terminally truncated mutants or deletion mutants of CT1 were constructed (Figure 5). Δ40-76 peptide lacking amino acid residues 40 to 76 of CT1 and Δ53-76 peptide lacking amino acid residues 53 to 76 of CT1 had lost suppressive effects on IL-6 induction by TNF-α and on anchorage-dependent growth. Δ1-8 lacking amino acid residues 1 to 8 of CT1 retained the original dominant negative activity to some extent. Δ1-39 peptide lacking amino acid residues 1 to 39 of CT1 as well as Δ1-32 peptide lacking amino acid residues 1 to 32 of CT1 was unstable and undetectable when expressed in A549 cells using viral vectors (specific data not shown).

These results strongly suggest that the region from amino acid residues 53 to 76 (SEQ ID NO: 78) or from 40 to 76 (SEQ ID NO: 79) of CT1 contains the SWI/SNF complex binding region and the NF-κB binding region of the d4 family. For peptides containing these two regions, the peptides having a cellular transfer signal and a nuclear localization signal added were synthesized. In the case of using such synthetic peptides, the peptides are capable of functioning as dominant negative mutants to d4 family proteins, as in CT1 (data not shown).

### Example 7

### (CT1 strongly suppresses tumorigenicity of epithelial cancer cells in mouse xenograft models)

To test the tumor suppressing activity of CT1, A549 cells ectopically expressing HA-DPF3-CT1 were injected into immune-deficient mice. As shown in Figure 6, the introduction of CT1 significantly delayed the onset of tumor formation and significantly reduced tumor growth. The present results demonstrated the therapeutic effect of the peptide of the present invention on epithelial cancer.

### (Description of Drawings)

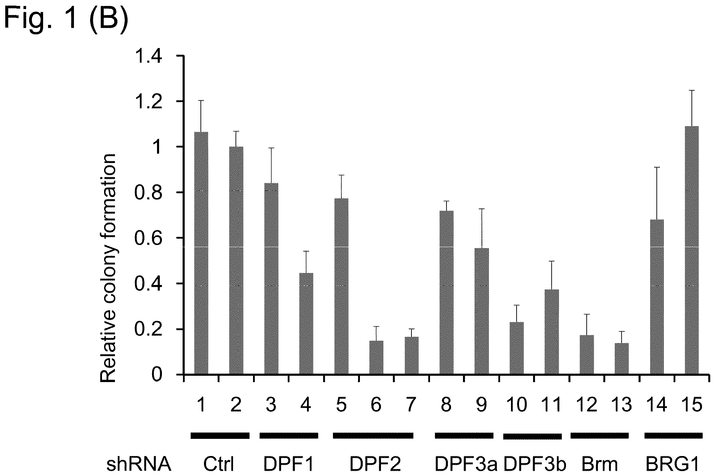

Figure 1. Knockdown effect of each of d4 family proteins as well as Brm and BRG1 on the anchorage-independent growth of HeLaS3 cells (A) and A549 cells (B). The numbers of colonies (100 nm or more in diameter) formed in soft agar were counted and normalized to the number of shCre#4-expressing cells (control; Kobayashi, K. et al., Sci. Rep. 5: 8427 (2015)). shDPF1-3'UTR#2 (SEQ ID NO: 49 or 16) and #4 (SEQ ID NO: 51 or 18) (lanes 3 and 4), shDPF2-3'UTR#3 (SEQ ID NO: 55 or 20), #4 (SEQ ID NO: 56 or 22) and #6 (SEQ ID NO: 57 or 24) (lanes 5, 6 and 7), shDPF3a-CDS#2 (SEQ ID NO: 68 or 26), and shDPF3a-3'UTR#4 (SEQ ID NO: 64 or 28) (lanes 8 and 9), shDPF3b-CDS#6 (SEQ ID NO: 72 or 30) and #7 (SEQ ID NO: 73 or 32) (lanes 10 and 11), shBrm-#4 (SEQ ID NO: 34) and #8 (SEQ ID NO: 36) (lanes 12 and 13), and shBRG1-CDS#2 (SEQ ID NO: 38) and #4 (SEQ ID NO: 40) (lanes 14 and 15). The data represent mean ± standard deviation (n = 3).

Figure 2. (A). Schematic diagram of structure of d4 family proteins and their mutants, CT1. NLS: nuclear localization signal. Zn: C2H2-type Kruppel-type zinc finger motif. PHD: plant homeodomain. The box shown by broken line indicates the N-terminal conserved region among the d4-family proteins. (B). Amino acid sequence alignment of the N-terminal regions of DPF1, DPF2 and DPF3. DPF1 has an additional N-terminal sequence composed of 27 amino acids relative to DPF2 and DPF3. Asterisks (*) indicate positions with identical amino acid residues. Colons (:) indicate positions where the amino acid residues are highly conserved. Panels of (C) and (D). Effect of the ectopic expression of Halo-DPF2-CT1 and Halo-DPF3-CT1 on the cell growth of A549 and HeLaS3 cells in monolayer culture (C) and that in soft agar (D). In the experiment of (C), the A549 and HeLaS3 cells were separately transduced with lentiviral vectors carrying Halo-DPF2-CT1 or Halo-DPF3-CT1. After puromycin selection, the cells were seeded onto 96-well plates, and the cell growth during monolayer culture was monitored every 24 hours using CellTiter-Glo(R) Luminescent Cell Viability Assay. At the same time with seeding onto the plates for monolayer culture, a part of cells of a parallel culture were suspended in soft agar in 60 mm plates, and the number of colonies (100 mm or more in diameter) was counted 18 to 25 days after seeding. Colony numbers formed by control cells transduced with EV-2 were taken as 1.0, and relative values were presented (D). The data represent mean ± standard deviation (n = 3). (E). Expression of Halo-tagged protein introduced in A549 cells in monolayer culture. Total proteins were prepared after culturing, and the expression of introduced genes was analyzed by Western blot using an anti-Halo tag antibody. As an internal standard, β-actin was used.

Figure 3. Inhibitory effect of CT1 after TNF-α stimulation was examined by quantitative RT-PCR analysis. HeLaS3 or A549 cells were transduced with Halo-DPF3-CT1 expression retroviral vectors or control empty vector (EV-2). These cells were stimulated by TNF-α for 1 hour (A) or for 30, 60, 90 and 120 minutes (B), and total RNA was prepared from the cells. The mRNA levels of IL-6, IL-8, TNF-α and ICAM1 were determined by quantitative RT-PCR. In (A), ratios to the mRNA levels of control HeLaS3 cells (transduced with EV-2) are shown. In (B), RNA levels before the TNF-α treatment were taken as 1.0. (C). RNA was also extracted from cultures maintained under TNF-α-untreated conditions, and the mRNA levels of 4 genes of IL-6, IL-8, TNF and ICAM1 were determined and presented as in (A).

Figure 4. (A) and (B). Effect of ectopically expressed HA-DPF3-CT1 on the cell growth of A549 or HeLaS3 cells in monolayer culture (A) and in soft ager (B) was examined. (C). Results of co-immunoprecipitation experiments of catalytic subunit(s) of SWI/SNF complex with HA-DPF3-CT1, HA-DPF3a and HA-DPF3b. Cellular nuclei were prepared from cells expressing HA-DPF3a, HA-DPF3b or HA-DPF3-CT1, and proteins derived from the cellular nuclei were immunoprecipitated with an anti-HA antibody, followed by SDS-PAGE of precipitates. Brm and BRG1 were detected by Western blot. EV-3 represents cells expressing HA tag alone and was used as a negative control.

Figure 5. The biological activities of truncation mutants derived from HA-DPF3-CT1 in A549 cells were summarized. A: Protein expression was evaluated by immunostaining with an antibody. B: One hour after treatment with TNF-α, the levels of IL-6 and IL-8 genes in A549 cells transduced with viral vectors for the expression of CT1 or its mutants were examined by RT-PCR and shown by percentage to their gene expression levels of control A549 cells transduced with EV-3. C: Relative colony formation to the control cells was shown by numerical values.

Figure 6. Tumorigenicity of xenograft A549 cells expressing HA-DPF3-CT1 is shown. A549 cells transduced with lentiviral vectors expressing HA-DPF3-CT1 or control vector (EV-1) at an MOI of 3.0 were injected into left trunks (1 × 10⁶ cells) of nude mice. Tumor volumes were measured, analyzed by two-way ANOVA with Tukey post link test (P < 0.001), and presented as mean ± standard deviation (n = 5).

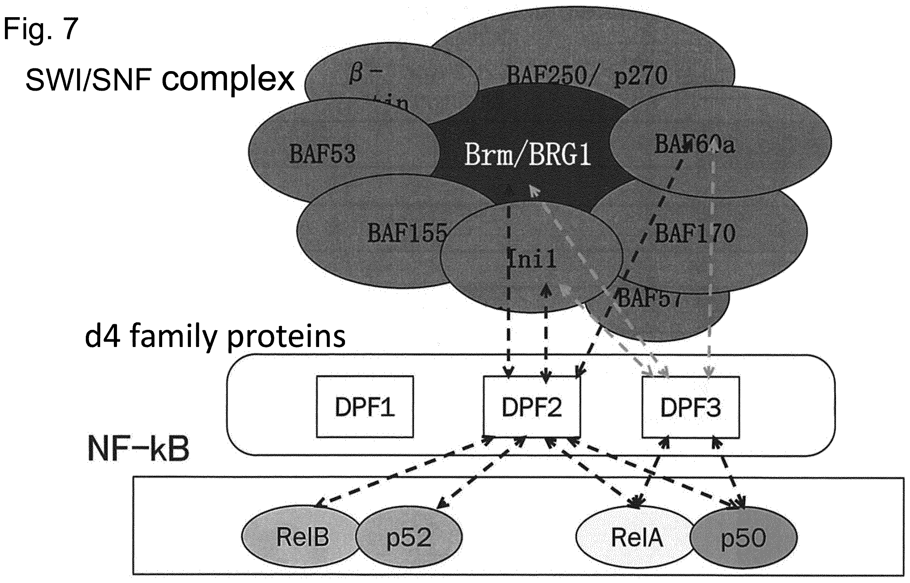

Figure 7. Schematic diagram of the interaction among SWI/SNF complex, d4 family proteins, and NF-κB is represented.

### (Summary of effect of dominant negative mutant to d4 family protein on NF-κB target gene in epithelial cancer)

When target genes of tumor necrosis factor TNF-α-induced NF-κB were classified according to CT1 sensitivity, the induction of IL-6 and IL-8 genes was SWI/SNF-dependent and was suppressed by CT1. In contrast, ICAM1 and TNF-α genes were SWI/SNF-independent and were insensitive to CT1. These properties are advantageous for therapeutic agents for epithelial cancer.

### Example 8

### (DPF1 and DPF3a/b transcripts are abundant in GICs in sphere cultures, but are expressionally suppressed upon differentiation)

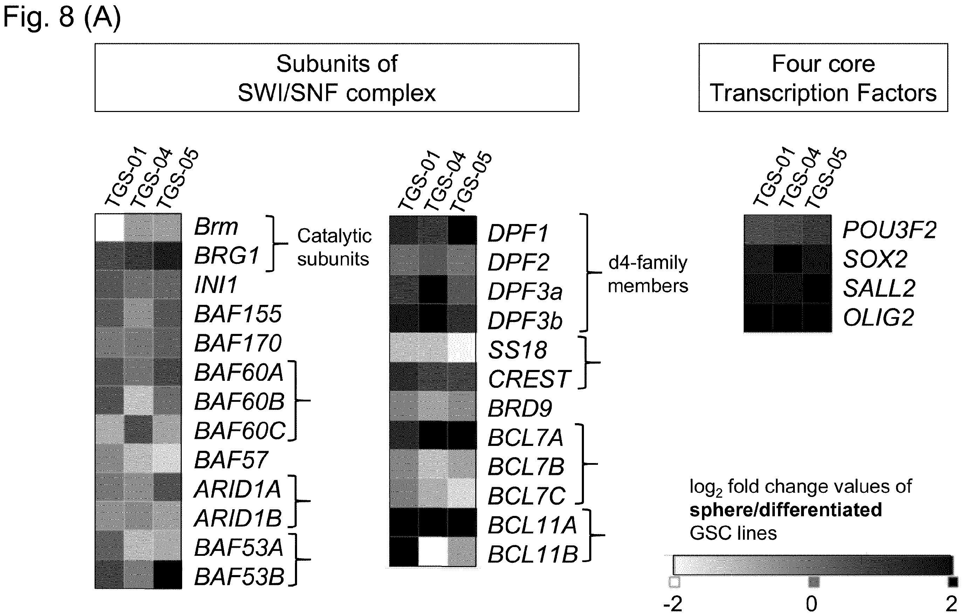

Three independent cell lines derived from glioblastoma (hereinafter, referred to as "GBM") patients (TGS-01, TGS-04 and TGS-05) have previously been reported to have stem cell (glioma initiating cell; hereinafter, referred to as "GIC")-like properties when cultured as non-adherent spheres in a particular medium without serum (Ikushima, H. et al., Cell Stem Cell 5, 504-514 (2009)). The differentiation of these three isolated GIC lines can be induced by cell culturing as adherent monolayers in a medium containing serum (Lee, J. et al., Cancer Cell 9, 391-403 (2006)). The present inventors prepared total RNA and total protein samples from GIC cultures of these three cells in spheres and differentiated GIC cultures. In first, by carrying out qRT-PCR and Western blotting using the prepared GIC samples, the expression of POU3F2, SOX2, SALL2 and OLIG2 was examined, which have been reported to be required for the reconstitution of differentiated cells into stem cells and for the maintenance of stemness of said stem cells (Suva, M. L. et al., Cell 157, 580-594 (2014)) (Figure 8A). By comparing expression at the RNA level between cells cultured in spheres and differentiated cells, the inventor showed that all of these four transcription factors are expressed at higher levels in sphere cultures than in differentiated cells, indicating these GIC cultures have very similar properties to those of previously reported stem-like tumor propagating cells (TPCs) (Lee, J. et al., Cancer Cell 9, 391-403 (2006)).

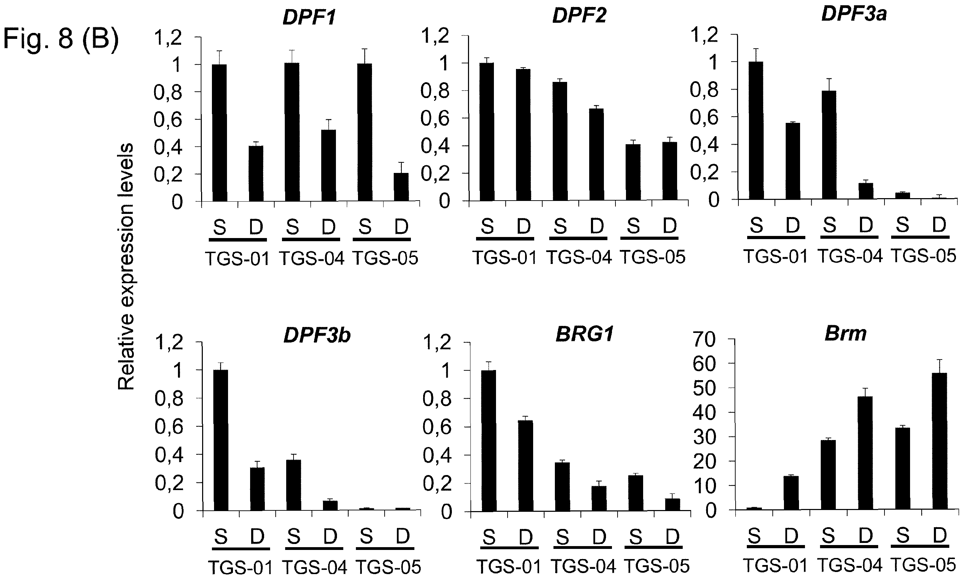

In next, using the same samples as above, the expression levels of core components of SWI/SNF complex and the expression of several proteins strongly associated with SWI/SNF complex were examined. The mRNA (Figures 8A and 8B) and protein (data not shown) levels of BRG1, which is the catalytic subunit thought to be involved in the stemness maintenance of embryonic and neural stem cells, were higher in sphere culture than in differentiated culture in all three independent cell lines (TGS-01, TGS-04 and TGS-05) isolated from GBM patients. Interestingly, the expression level of DPF3a/b was variable among the sphere cultures of the three cell lines (Figure 8B). Among the d4 family members, DPF1 and DPF3a/b were found to be more highly expressed in sphere culture than in differentiated culture (Figure 8A).

### Example 9

### (DPF1 and DPF3a play important roles in maintaining GIC stemness)

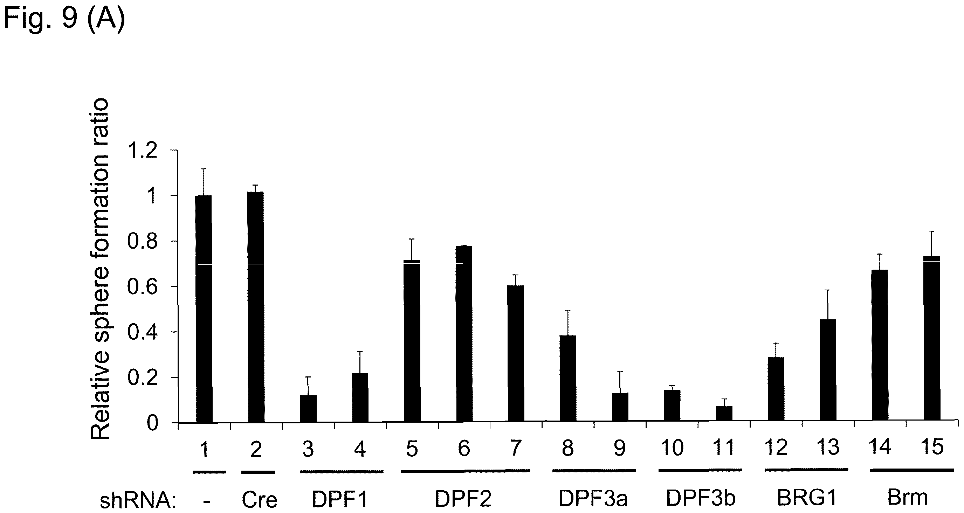

To test whether BRG1 and d4 family proteins would be involved in the stemness maintenance of GICs, knockdown experiments of their gene expression were performed using at least two sets of shRNAs with efficient suppressing activity. Results of preliminary experiments showed that the biological effects of the knockdown of DPF1 and DPF3a seemed to be so prompt that stable transductants in non-adherent sphere cultures could not be isolated by puromycin selection. Accordingly, shRNA expression lentiviral vectors coexpressing GFP were used. To evaluate the stemness of GICs, GFP-positive cells were single sorted into a well 48 hours after transduction, and sphere forming activity was evaluated by sphere formation assay. In this assay, the knockdown of BRG1, DPF1, DPF3a and DPF3b drastically reduced the sphere forming activity of TGS-01 cells, whereas DPF2 knockdown showed only marginal effects (Figure 9A). To examine whether this important function of DPF1 and DPF3a for stemness maintenance could be extended to the other GIC lines, we subjected TGS-04 and TGS-05 cells to DPF1 or DPF3a knockdown and consequently found that the sphere forming activity was reduced in both the cells, as in the TGS-01 cells (data not shown).

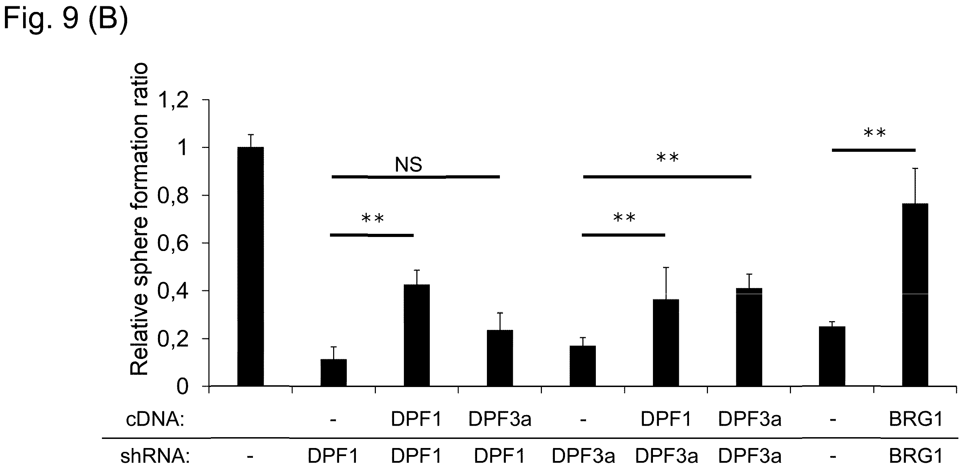

To eliminate the possibility where shRNA constructs effect other than a target (off-target effects), rescue experiments were performed. Since the loss of GIC stemness occurs very early time after introduction of shRNA constructs of d4 family genes, dual lentiviral expression vectors carrying expression units of both shRNA (driven by mU6 pol III promoter) and the corresponding cDNA (driven by EF1α pol II promoter, which is known to be relatively resistant to gene silencing in stem cells) were developed. In this vector, the cDNAs were designed to be resistant to the corresponding shRNA. In the case of BRG1, DPF1 and DPF3a, the corresponding cDNA coexpression partially rescued the sphere forming activity (Figure 9B). Interestingly, DPF3a knockdown was also rescued by the ectopic expression of DPF1, whereas DPF1 knockdown was not significantly rescued by the ectopic expression of DPF3a (Figure 9B). These results suggested that DPF1 provides dominant effects over DPF3a on stem cell maintenance. Because rescue experiments using DPF3b cDNA in DPF3b knockdown cells were not successful and also because the mRNA level of DPF3b was close to a detection limit of qRT-PCR in these GIC lines, no analysis was conducted on rescue experiments of DPF3b in GICs.

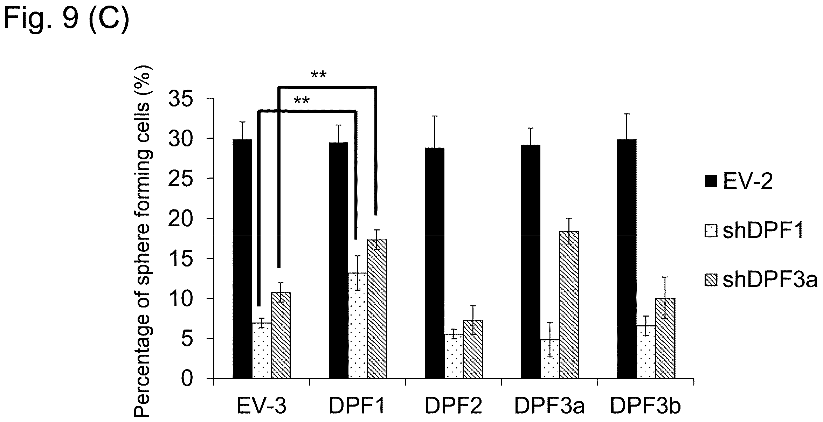

Using TGS-01 cell-derived cells ectopically expressing FLAG-tagged d4 family proteins, knockdown experiments of d4 family genes were also performed in the cells. As a result, similar tendencies to those of the experiments using dual lentiviral expression vectors were seen (Figure 9C). In this experiment, the ectopic expression of d4 family proteins did not increase the sphere forming activity of GIC cell lines, which was worthy of attention. This means that the expression of these proteins from their endogenous genes was at the saturated levels sufficient to maintain the stemness of GICs.

Cells ectopically expressing DPF3a were found to be sensitive to additional DPF1 knockdown (Figure 9C). These results were able to reconfirm the previous observations using the dual expression vectors, that DPF1 is dominant over DPF3a. In these experiments, however, the rescue of sphere forming activity by the corresponding cDNA expression was generally not complete. This may be partly because of the tendency of gene silencing of cDNA expression from lentivirus in long-term stem cell culture even using EF1α promoter.

### Example 10

### (BRG1 can be functionally substituted with Brm as a catalytic subunit of SWI/SNF core complex that contributes to the stemness of GICs)

While higher expression levels of Brm mRNA in differentiated cells were basically similar among the three GIC lines (Figure 8A), the mRNA levels of Brm in sphere cultures of TGS-04 and TGS-05 cells were found to be significantly high when compared with those of TGS-01 cultures (Figure 8B). Meanwhile, when the expression levels of Brm protein in TGS-04 and TGS-05 cells were analyzed by Western blotting, the Brm levels were rather higher in sphere cultures than the levels in differentiated cultures (data not shown). This indicates that there are some posttranslational controls of Brm expression in the cells described above. These results also suggested that SWI/SNF components were significantly inhomogeneous among the three GIC cultures.

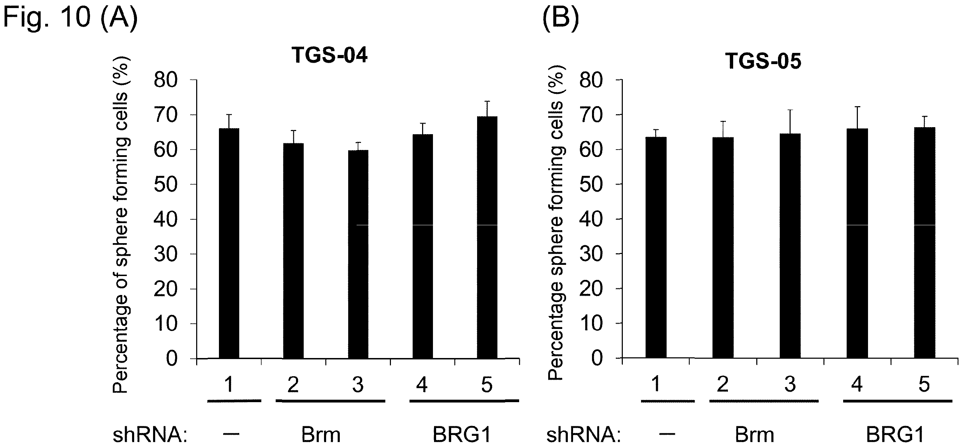

The present inventors further found that the sphere forming activity of TGS-04 and TGS-05 cells was insensitive to the knockdown of any one of BRG1 and Brm (Figures 10A and 10B). This observation was evidently in contrast to our observation in TGS-01 cells (Figure 9A) where the BRG1 knockdown caused reduction in sphere forming activity. It is supposed that, because of high level expression of both BRG1 and Brm in TGS-04 and TGS-05 cells, single knockdown of either of them probably cannot significantly affect the sphere forming activity. This suggested that Brm can also function as an effective catalytic subunit of SWI/SNF complex for the stem cell maintenance of GICs.

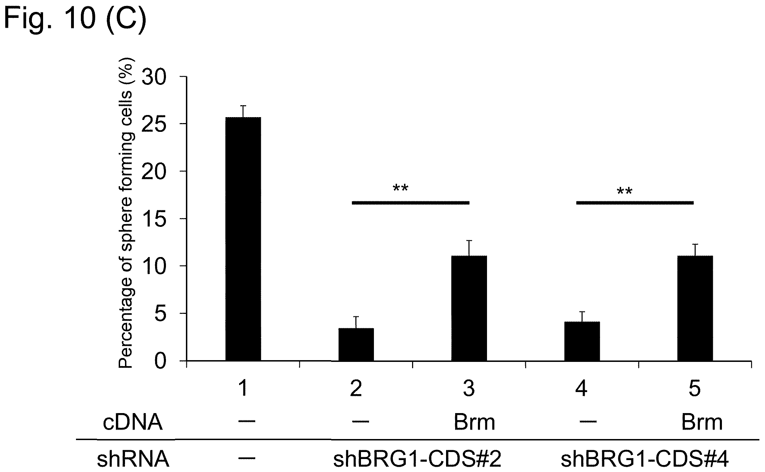

To test whether Brm could indeed substitute BRG1 functions, Brm and shBRG1 were then ectopically coexpressed in TGS-01 cells. Reduction in sphere forming activity by BRG1 knockdown was rescued by the ectopic expression of Brm (Figure 10C). On the other hand, the ectopic expression of Brm itself did not substantially affect the sphere forming activity of the cells (data not shown). These results indicated that Brm, if expressed in GICs at high levels, can also contribute to the maintenance of stemness. Therefore, the simultaneous knockdown of BRG1 and Brm in TGS-04 and TGS-05 cells highly expressing BRG1 and Brm significantly reduced the sphere forming activity of the cells (data not shown).

### Example 11

### (Knockdown of DPF1 and DPF3a in GIC brings about strong anti-tumorigenic activities)

To further confirm the stemness maintenance function of DPF1 and DPF3a, TGS-01 cells transduced with shDPF1 or shDPF3a expression vectors or EV-2 (control) were orthotopically transplanted into immunocompromised mice (nude mice). Rapid reduction in the survival rate of mice inoculated with control cells was observed, whereas both shDPF1 and shDPF3a improved the survival rate of mice inoculated with cells expressing shDPF1 or shDPF3a, as compared with the control cell-inoculated mice (Figure 11). Notably, the expression of shDPF1 showed much greater effects than that of shDPF3a (5 out of 6 mice transplanted with shDPF1-expressing cells showed full survival during the test period). These results suggested that DPF1 is very important for the stemness maintenance of GIC and has the potential as a therapeutic target of GBM. The present results demonstrated the therapeutic effect of the shRNA of the present invention on glioblastoma. Unexpectedly, shRNA against DPF1, in particular, among d4 family genes was very remarkably effective. This tendency is different from the results showing that shRNA against DPF2 or DPF3 is more effective than shRNA against DPF1 for epithelial cancer (Figure 1).

### Example 12

### (Maintenance of stemness by DPF1 and DPF3a does not require NF-κB activation)

In Examples using epithelial cancer cell lines, d4 family proteins were shown to function as adaptor proteins linking SWI/SNF complex with NF-κB dimers. Considering the rather strong involvement of the expression of BRG1, DPF1 and DPF3a in the stemness maintenance of GICs, whether SWI/SNF-dependent NF-κB activation would contribute to the biological activity (stemness maintenance) of GICs was next tested. When IκBαSR, a mutant of an inhibitor IκBα of the NF-κB activation pathway, was introduced into TGS-01 cells, the inventors did not detect any effects on the sphere forming activity. Specifically, the percentages of sphere forming cells in control cells transduced with EV-2 and IκBαSR-expressing cells were 20.57 ± 1.59% and 21.35 ± 0.60%, respectively, with no difference (data not shown). This suggested that in GICs, DPF1 and DPF3a would have clearly distinct functions other than adaptors between SWI/SNF complex and NF-κB and need to play essential roles for the stemness maintenance of GIC through the functions. In the immunostaining of a NF-κB subunit RelA or RelB, their signals reside mainly in the cytoplasm, and in proximity ligation assay (PLA) also mentioned later, the co-localization of SWI/SNF complex and NF-κB was not observed, also suggesting similar possibilities (data not shown).

### Example 13

### (Formation of a large complex containing SWI/SNF core complex and corepressor complex requires DPF1 and DPF3a as adaptors).

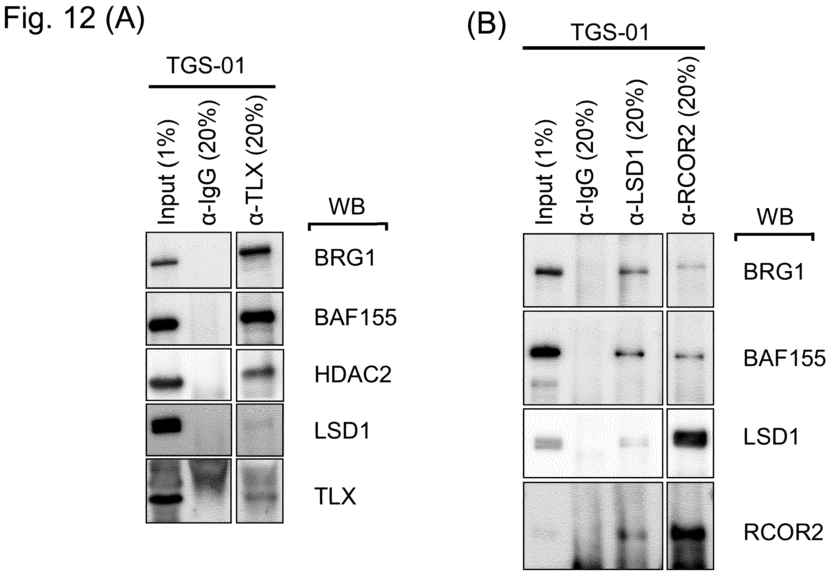

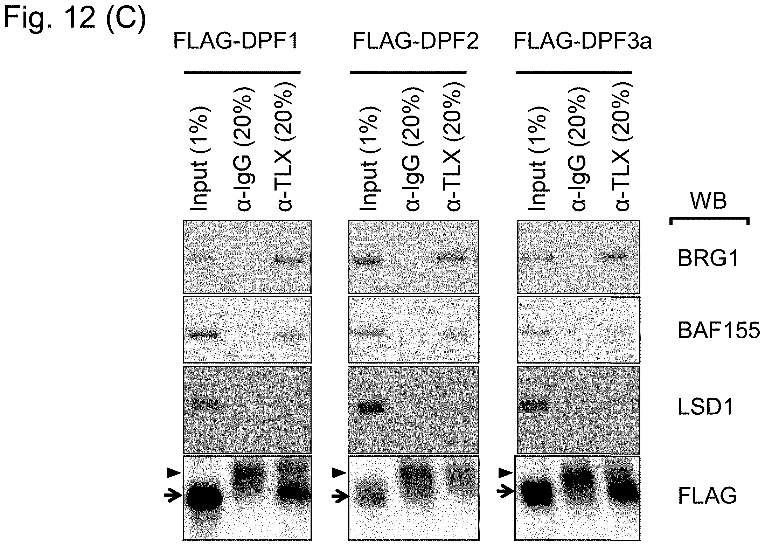

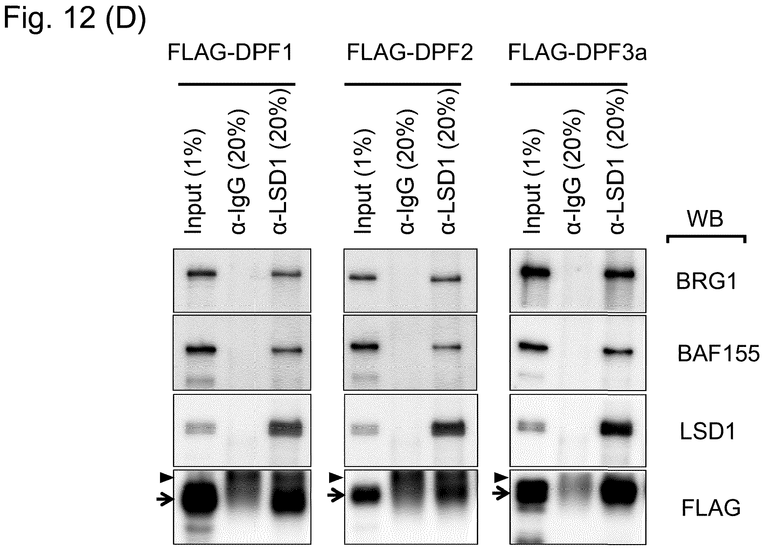

From the results showing that the stemness maintenance of GIC was suppressed promptly and strongly by shDPF1 and, to a lesser extent, by shDPF3a, the inventors hypothesized that DPF1 and DPF3a are capable of functioning as adaptors between transcriptional regulators other than NF-κB, important for the maintenance of GIC stemness, and SWI/SNF core complex. Through a trial and error process, the inventors found that antibodies against TLX (NR2E1), LSD1 (lysine-specific demethylase 1) and RCOR2 (REST corepressor 2; CoREST2) among the candidates that might form a large complex with SWI/SNF core complex co-immunoprecipitate both BRG1 and BAF155 simultaneously from TGS-01 cell lysates (Figures 12A and 12B). TLX known as a nuclear orphan receptor with a transcriptional suppression function has previously been shown to regulate neuronal stem cells and to be essential for the stemness maintenance of GIC in knockdown experiments. LSD1, an epigenetic negative regulator with histone demethylase activity, has also been reported to control the stemness of GICs together with a well-known corepressor RCOR2 (REST corepressor 2: CoREST2). Importantly, the expression levels of TLX protein were not variable between during sphere culture and after induction of differentiation of 3 types of GICs, whereas the levels of LSD1 and RCOR2 were higher in sphere culture. When lysates of TGS-01 cells were immunoprecipitated using an anti-TLX antibody, LSD1 and HDAC2 were detected in these immunoprecipitates (Figure 12A). Moreover, when these cell lysates were immunoprecipitated with an anti-LSD1 antibody, RCOR2 was detected; and when the cell lysates were rather immunoprecipitated with an anti-RCOR2 antibody, LSD1 was detected (Figure 12B). This confirmed previous reports indicating tight dimer formation between these two proteins. Similarly, when the cellular lysates of TGS-04 and TGS-05 cells were immunoprecipitated with an anti-TLX antibody, both BRG1 and BAF155 were detected in the immunoprecipitates (data not shown). Furthermore, when lysates of TGS-01 cells ectopically expressing FLAG-tagged DPF1, DPF2 or DPF3a were immunoprecipitated by either an anti-TLX antibody or an anti-LSD1 antibody, DPF1, DPF2 or DPF3a as well as BRG1, BAF155 and LSD1 were detected (Figures 12C and 12D). Although similar results were also obtained using FLAG-tagged DPF2, the expression levels of FLAG-tagged DPF2 were low. Overall, these results suggest that DPF1 and DPF3a are capable of functioning as adaptor proteins linking between SWI/SNF complex and corepressor complex containing TLX and LSD1/RCOR2.

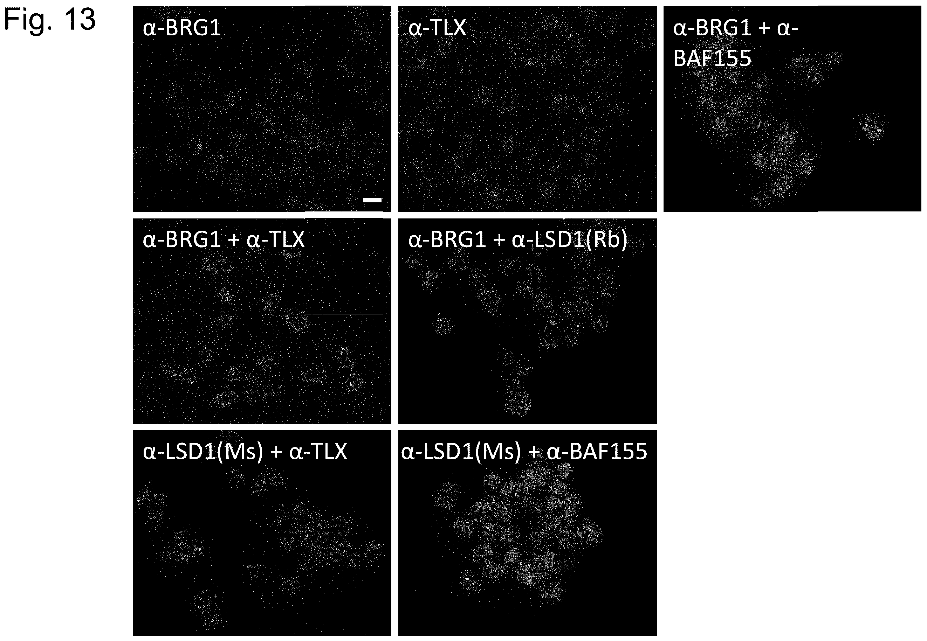

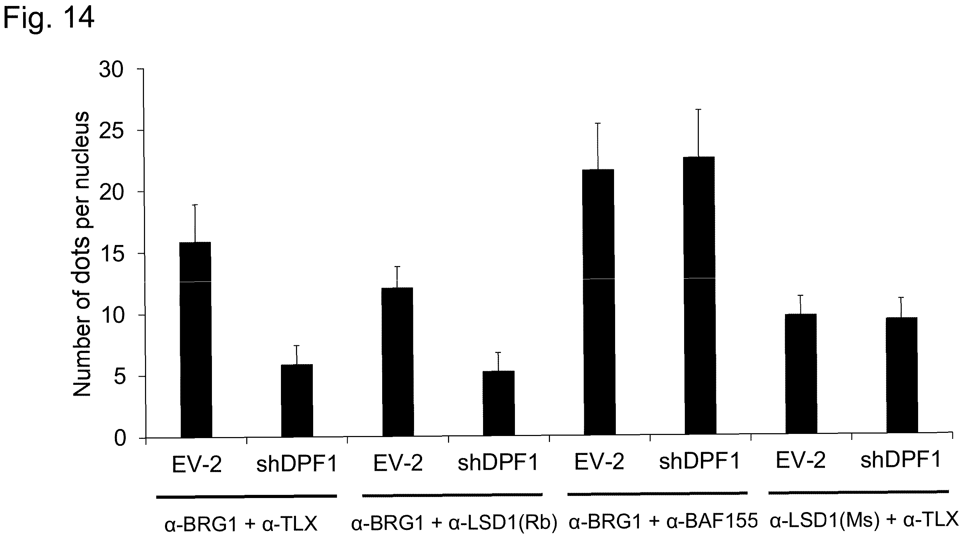

Considering that biochemical protocols that are used to lyse cells and isolate protein complexes from extracts are prone to disrupt bona fide protein interactions that are present in cellular nuclei, proximity ligation assay (PLA) was performed *in situ.* This method visualizes interactions between proximally located proteins in cells after fixing labile large complexes. All the proteins analyzed (BRG1, BAF155, TLX and LSD1) were first confirmed to be strictly localized in the nuclei by immunofluorescence assay, and the same antibodies as those in the assay were used for PLA. As in a positive control using the antibody pairs of BRG1/BAF155 (two subunits of SWI/SNF core complex), antibody pairs of anti-BRG1/anti-TLX, anti-BRG1/anti-LSD1, anti-LSD1/anti-TLX, and anti-LSD1/anti-BAF155 were used to detect the proximal location between these 5 pairs of proteins in GICs (Figure 13). In the case of using only one of the paired antibodies, only marginal signals were detected. When TGS-01 cells transduced with FLAG-tagged DPF1 expression vectors were analyzed by PLA, the proximal location between DPF1 and TLX, between DPF1 and LSD1, or between DPF1 and BAF155 was able to be detected. However, in the case of using an anti-Flag antibody alone, only very marginal signals were detected (data not shown). These results indicated that corepressor complex containing TLX and LSD1 closely binds to SWI/SNF core complex. To examine the influence of DPF1 knockdown on the larger SWI/SNF complex, TGS-01 cells expressing shDPF1 were analyzed by PLA. As a result, signals detecting proximal localization between BRG1 and TLX or between BRG1 and LSD1 were drastically decreased. On the other hand, signals detecting proximal localization between BRG1 and BAF155 or between LSD1 and TLX remained unaffected (Figure 14). These results are consistent with the idea that DPF1 works as an adaptor connecting between SWI/SNF core complex and corepressor complex containing TLX and LSD1/RCOR2.

### Example 14

### (Study on optimization of shRNA sequences against DPF1 and DPF3 canceling GIC stemness)

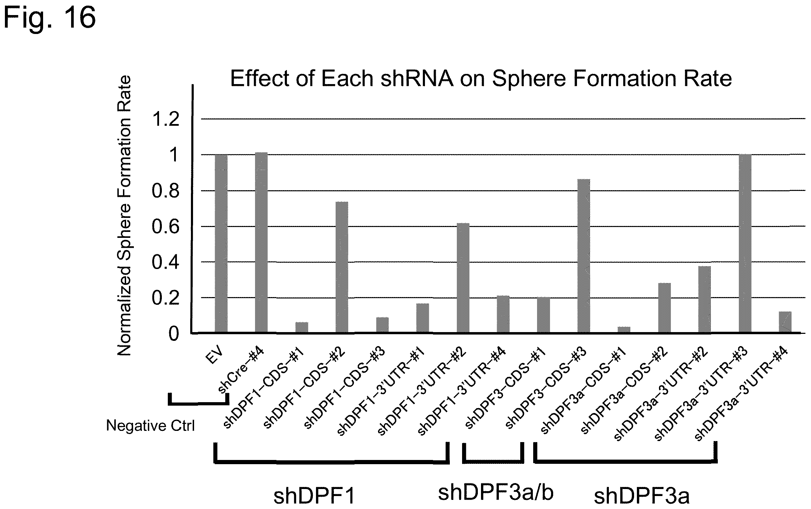

As shown in Example 11, the knockdown of DPF1 and/or DPF3a in GICs exhibited strong anti-tumorigenic activity by canceling the stemness of GICs. Accordingly, shRNA against d4 family genes, which could cancel the stemness of GICs and be also suitable for the treatment of glioblastoma, was studied in more detail. Specifically, in addition to the shRNA used in Example 9, shRNA specific for DPF1, shRNA expected to have common effects on DPF3a and DPF3b, and shRNA specific for DPF3a were each newly generated at plural numbers, and each shRNA was studied for its effect on the sphere formation of TGS-01 cells. The results are shown in Figure 16.

Together with shDPF1-CDS#1 (the sequence of the sense strand of the corresponding siRNA is described in SEQ ID NO: 41; the same applies to the following) and shDPF1-3'UTR#4 (SEQ ID NO: 51) as shRNA against DPF1 used in Example 9, shDPF1-CDS#2 (SEQ ID NO: 43), shDPF1-CDS#3 (SEQ ID NO: 45), shDPF1-3'UTR#1 (SEQ ID NO: 47) and shDPF1-3'UTR#2 (SEQ ID NO: 49) were examined for their effects. As a result, shDPF1-CDS#3 and shDPF1-3'UTR#1 exhibited effects equivalent to those of shDPF1-CDS#1 and shDPF1-3'UTR#4, whereas shDPF1-CDS#2 and shDPF1-3'UTR#2 exhibited no remarkable inhibitory effect on the sphere formation of TGS-01 cells.

As for two shRNAs expected to have an effect of the knockdown thereof on DPF3a and DPF3b in common, i.e., shDPF3-CDS#1 (SEQ ID NO: 58), shDPF3-CDS#3 (SEQ ID NO: 59), shDPF3-CDS#3 had a weak sphere formation inhibitory effect in contrast to shDPF3-CDS#1 which exhibited a significant inhibitory effect.

Together with shDPF3a-3'UTR#2 (SEQ ID NO: 60) and shDPF3a-3'UTR#4 (SEQ ID NO: 64) as shRNA against DPF3a used in Example 9, shDPF3a-CDS#1 (SEQ ID NO: 66), shDPF3a-CDS#2 (SEQ ID NO: 68) and shDPF3a-3'UTR#3 (SEQ ID NO: 62) were examined for their effects. As a result, shDPF3a-CDS#1 and shDPF3a-CDS#2 exhibited a sphere formation inhibitory effect equivalent to or greater than that of shDPF3a-3'UTR#2 and shDPF3a-3'UTR#4 used in Example 9, whereas shDPF3a-3'UTR#3 had a weak inhibitory effect.

### (Description of Drawings)

Figure 8. Heat map showing the expression of mRNA encoding SWI/SNF core complex subunits and mRNA of its associated genes in preparations from three GICs and preparations from the corresponding differentiated cells. (A). mRNA expression in sphere cultures of TGS-01, TGS-04 or TGS-05 was analyzed by qRT PCR and compared with that in differentiated monolayer cultures. (B). Relative gene expression levels of d4 family members, BRG1 and BRM were analyzed by qRT-PCR originated from the same experiment as (A) and indicated in bar graphs. S: sphere culture, D: differentiated monolayer culture. Error bars represent standard deviation of the mean from triplicate experiments.

Figure 9. Effect of the knockdown of a d4 family member, BRG1 or Brm, on the sphere forming activity of TGS-01 cells. (A). Relative ability is shown to form spheres of TGS-01 cells transduced with retroviral vectors expressing various shRNAs or a control (EV-1; lane 1): shDPF1-CDS#1 (SEQ ID NO: 41) (lane 3), shDPF1-3'UTR#4 (SEQ ID NO: 51 or 18) (lane 4), shDPF2-3'UTR#3 (SEQ ID NO: 55), shDPF2-3'UTR#4 (SEQ ID NO: 56) and shDPF2-3'UTR#6 (SEQ ID NO: 57) (lanes 5 to 7), shDPF3a-3'UTR#2 (SEQ ID NO: 60) (lane 8), shDPF3a-3'UTR#4 (SEQ ID NO: 64) (lane 9), shDPF3b-CDS#6 (SEQ ID NO: 72) and shDPF3b-CDS#7 (SEQ ID NO: 73) (lanes 10 and 11), shBRG1-CDS#2 (SEQ ID NO: 38) and shBRG1-CDS#4 (SEQ ID NO: 40) (lanes 12 and 13), and shBrm#4 (SEQ ID NO: 34) and shBrm#8 (SEQ ID NO: 36) (lanes 14 and 15). (B). Relating to rescue experiments. Relative sphere formation ratio of TGS-01 cells transduced with dual lentiviral vectors based on pLE-IG simultaneously expressing mRNA (3 × FLAG-DPF1, 3 × FLAG-DPF3a and 3 × FLAG-BRG1) and shRNA (shCre#4 (Kobayashi, K. et al., Sci. Rep. 5: 8427 (2015)), shDPF1-3'UTR#4 (SEQ ID NO: 51 or 18), shDPF3a-3'UTR#4 (SEQ ID NO: 64 or 28) and shBRG1-CDS#2 (SEQ ID NO: 38)). (C). TGS-01 cells transduced with d4 family protein expression lentiviral vectors or empty vector (EV-3) were secondarily transduced with lentiviral vectors expressing shDPF1-3'UTR#4, shDPF3a-3'UTR#4, or shBRG1-CDS#2, or empty vector (EV-2). Error bars represent standard deviation of the mean from triplicate experiments. NS: not significant. *p < 0.05, **p < 0.01 by Student's t-test.

Figure 10. Effect of the knockdown of BRG1 or Brm on the sphere forming activity of TGS-04 and TGS-05 cells. Percentage of sphere formation cells in culture of TGS-04 (A) and TGS-05 (B) transduced with lentiviral vectors expressing shBrm or shBRG1 or empty vector (EV-2; lane 1). shBrm#4 (SEQ ID NO: 34) and shBrm#8 (SEQ ID NO: 36) (lanes 2 and 3), and shBRG1-CDS#2 (SEQ ID NO: 38) and shBRG1-CDS#4 (SEQ ID NO: 40) (lanes 4 and 5). (C). Relating to the rescue experiments. Percentage of sphere forming cells in culture of TGS-01 transduced with dual lentiviral vectors coexpressing 3 × FLAG-Brm and shBRG1. Lanes 2 and 3 are shBRG1-CDS#2, and lanes 4 and 5 are shBRG1-CDS#4. Error bars represent standard deviation of the mean from triplicate experiments. NS: not significant. **p < 0.01 by Student's t-test.

Figure 11. Kaplan-Meier survival curves of mice orthotopically transplanted with TGS-01 cells. TGS-01 cells transduced with lentiviral vectors expressing shDPF1-3'UTR#4 (SEQ ID NO: 51 or 18) or shDPF3a-3'UTR#4 (SEQ ID NO: 64 or 28), or empty vector (EV-2) were sorted by flow cytometry two days after transduction, and the obtained cells (3 × 10³ cells each) were injected into cerebral hemisphere of 6-week-old female nude mice. *p < 0.05, **p < 0.01 by log-rank test.

Figure 12. Detection of larger SWI/SNF complexes containing TLX and LSD1/RCOR2 in GICs.

Results of co-immunoprecipitation of subunits of SWI/SNF complex with corepressor complex are shown. (A) and (B). Lysates of TGS-01 cells were immunoprecipitated with an anti-TLX antibody (A) or an anti-LSD1 or anti-RCOR2 antibody (B), and the obtained immunoprecipitates were analyzed by Western blotting. (C) and (D). Lysates of TGS-01 cells ectopically expressing 3 × FLAG-DPF1, 3 × FLAG-DPF2 or 3 × FLAG-DPF3a were immunoprecipitated with an anti-TLX antibody (C) or an anti-LSD1 antibody (C), and the immunoprecipitates were analyzed by Western blotting. Arrow: FLAG-tagged d4 family protein; arrowhead: IgG heavy chain. Samples were derived from the same experiment, and gels/blots were treated in parallel.

Figure 13. Proximal localization among SWI/SNF core complex, TLX and LSD1/RCOR2 in TGS-01 cells as detected by PLA.

TGS-01 cells were fixed, incubated with a single antibody or antibody pairs, and detected by PLA. Dots indicated the interaction among proteins, and nuclei were stained with DAPI. Fluorescent images were obtained using quick-full-focus of BZ-X710 (Keyence Corp.) at a depth of approximately 10 µm. Scale bars indicate 10 µm. Rb: rabbit, Ms: mouse.

Figure 14. Effect of the knockdown of DPF1 on the proximal localization among SWI/SNF core complex, TLX and LSD1/RCOR2 in TGS-01 cells as detected by PLA.

TGS-01 cells transduced with shDPF1-3'UTR#4 (SEQ ID NO: 51 or 18) expression lentiviral vectors or empty vector (EV-2) were fixed three days after the transduction and incubated with antibody pairs. The number of dots per nucleus of a GFP-positive cell was counted. Error bars represent 95% confidence interval of the mean (N = 50).

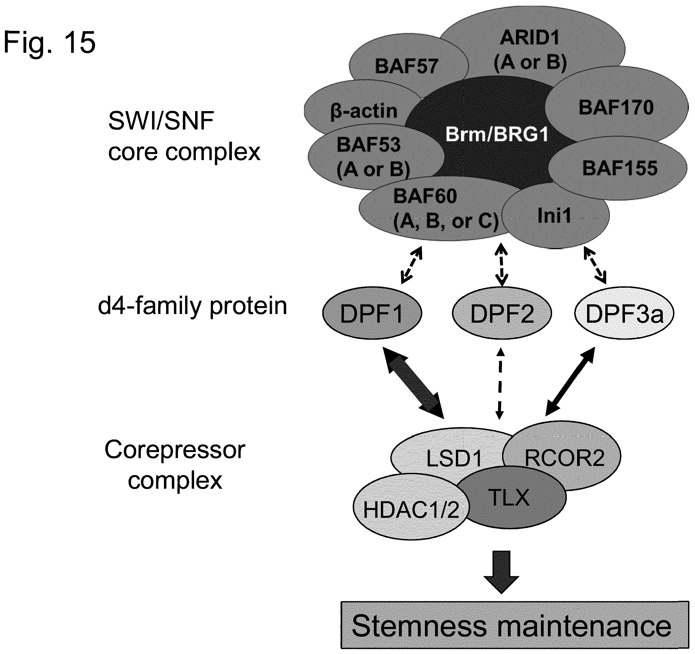

Figure 15. Schematic diagram of the interaction among SWI/SNF complex, d4 family proteins, and TLX is provided.

Figure 16. Results of studying shRNA against d4 family genes suitable for the loss of GIC stemness by using an inhibitory effect on the sphere formation of TGS-01 cells as an index.

### (Summary of effects brought about by using polynucleotide that inhibits the expression of d4 family protein)

The shRNA-mediated knockdown of DPF1 or DPF3a gene in mouse xenograft models orthotopically transplanted with GICs significantly reduced both the ability to form spheres and tumor forming activity. Rescue experiments revealed that DPF1 has dominant effects over DPF3a. The knockdown of either DPF1 or DPF3a was shown to promptly cancel the stemness of GICs. These experiments demonstrated that, via a d4 family protein, SWI/SNF core complex forms a large SWI/SNF complex with particular corepressor complex; and said large SWI/SNF complex is an essential determinant of the important features of GICs (Figure 15). Thus, DPF1 and DPF3a, particularly, DPF1, have been found to be a novel therapeutic target of GBM.

### Industrial Applicability

The medicament according to the present invention can be expected to be utilized as a novel medicament for epithelial cancer or glioma. Specifically, a dominant negative mutant to a d4 family protein acts as a dominant negative mutant to all d4 family proteins and can thus serve as an approach effective for the treatment of epithelial cancer by suppressing only the ability of NF-µB to promote tumor. Furthermore, suppressing DPF1 with RNAi can serve as an approach effective for the treatment of glioma.

### Free Text of Sequence Listing

SEQ ID NO: 1: Amino acid sequence of DPF1-CT1
SEQ ID NO: 2: Amino acid sequence of DPF2-CT1
SEQ ID NO: 3: Amino acid sequence of DPF3a/b-CT1
SEQ ID NO: 4: Amino acid sequence of a Δ1-8 mutant (residues 9 to 84) of DPF3-CT1
SEQ ID NO: 5: Amino acid sequence of a Δ1-32 mutant (residues 33 to 84) of DPF3-CT1
SEQ ID NO: 6: Amino acid sequence of a Δ1-39 mutant (residues 40 to 84) of DPF3-CT1
SEQ ID NO: 7: Amino acid sequence of a Δ1-52 mutant (residues 53 to 84) of DPF3-CT1
SEQ ID NO: 8: Amino acid sequence of a Δ40-76 mutant of DPF3-CT1
SEQ ID NO: 9: Amino acid sequence of a Δ53-76 mutant of DPF3-CT1
SEQ ID NO: 10: Nucleotide sequence of DPF1-CT1
SEQ ID NO: 11: Nucleotide sequence of DPF2-CT1
SEQ ID NO: 12: Nucleotide sequence of DPF3a-CT1
SEQ ID NO: 13: Nucleotide sequence of DPF3b-CT1
SEQ ID NO: 14: shIL-6-3'UTR#1-sense oligonucleotide sequence (in a form converted to DNA)
SEQ ID NO: 15: shIL-6-3'UTR#1-antisense oligonucleotide sequence (in a form converted to DNA)
SEQ ID NO: 16: shDPF1-3'UTR#2-sense oligonucleotide sequence (in a form converted to DNA)
SEQ ID NO: 17: shDPF1-3'UTR#2-antisense oligonucleotide sequence (in a form converted to DNA)
SEQ ID NO: 18: shDPF1-3'UTR#4-sense oligonucleotide sequence (in a form converted to DNA)
SEQ ID NO: 19: shDPF1-3'UTR#4-antisense oligonucleotide sequence (in a form converted to DNA)
SEQ ID NO: 20: shDPF2-3'UTR#3-sense oligonucleotide sequence (in a form converted to DNA)
SEQ ID NO: 21: shDPF2-3'UTR#3-antisense oligonucleotide sequence (in a form converted to DNA)
SEQ ID NO: 22: shDPF2-3'UTR#4-sense oligonucleotide sequence (in a form converted to DNA)
SEQ ID NO: 23: shDPF2-3'UTR#4-antisense oligonucleotide sequence (in a form converted to DNA)
SEQ ID NO: 24: shDPF2-3'UTR#6-sense oligonucleotide sequence (in a form converted to DNA)
SEQ ID NO: 25: shDPF2-3'UTR#6-antisense oligonucleotide sequence (in a form converted to DNA)
SEQ ID NO: 26: shDPF3a-CDS#2-sense oligonucleotide sequence (in a form converted to DNA)
SEQ ID NO: 27: shDPF3a-CDS#2-antisense oligonucleotide sequence (in a form converted to DNA)
SEQ ID NO: 28: shDPF3a-3'UTR#4-sense oligonucleotide sequence (in a form converted to DNA)
SEQ ID NO: 29: shDPF3a-3'UTR#4-antisense oligonucleotide sequence (in a form converted to DNA)
SEQ ID NO: 30: shDPF3b-CDS#6-sense oligonucleotide sequence (in a form converted to DNA)
SEQ ID NO: 31: shDPF3b-CDS#6-antisense oligonucleotide sequence (in a form converted to DNA)
SEQ ID NO: 32: shDPF3b-CDS#7-sense oligonucleotide sequence (in a form converted to DNA)
SEQ ID NO: 33: shDPF3b-CDS#7-antisense oligonucleotide sequence (in a form converted to DNA)
SEQ ID NO: 34: shBrm#4-sense oligonucleotide sequence (in a form converted to DNA)
SEQ ID NO: 35: shBrm#4-antisense oligonucleotide sequence (in a form converted to DNA)
SEQ ID NO: 36: shBrm#8-sense oligonucleotide sequence (in a form converted to DNA)
SEQ ID NO: 37: shBrm#8-antisense oligonucleotide sequence (in a form converted to DNA)
SEQ ID NO: 38: shBRG1-CDS#2-sense oligonucleotide sequence (in a form converted to DNA)
SEQ ID NO: 39: shBRG1-CDS#2-antisense oligonucleotide sequence (in a form converted to DNA)
SEQ ID NO: 40: shBRG1-CDS#4-sense oligonucleotide sequence (in a form converted to DNA)
SEQ ID NO: 41: shDPF1-CDS#1-sense oligonucleotide sequence (in a form converted to DNA)
SEQ ID NO: 42: shDPF1-CDS#1-antisense oligonucleotide sequence (in a form converted to DNA)
SEQ ID NO: 43: shDPF1-CDS#2-sense oligonucleotide sequence (in a form converted to DNA)
SEQ ID NO: 44: shDPF1-CDS#2-antisense oligonucleotide sequence (in a form converted to DNA)
SEQ ID NO: 45: shDPF1-CDS#3-sense oligonucleotide sequence (in a form converted to DNA)
SEQ ID NO: 46: shDPF1-CDS#3-antisense oligonucleotide sequence (in a form converted to DNA)
SEQ ID NO: 47: shDPF1-3'UTR#1-sense oligonucleotide sequence (in a form converted to DNA)
SEQ ID NO: 48: shDPF1-3'UTR#1-antisense oligonucleotide sequence (in a form converted to DNA)
SEQ ID NO: 49: shDPF1-3'UTR#2-sense oligonucleotide sequence (in a form converted to DNA)
SEQ ID NO: 50: shDPF1-3'UTR#2-antisense oligonucleotide sequence (in a form converted to DNA)
SEQ ID NO: 51: shDPF1-3'UTR#4-sense oligonucleotide sequence (in a form converted to DNA)
SEQ ID NO: 52: shDPF1-3'UTR#4-antisense oligonucleotide sequence (in a form converted to DNA)
SEQ ID NO: 53: shDPF2-#1-sense oligonucleotide sequence (in a form converted to DNA)
SEQ ID NO: 54: shDPF2-#2-sense oligonucleotide sequence (in a form converted to DNA)
SEQ ID NO: 55: shDPF2-3'UTR#3-sense oligonucleotide sequence (in a form converted to DNA)
SEQ ID NO: 56: shDPF2-3'UTR#4-sense oligonucleotide sequence (in a form converted to DNA)
SEQ ID NO: 57: shDPF2-3'UTR#6-sense oligonucleotide sequence (in a form converted to DNA)
SEQ ID NO: 58: shDPF3-CDS#1-sense oligonucleotide sequence (in a form converted to DNA)
SEQ ID NO: 59: shDPF3-CDS#3-sense oligonucleotide sequence (in a form converted to DNA)
SEQ ID NO: 60: shDPF3a-3'UTR#2-sense oligonucleotide sequence (in a form converted to DNA)
SEQ ID NO: 61: shDPF3a-3'UTR#2-antisense oligonucleotide sequence (in a form converted to DNA)
SEQ ID NO: 62: shDPF3a-3'UTR#3-sense oligonucleotide sequence (in a form converted to DNA)
SEQ ID NO: 63: shDPF3a-3'UTR#3-antisense oligonucleotide sequence (in a form converted to DNA)
SEQ ID NO: 64: shDPF3a-3'UTR#4-sense oligonucleotide sequence (in a form converted to DNA)
SEQ ID NO: 65: shDPF3a-3'UTR#4-antisense oligonucleotide sequence (in a form converted to DNA)
SEQ ID NO: 66: shDPF3a-CDS#1-sense oligonucleotide sequence (in a form converted to DNA)
SEQ ID NO: 67: shDPF3a-CDS#1-antisense oligonucleotide sequence (in a form converted to DNA)
SEQ ID NO: 68: shDPF3a-CDS#2-sense oligonucleotide sequence (in a form converted to DNA)
SEQ ID NO: 69: shDPF3a-CDS#2-antisense oligonucleotide sequence (in a form converted to DNA)
SEQ ID NO: 70: shDPF3b-CDS#3-sense oligonucleotide sequence (in a form converted to DNA)
SEQ ID NO: 71: shDPF3b-CDS#4-sense oligonucleotide sequence (in a form converted to DNA)
SEQ ID NO: 72: shDPF3b-CDS#6-sense oligonucleotide sequence (in a form converted to DNA)
SEQ ID NO: 73: shDPF3b-CDS#7-sense oligonucleotide sequence (in a form converted to DNA)
SEQ ID NO: 74: Nucleotide sequence of GenBank accession No. NM_001135155.2
SEQ ID NO: 75: Nucleotide sequence of GenBank accession No. NM_006268.4
SEQ ID NO: 76: Nucleotide sequence of GenBank accession No. NM_012074.4
SEQ ID NO: 77: Nucleotide sequence of GenBank accession No. NM_001280542.1
SEQ ID NO: 78: Region from amino acid residues 53 to 76 of DPF3-CT1
SEQ ID NO: 79: Region from amino acid residues 40 to 76 of DPF3-CT1

## Claims

1. An inhibitor which
(a) inhibits the SWI/SNF complex-dependent gene expression regulation by NF-κB or a corepressor complex, or
(b) inhibits an adaptor function of a d4 family protein.

2. The inhibitor according to claim 1, wherein the inhibiting is the competitive inhibition of the function of the d4 family protein, or the suppression of the expression of the d4 family protein.

3. The inhibitor according to claim 2, wherein the d4 family protein is at least one selected from DPF1, DPF2, DPF3a and DPF3b.

4. The inhibitor according to any one of claims 1 to 3, which is a dominant negative mutant to the d4 family protein.

5. The inhibitor according to claim 4, wherein the dominant negative mutant is a polypeptide comprising an amino acid sequence represented by any of SEQ ID NOs: 1 to 7, or a polypeptide which comprises an amino acid sequence having 90% or higher identity to the amino acid sequence represented by any of SEQ ID NOs: 1 to 7, and has a competitive inhibitory activity against the d4 family.

6. The inhibitor according to any one of claims 1 to 5, which binds to the SWI/SNF complex.

7. The inhibitor according to any one of claims 4 to 6, which binds to the NF-κB and/or the corepressor complex.

8. The inhibitor according to any one of claims 1 to 7, which reduces the expression of IL-6 and/or IL-8.

9. The inhibitor according to any one of claims 1 to 3, wherein the inhibitor is an expression vector comprising a polynucleotide encoding an amino acid sequence represented by any of SEQ ID NOs: 1 to 7, or an amino acid sequence having 90% or higher identity to the amino acid sequence represented by any of SEQ ID NOs: 1 to 7.

10. The inhibitor according to any one of claims 1 to 3, wherein the inhibitor is a polynucleotide that suppresses the expression of the d4 family gene.

11. The inhibitor according to claim 10, wherein the polynucleotide is RNA that exerts RNAi, or an antisense oligonucleotide, the RNA or the antisense oligonucleotide that suppresses the expression of the d4 family gene.

12. The inhibitor according to claim 11, wherein the RNA that exerts RNAi and suppresses the expression of the d4 family gene is siRNA or shRNA that is specific for any d4 family gene of SEQ ID NOs: 74 to 77 and suppresses the expression of the d4 family gene.

13. The inhibitor according to claim 11 or 12, wherein the polynucleotide that suppresses the expression of the d4 family gene is siRNA or shRNA selected from double-stranded RNA consisting of a sequence selected from the group consisting of SEQ ID NOs: 41 to 73 and a complementary sequence of the sequence, and double-stranded RNA comprising a sequence comprising deletion, substitution, insertion and/or addition of one to several nucleotides in the sequence and a complementary sequence thereof.

14. The inhibitor according to any one of claims 11 to 13, wherein the RNA that inhibits the expression of the d4 family gene is siRNA comprising annealed polynucleotides of a set selected from a combination of the sequence of SEQ ID NO: 41 and the sequence of SEQ ID NO: 42, a combination of the sequence of SEQ ID NO: 43 and the sequence of SEQ ID NO: 44, a combination of the sequence of SEQ ID NO: 45 and the sequence of SEQ ID NO: 46, a combination of the sequence of SEQ ID NO: 47 and the sequence of SEQ ID NO: 48, a combination of the sequence of SEQ ID NO: 49 and the sequence of SEQ ID NO: 50, a combination of the sequence of SEQ ID NO: 51 and the sequence of SEQ ID NO: 52, a combination of the sequence of SEQ ID NO: 60 and the sequence of SEQ ID NO: 61, a combination of the sequence of SEQ ID NO: 62 and the sequence of SEQ ID NO: 63, a combination of the sequence of SEQ ID NO: 64 and the sequence of SEQ ID NO: 65, a combination of the sequence of SEQ ID NO: 66 and the sequence of SEQ ID NO: 67, and a combination of the sequence of SEQ ID NO: 68 and the sequence of SEQ ID NO: 69, or shRNA comprising the sequence of the siRNA and a loop sequence.

15. The inhibitor according to claim 11, wherein the inhibitor is shRNA comprising a polynucleotide having any sequence selected from SEQ ID NOs: 16 to 33.

16. A pharmaceutical composition comprising an inhibitor according to any one of claims 1 to 15.

17. The pharmaceutical composition according to claim 16, which is an anticancer agent.

18. The pharmaceutical composition according to claim 16, which is a therapeutic agent for epithelial cancer.

19. The pharmaceutical composition according to claim 16, which is a therapeutic agent for glioblastoma.

20. The pharmaceutical composition according to claim 19, which comprises a polynucleotide that suppresses the expression of DPF1 gene as the inhibitor.


SEQUENCE LISTING

<110>  Japan Agency for Medical Research and Development

<120>  Anti-tumor agent inhibiting functions of SWI/SNF complex

<130>  G1773WO

<150>  JP 2017-075506
<151>  2017-04-05

<150>  JP 2017-081615
<151>  2017-04-17

<160>  79

<170>  PatentIn version 3.5

<210>  1
<211>  110
<212>  PRT
<213>  Artificial Sequence

<220>
<223>  DPF1-CT1


<220>
<221>  MISC_FEATURE
<222>  (28)..(110)
<223>  corresponding to DPF2-CT1 and DPF3-CT1

<220>
<221>  SIGNAL
<222>  (103)..(110)
<223>  nuclear localization signal

<400>  1

Met Gly Gly Leu Ser Ala Arg Pro Thr Ala Gly Arg Thr Asp Pro Ala
1               5                   10                  15


Gly Thr Cys Trp Gly Gln Asp Pro Gly Ser Lys Met Ala Thr Val Ile
            20                  25                  30


Pro Gly Pro Leu Ser Leu Gly Glu Asp Phe Tyr Arg Glu Ala Ile Glu
        35                  40                  45


His Cys Arg Ser Tyr Asn Ala Arg Leu Cys Ala Glu Arg Ser Leu Arg
    50                  55                  60


Leu Pro Phe Leu Asp Ser Gln Thr Gly Val Ala Gln Asn Asn Cys Tyr
65                  70                  75                  80


Ile Trp Met Glu Lys Thr His Arg Gly Pro Gly Leu Ala Pro Gly Gln
                85                  90                  95

Ile Tyr Thr Tyr Pro Ala Arg Cys Trp Arg Lys Lys Arg Arg
            100                 105                 110

<210>  2
<211>  84
<212>  PRT
<213>  Artificial Sequence

<220>
<223>  DPF2-CT1

<220>
<221>  SIGNAL
<222>  (77)..(84)
<223>  nuclear localization signal

<400>  2

Met Ala Ala Val Val Glu Asn Val Val Lys Leu Leu Gly Glu Gln Tyr
1               5                   10                  15

Tyr Lys Asp Ala Met Glu Gln Cys His Asn Tyr Asn Ala Arg Leu Cys
            20                  25                  30

Ala Glu Arg Ser Val Arg Leu Pro Phe Leu Asp Ser Gln Thr Gly Val
        35                  40                  45

Ala Gln Ser Asn Cys Tyr Ile Trp Met Glu Lys Arg His Arg Gly Pro
    50                  55                  60

Gly Leu Ala Ser Gly Gln Leu Tyr Ser Tyr Pro Ala Arg Arg Trp Arg
65                  70                  75                  80

Lys Lys Arg Arg

<210>  3
<211>  84
<212>  PRT
<213>  Artificial Sequence

<220>
<223>  DPF3-CT1

<220>
<221>  SIGNAL
<222>  (77)..(84)
<223>  nuclear localization signal

<400>  3

Met Ala Thr Val Ile His Asn Pro Leu Lys Ala Leu Gly Asp Gln Phe
1               5                   10                  15

Tyr Lys Glu Ala Ile Glu His Cys Arg Ser Tyr Asn Ser Arg Leu Cys
            20                  25                  30

Ala Glu Arg Ser Val Arg Leu Pro Phe Leu Asp Ser Gln Thr Gly Val
        35                  40                  45

Ala Gln Asn Asn Cys Tyr Ile Trp Met Glu Lys Arg His Arg Gly Pro
    50                  55                  60

Gly Leu Ala Pro Gly Gln Leu Tyr Thr Tyr Pro Ala Arg Cys Trp Arg
65                  70                  75                  80

Lys Lys Arg Arg

<210>  4
<211>  75
<212>  PRT
<213>  Artificial Sequence

<220>
<223>  DPF3-CT1 delta 1-8 (9-84aar)

<400>  4

Lys Ala Leu Gly Asp Gln Phe Tyr Lys Glu Ala Ile Glu His Cys Arg
1               5                   10                  15

Ser Tyr Asn Ser Arg Leu Cys Ala Glu Arg Ser Val Arg Leu Pro Phe
            20                  25                  30

Leu Asp Ser Gln Thr Gly Val Ala Gln Asn Asn Cys Tyr Ile Trp Met
        35                  40                  45

Glu Lys Arg His Arg Gly Pro Gly Leu Ala Pro Gly Gln Leu Tyr Thr
    50                  55                  60

Tyr Pro Ala Arg Cys Trp Arg Lys Lys Arg Arg
65                  70                  75

<210>  5
<211>  52
<212>  PRT
<213>  Artificial Sequence

<220>
<223>  DPF3-CT1 delta1-32 (33-84 aar)

<400>  5

Ala Glu Arg Ser Val Arg Leu Pro Phe Leu Asp Ser Gln Thr Gly Val
1               5                   10                  15

Ala Gln Asn Asn Cys Tyr Ile Trp Met Glu Lys Arg His Arg Gly Pro
                20                  25                  30

Gly Leu Ala Pro Gly Gln Leu Tyr Thr Tyr Pro Ala Arg Cys Trp Arg
        35                  40                  45

Lys Lys Arg Arg
    50

<210>  6
<211>  43
<212>  PRT
<213>  Artificial Sequence

<220>
<223>  DPF3-CT1 delta1-39 (40-84 aar)

<400>  6

Leu Asp Ser Gln Thr Gly Val Ala Gln Asn Asn Cys Tyr Ile Trp Met
1               5                   10                  15

Glu Lys Arg His Arg Gly Pro Gly Leu Ala Pro Gly Gln Leu Tyr Thr
            20                  25                  30

Tyr Pro Ala Arg Cys Trp Arg Lys Lys Arg Arg
        35                  40

<210>  7
<211>  31
<212>  PRT
<213>  Artificial Sequence

<220>
<223>  DPF3-CT1delta 1-52 (53-84 aar)

<400>  7

Tyr Ile Trp Met Glu Lys Arg His Arg Gly Pro Gly Leu Ala Pro Gly
1               5                   10                  15

Gln Leu Tyr Thr Tyr Pro Ala Arg Cys Trp Arg Lys Lys Arg Arg
            20                  25                  30

<210>  8
<211>  47
<212>  PRT
<213>  Artificial Sequence

<220>
<223>  DPF3-CT1 delta 40-76

<400>  8

Met Ala Thr Val Ile His Asn Pro Leu Lys Ala Leu Gly Asp Gln Phe
1               5                   10                  15

Tyr Lys Glu Ala Ile Glu His Cys Arg Ser Tyr Asn Ser Arg Leu Cys
            20                  25                  30

Ala Glu Arg Ser Val Arg Leu Arg Cys Trp Arg Lys Lys Arg Arg
        35                  40                  45

<210>  9
<211>  60
<212>  PRT
<213>  Artificial Sequence

<220>
<223>  DPF3-CT1 delta 53-76

<400>  9

Met Ala Thr Val Ile His Asn Pro Leu Lys Ala Leu Gly Asp Gln Phe
1               5                   10                  15

Tyr Lys Glu Ala Ile Glu His Cys Arg Ser Tyr Asn Ser Arg Leu Cys
            20                  25                  30

Ala Glu Arg Ser Val Arg Leu Pro Phe Leu Asp Ser Gln Thr Gly Val
        35                  40                  45

Ala Gln Asn Asn Arg Cys Trp Arg Lys Lys Arg Arg
    50                  55                  60

<210>  10
<211>  330
<212>  DNA
<213>  Artificial Sequence

<220>
<223>  DPF1-CT1 from M_001135155.2 Homo sapiens double PHD fingers 1
       (DPF1), transcript variant 1, mRNA

<400>  10
atgggcggcc tcagcgcccg cccgaccgct gggaggaccg acccggcggg gacctgctgg      60

gggcaggacc cggggagcaa gatggccact gtcatccctg gccccctgag cctaggcgag      120

gacttctacc gcgaggccat cgagcactgc cgcagttaca acgcgcgcct gtgcgccgag      180

cgcagcctgc gactgccctt cctcgactcg cagaccggcg tggcccagaa caactgctac      240

atctggatgg agaagaccca ccgcgggccg ggtttggccc cgggacagat ttacacgtac      300

cccgcccgct gttggaggaa gaaacggaga                                        330

<210>  11

<211>  252
<212>  DNA
<213>  Artificial Sequence

<220>
<223>  DPF2-CT1 from NM_006268.4 Homo sapiens double PHD fingers 2
       (DPF2), transcript variant 1, mRNA

<400>  11
atggcggctg tggtggagaa tgtagtgaag ctccttgggg agcagtacta caaagatgcc      60

atggagcagt gccacaatta caatgctcgc ctctgtgctg agcgcagcgt gcgcctgcct     120

ttcttggact cacagaccgg agtagcccag agcaattgtt acatctggat ggaaaagcga     180

caccggggtc caggattggc ctccggacag ctgtactcct accctgcccg gcgctggcgg     240

aaaaagcggc ga                                                         252


<210>  12
<211>  252
<212>  DNA
<213>  Artificial Sequence

<220>
<223>  DPF3a-CT1 from NM_012074.4 Homo sapiens double PHD fingers 3
       (DPF3), transcript variant 1, mRNA

<400>  12
atggcgactg tcattcacaa ccccctgaaa gcgctcgggg accagttcta caaggaagcc      60

attgagcact gccggagtta caactcacgg ctgtgtgcag agcgcagcgt gcgtcttccc     120

ttcctggact cacagactgg ggtggcccag aacaactgct acatctggat ggagaagagg     180

caccgaggcc caggccttgc cccgggccag ctgtatacat accctgcccg ctgctggcgc     240

aagaagagac ga                                                         252


<210>  13
<211>  252
<212>  DNA
<213>  Artificial Sequence

<220>
<223>  DPF3b-CT1 from NM_001280542.1 Homo sapiens double PHD fingers 3
       (DPF3), transcript variant 2, mRNA

<400>  13
atggcgactg tcattcacaa ccccctgaaa gcgctcgggg accagttcta caaggaagcc      60

attgagcact gccggagtta caactcacgg ctgtgtgcag agcgcagcgt gcgtcttccc     120

ttcctggact cacagactgg ggtggcccag aacaactgct acatctggat ggagaagagg     180

caccgaggcc caggccttgc cccgggccag ctgtatacat accctgcccg ctgctggcgc     240

aagaagagac ga                                                         252


<210>  14

<211>  64
<212>  DNA
<213>  Artificial Sequence

<220>
<223>  shIL-6-3'UTR#1-sense

<400>  14
tttgtattta tataatgtat aaatgcttcc tgtcacattt atacattata taaatacttt      60

tttg                                                                  64


<210>  15
<211>  64
<212>  DNA
<213>  Artificial Sequence

<220>
<223>  shIL-6-3'UTR#1-antisense

<400>  15
aattcaaaaa agtatttata taatgtataa atgtgacagg aagcatttat acattatata      60

aata                                                                  64


<210>  16
<211>  64
<212>  DNA
<213>  Artificial Sequence

<220>
<223>  shDPF1-3'UTR#2-sense

<400>  16
tttgttaata tatacaaaga gtccgcttcc tgtcacggac tctttgtata tattaacttt      60

tttg                                                                  64


<210>  17
<211>  64
<212>  DNA
<213>  Artificial Sequence

<220>
<223>  shDPF1-3'UTR#2-antisense

<400>  17
aattcaaaaa agttaatata tacaaagagt ccgtgacagg aagcggactc tttgtatata      60

ttaa                                                                  64


<210>  18
<211>  64
<212>  DNA
<213>  Artificial Sequence

<220>
<223>  shDPF1-3'UTR#4-sense

<400>  18
tttgaattaa cttgttctgt gtatgcttcc tgtcacatac acagaacaag ttaattcttt      60

tttg                                                                    64


<210>  19
<211>  64
<212>  DNA
<213>  Artificial Sequence

<220>
<223>  shDPF1-3'UTR#4-antisense

<400>  19
aattcaaaaa agaattaact tgttctgtgt atgtgacagg aagcatacac agaacaagtt      60

aatt                                                                    64


<210>  20
<211>  64
<212>  DNA
<213>  Artificial Sequence

<220>
<223>  shDPF2-3'UTR#3-sense

<400>  20
tttgtagctt caccttgtta ttccgcttcc tgtcacggaa taacaaggtg aagctacttt      60

tttg                                                                    64


<210>  21
<211>  64
<212>  DNA
<213>  Artificial Sequence

<220>
<223>  shDPF2-3'UTR#3-antisense

<400>  21
aattcaaaaa agtagcttca ccttgttatt ccgtgacagg aagcggaata acaaggtgaa      60

gcta                                                                    64


<210>  22
<211>  64
<212>  DNA
<213>  Artificial Sequence

<220>
<223>  shDPF2-3'UTR#4-sense

<400>  22
tttgctctta actgaattgg gagcgcttcc tgtcacgctc ccaattcagt taagagcttt      60

tttg                                                                    64


<210>  23

<211>  64
<212>  DNA
<213>  Artificial Sequence

<220>
<223>  shDPF2-3'UTR#4-antisense

<400>  23
aattcaaaaa agctcttaac tgaattggga gcgtgacagg aagcgctccc aattcagtta      60

agag                                                                    64


<210>  24
<211>  64
<212>  DNA
<213>  Artificial Sequence

<220>
<223>  shDPF2-3'UTR#6-sense

<400>  24
tttggtgatc acagggttca aacagcttcc tgtcactgtt tgaaccctgt gatcaccttt      60

tttg                                                                    64


<210>  25
<211>  64
<212>  DNA
<213>  Artificial Sequence

<220>
<223>  shDPF2-3'UTR#6-antisense

<400>  25
aattcaaaaa aggtgatcac agggttcaaa cagtgacagg aagctgtttg aaccctgtga      60

tcac                                                                    64


<210>  26
<211>  64
<212>  DNA
<213>  Artificial Sequence

<220>
<223>  shDPF3a-CDS#2-sense

<400>  26
tttgctttga tgaggacgat ttgggcttcc tgtcacccaa atcgtcctca tcaaagcttt      60

tttg                                                                    64


<210>  27
<211>  64
<212>  DNA
<213>  Artificial Sequence

<220>
<223>  shDPF3a-CDS#2-antisense

<400>  27
aattcaaaaa agctttgatg aggacgattt gggtgacagg aagcccaaat cgtcctcatc      60

aaag                                                                  64


<210>  28
<211>  64
<212>  DNA
<213>  Artificial Sequence

<220>
<223>  shDPF3a-3'UTR#4-sense

<400>  28
tttgaaatcg aagcaatatc ctgtgcttcc tgtcacacag gatattgctt cgatttcttt      60

tttg                                                                  64


<210>  29
<211>  64
<212>  DNA
<213>  Artificial Sequence

<220>
<223>  shDPF3a-3'UTR#4-antisense

<400>  29
aattcaaaaa agaaatcgaa gcaatatcct gtgtgacagg aagcacagga tattgcttcg      60

attt                                                                  64


<210>  30
<211>  64
<212>  DNA
<213>  Artificial Sequence

<220>
<223>  shDPF3b-3'CDS#6-sense

<400>  30
tttgggaact gctcaaagag aaaggcttcc tgtcaccttt ctctttgagc agttcccttt      60

tttg                                                                  64


<210>  31
<211>  64
<212>  DNA
<213>  Artificial Sequence

<220>
<223>  shDPF3b-3'CDS#6-antisense

<400>  31
aattcaaaaa agggaactgc tcaaagagaa aggtgacagg aagcctttct ctttgagcag      60

ttcc                                                                  64


<210>  32

<211>  64
<212>  DNA
<213>  Artificial Sequence

<220>
<223>  shDPF3b-3'CDS#7-sense

<400>  32
tttgatgacc agctactctt ctgcgcttcc tgtcacgcag aagagtagct ggtcatcttt      60

tttg                                                                    64


<210>  33
<211>  64
<212>  DNA
<213>  Artificial Sequence

<220>
<223>  shDPF3b-3'CDS#7-antisense

<400>  33
aattcaaaaa agatgaccag ctactcttct gcgtgacagg aagcgcagaa gagtagctgg      60

tcat                                                                    64


<210>  34
<211>  64
<212>  DNA
<213>  Artificial Sequence

<220>
<223>  shBrm#4-sense

<400>  34
tttgaatgtg gtgttggtgc tttcgcttcc tgtcacgaaa gcaccaacac cacattcttt      60

tttg                                                                    64


<210>  35
<211>  64
<212>  DNA
<213>  Artificial Sequence

<220>
<223>  shBrm#4-antisense

<400>  35
aattcaaaaa agaatgtggt gttggtgctt tcgtgacagg aagcgaaagc accaacacca      60

catt                                                                    64


<210>  36
<211>  64
<212>  DNA
<213>  Artificial Sequence

<220>
<223>  shBrm#8-sense

<400>  36
tttgtgataa actacaaaga tagggcttcc tgtcacccta tctttgtagt ttatcacttt      60

tttg                                                                    64


<210>  37
<211>  64
<212>  DNA
<213>  Artificial Sequence

<220>
<223>  shBrm#8-antisense

<400>  37
aattcaaaaa agtgataaac tacaaagata gggtgacagg aagccctatc tttgtagttt      60

atca                                                                    64


<210>  38
<211>  64
<212>  DNA
<213>  Artificial Sequence

<220>
<223>  shBRG1-CDS#2-sense

<400>  38
tttgttggaa gtacatgatt gtgggcttcc tgtcacccac aatcatgtac ttccaacttt      60

tttg                                                                    64


<210>  39
<211>  64
<212>  DNA
<213>  Artificial Sequence

<220>
<223>  shBRG1-CDS#2-antisense

<400>  39
aattcaaaaa agttggaagt acatgattgt gggtgacagg aagcccacaa tcatgtactt      60

ccaa                                                                    64


<210>  40
<211>  64
<212>  DNA
<213>  Artificial Sequence

<220>
<223>  shBRG1-CDS#4-sense

<400>  40
tttgcgtatc gcggctttaa atacgcttcc tgtcacgtat ttaaagccgc gatacgcttt      60

tttg                                                                    64


<210>  41

<211>  21
<212>  DNA
<213>  Artificial Sequence

<220>
<223>  shDPF1-CDS#1-sense

<400>  41
gacacccctc gtgtttacaa t                                                21


<210>  42
<211>  21
<212>  DNA
<213>  Artificial Sequence

<220>
<223>  shDPF1-CDS#1-antisense

<400>  42
attgtaaaca cgaggggtgt c                                                21


<210>  43
<211>  21
<212>  DNA
<213>  Artificial Sequence

<220>
<223>  shDPF1-CDS#2 sense

<400>  43
gaagaacagg gccaaaggaa a                                                21


<210>  44
<211>  21
<212>  DNA
<213>  Artificial Sequence

<220>
<223>  shDPF1-CDS#2 antisense

<400>  44
tttcctttgg ccctgttctt c                                                21


<210>  45
<211>  21
<212>  DNA
<213>  Artificial Sequence

<220>
<223>  shDPF1-CDS#3 sense

<400>  45
ggcacaaagg aaacacacag c                                                21


<210>  46
<211>  21
<212>  DNA
<213>  Artificial Sequence

<220>
<223>  shDPF1-CDS#3 antisense

<400>  46
gctgtgtgtt tcctttgtgc c                                                21


<210>  47
<211>  21
<212>  DNA
<213>  Artificial Sequence

<220>
<223>  shDPF1-3'UTR#1 sense

<400>  47
gcttctcccc tcaaagccat a                                                21


<210>  48
<211>  21
<212>  DNA
<213>  Artificial Sequence

<220>
<223>  shDPF1-3'UTR#1 antisense

<400>  48
tatggctttg aggggagaag c                                                21


<210>  49
<211>  21
<212>  DNA
<213>  Artificial Sequence

<220>
<223>  shDPF1-3'UTR#2-sense

<400>  49
gttaatatat acaaagagtc c                                                21


<210>  50
<211>  21
<212>  DNA
<213>  Artificial Sequence

<220>
<223>  shDPF1-3'UTR#2 antisense

<400>  50
ggactctttg tatatattaa c                                                21


<210>  51
<211>  21
<212>  DNA
<213>  Artificial Sequence

<220>
<223>  shDPF1-3'UTR#4 sense

<400>  51
gaattaactt gttctgtgta t                                                21


<210>  52
<211>  21
<212>  DNA
<213>  Artificial Sequence

<220>
<223>  shDPF1-3'UTR#4 antisense

<400>  52
atacacagaa caagttaatt c                                                21


<210>  53
<211>  21
<212>  DNA
<213>  Artificial Sequence

<220>
<223>  shDPF2-#1-sense

<400>  53
ggaacagtag cttcaccttg t                                                21


<210>  54
<211>  21
<212>  DNA
<213>  Artificial Sequence

<220>
<223>  shDPF2-#2-sense

<400>  54
gcatccatct tgcctccaat t                                                21


<210>  55
<211>  21
<212>  DNA
<213>  Artificial Sequence

<220>
<223>  shDPF2-3'UTR#3-sense

<400>  55
gtagcttcac cttgttattc c                                                21


<210>  56
<211>  21
<212>  DNA
<213>  Artificial Sequence

<220>
<223>  shDPF2-3'UTR#4 sense

<400>  56
gctcttaact gaattgggag c                                                21

<210>  57
<211>  21
<212>  DNA
<213>  Artificial Sequence

<220>
<223>  shDPF2-3'UTR#6-sense

<400>  57
ggtgatcaca gggttcaaac a                                                21


<210>  58
<211>  21
<212>  DNA
<213>  Artificial Sequence

<220>
<223>  shDPF3-CDS#1-sense

<400>  58
gtcattccca ataactactg t                                                21


<210>  59
<211>  21
<212>  DNA
<213>  Artificial Sequence

<220>
<223>  shDPF3-CDS#3-sense

<400>  59
ggaaagcatc caggaaatac a                                                21


<210>  60
<211>  21
<212>  DNA
<213>  Artificial Sequence

<220>
<223>  shDPF3a-3'UTR#2-sense

<400>  60
gcatcgtgat gcgctattta t                                                21


<210>  61
<211>  21
<212>  DNA
<213>  Artificial Sequence

<220>
<223>  shDPF3a-3'UTR#2 antisense

<400>  61
ataaatagcg catcacgatg c                                                21


<210>  62

<211>  21
<212>  DNA
<213>  Artificial Sequence

<220>
<223>  shDPF3a-3'UTR#3-sense

<400>  62
gttaagaaac gtatcaatac c                                                21


<210>  63
<211>  21
<212>  DNA
<213>  Artificial Sequence

<220>
<223>  shDPF3a-3'UTR#3 antisense

<400>  63
ggtattgata cgtttcttaa c                                                21


<210>  64
<211>  21
<212>  DNA
<213>  Artificial Sequence

<220>
<223>  shDPF3a-3'UTR#4-sense

<400>  64
gaaatcgaag caatatcctg t                                                21


<210>  65
<211>  21
<212>  DNA
<213>  Artificial Sequence

<220>
<223>  shDPF3a-3'UTR#4 antisense

<400>  65
acaggatatt gcttcgattt c                                                21


<210>  66
<211>  21
<212>  DNA
<213>  Artificial Sequence

<220>
<223>  shDPF3a-CDS#1-sense

<400>  66
gaaagtgaca cgtcaacttt c                                                21


<210>  67
<211>  21
<212>  DNA
<213>  Artificial Sequence

<220>
<223>  shDPF3a-CDS#1-antisense

<400>  67
gaaagttgac gtgtcacttt c                                                21


<210>  68
<211>  21
<212>  DNA
<213>  Artificial Sequence

<220>
<223>  shDPF3a-CDS#2 sense

<400>  68
gctttgatga ggacgatttg g                                                21


<210>  69
<211>  21
<212>  DNA
<213>  Artificial Sequence

<220>
<223>  shDPF3a-CDS#2-antisense

<400>  69
ccaaatcgtc ctcatcaaag c                                                21


<210>  70
<211>  21
<212>  DNA
<213>  Artificial Sequence

<220>
<223>  shDPF3b-CDS#3-sense

<400>  70
gtcacccaac ctgcctgcag t                                                21


<210>  71
<211>  21
<212>  DNA
<213>  Artificial Sequence

<220>
<223>  shDPF3b-CDS#4-sense

<400>  71
gctgtcaaga cctacaagtg g                                                21


<210>  72
<211>  21
<212>  DNA
<213>  Artificial Sequence

<220>
<223>  shDPF3b-CDS#6-sense

<400>  72
gggaactgct caaagagaaa g                                                21


<210>  73
<211>  21
<212>  DNA
<213>  Artificial Sequence

<220>
<223>  shDPF3b-CDS#7-sense

<400>  73
gatgaccagc tactcttctg c                                                21


<210>  74
<211>  2348
<212>  DNA
<213>  Homo sapiens

<300>
<308>  GenBank/NM_001135155.2
<309>  2016-02-12
<313>  (1)..(2348)

<400>  74
gtgctcccgc cccccgggaa tgaatggatg ggcggcctca gcgcccgccc gaccgctggg      60

aggaccgacc cggcggggac ctgctggggg caggacccgg ggagcaagat ggccactgtc     120

atccctggcc ccctgagcct aggcgaggac ttctaccgcg aggccatcga gcactgccgc     180

agttacaacg cgcgcctgtg cgccgagcgc agcctgcgac tgcccttcct cgactcgcag     240

accggcgtgg cccagaacaa ctgctacatc tggatggaga agacccaccg cgggccgggt     300

ttggccccgg gacagattta cacgtacccc gcccgctgtt ggaggaagaa acggagactc     360

aacatcctgg aggaccccag actcaggccc tgcgagtaca agatcgactg tgaagcaccc     420

ctgaagaagg agggtggcct cccggaaggg ccggtcctcg aggctctact gtgtgcagag     480

acgggggaga agaagattga gctgaaggag gaggagacca ttatggactg tcagaaacag     540

cagttgctgg agtttccgca tgacctcgag gtggaagact tggaggatga cattcccagg     600

aggaagaaca gggccaaagg aaaggcatat ggcatcgggg gtctccggaa acgccaggac     660

accgcttccc tggaggaccg agacaagccg tatgtctgtg atatctgtgg gaaacggtat     720

aagaaccggc cggggctcag ctaccactac acccacaccc acctggccga ggaggagggg     780

gaggagaacg ccgaacgcca cgccctgccc ttccaccgga aaaacaacca taaacagttt     840

tacaaagaat tggcctgggt ccctgaggca caaaggaaac acacagccaa gaaggcgccc     900

gacggcactg tcatccccaa cggctactgt gacttctgcc tggggggctc caagaagacg     960

gggtgtcccg aggacctcat ctcctgtgcg gactgtgggc gatcaggaca cccctcgtgt    1020

ttacaattca cggtgaacat gacggcagcc gtgcggacct accgctggca gtgcatcgag    1080

tgcaaatcct gcagcctgtg cggaacctcc gagaacgacg accagctgct gttttgtgat 1140

gactgcgatc ggggttacca catgtactgc ctgagtcccc ccatggcgga gcccccggaa 1200

gggagctgga gctgtcacct ctgtctccgg cacctgaagg aaaaggcttc tgcttacatc 1260

accctcacct aggccggctc ggctcgccgc gactctgggg tggtgctcgc ctacctgcct 1320

ctccgagctc ctcaattctc ccccaccctg aacatcccgc agggggaggg ggagaggggg 1380

aagccgagag ggggctgggc caccccctcc cctctgtgca agtggaatgt ctgccctgtg 1440

ggtgggtggg cccggccagg gcctctccct ccctccctcc ctctctgtcc cttggcaaat 1500

ggacaccagg ggcttctccc ctcaaagcca taccccgcct ctgggcgggc atggggggtg 1560

gtgggtgcca gccaggggca tggacagagc ctttttctaa agaaaaagac aaaaagttaa 1620

aaaaaaaaaa aagaagaaaa gaaaagaagt taatatatac aaagagtcct ccaaggcctg 1680

gctgggtgga ggggcgctgc tgagagtgtc caccgggcac ccgcctctgc cggccccccg 1740

ccgggcgccc caaccccaat ttctggagct gcagccgtcc cgcgccccac ccaaggtggg 1800

cgccttcccc tcttgtgccc agggcggtgg gcgtggtgtc cacccgcccc tcctggtgcc 1860

cacggtggat actgcatgat gtgaaccttg gttttgaact ctgttcctgc ccctccccga 1920

ccgccccagc ctgtgcccgc cccgtgcctg ccgtggctgg tgggtggcgg tggtggggcc 1980

gggtgggccc ccgcccagcg cctgctggaa tgagaagcac agactccgcc acggactcct 2040

tttctctccc tcctcccgcc ccgccaggcc tggcggcccc cgcccccctc gctggccatt 2100

ttgggggagt gagggggcgt ggttgtttct tgtggttgtg tgtgtttgtt gttcgggttt 2160

taaaaaaggg aaactgagac tgcaggtggg ggaggtggtg ggttttgggg ggatgtcccc 2220

taatccagga gtgccccctc acttgtcacc gagtctcctc tattgcctgc ctctgctgtg 2280

aattaacttg ttctgtgtat taaactgggc ctgacccctc tgcccacgaa aaaaaaaaaa 2340

aaaaaaa 2348

<210> 75
<211> 2545
<212> DNA
<213> Homo sapiens

<300>
<308> GenBank/NM_006268.4
<309> 2016-02-06
<313> (1)..(2545)

<400> 75
agtgctcgct ctagtgcgcg cgcccggacg gcgcctgcgc agagggcaag gaacctggta 60

ccccggtgcg gtcccggcgc ctgcgcgctg cggactgtgg ggcttctcgg cccgaggcag 120

aggaacaggg aagatggcgg ctgtggtgga gaatgtagtg aagctccttg gggagcagta 180

ctacaaagat gccatggagc agtgccacaa ttacaatgct cgcctctgtg ctgagcgcag 240
cgtgcgcctg cctttcttgg actcacagac cggagtagcc cagagcaatt gttacatctg 300
gatggaaaag cgacaccggg gtccaggatt ggcctccgga cagctgtact cctaccctgc 360
ccggcgctgg cggaaaaagc ggcgagccca tccccctgag gatccacgac tttccttccc 420
atctattaag ccagacacag accagaccct gaagaaggag gggctgatct ctcaggatgg 480
cagtagttta gaggctctgt tgcgcactga ccccctggag aagcgaggtg ccccggatcc 540
ccgagttgat gatgacagcc tgggcgagtt tcctgtgacc aacagtcgag cgcgaaagcg 600
gatcctagaa ccagatgact tcctggatga cctcgatgat gaagactatg aagaagatac 660
tcccaagcgt cggggaaagg ggaaatccaa gggtaagggt gtgggcagtg cccgtaagaa 720
gctggatgct tccatcctgg aggaccggga taagccctat gcctgtgaca tttgtggaaa 780
acgttacaag aaccgaccag gcctcagtta ccactatgcc cactcccact tggctgagga 840
ggagggcgag gacaaggaag actctcaacc acccactcct gtttcccaga ggtctgagga 900
gcagaaatcc aaaaagggtc ctgatggatt ggccttgccc aacaactact gtgacttctg 960
cctgggggac tcaaagatta acaagaagac gggacaaccc gaggagctgg tgtcctgttc 1020
tgactgtggc cgctcagggc atccatcttg cctccaattt acccccgtga tgatggcggc 1080
agtgaagaca taccgctggc agtgcatcga gtgcaaatgt tgcaatatct gcggcacctc 1140
cgagaatgac gaccagttgc tcttctgtga tgactgcgat cgtggctacc acatgtactg 1200
tctcaccccg tccatgtctg agccccctga aggaagttgg agctgccacc tgtgtctgga 1260
cctgttgaaa gagaaagctt ccatctacca gaaccagaac tcctcttgat gtggccaccc 1320
acctgctccc cgacatatct aaggctgttt ctctcctcca cttcatattt catacccatc 1380
tttcccttct tcctcctctc cttcacaaat ccagagaacc ttggggtggt tgtgccagcc 1440
tgcctttggc agctgcaagc tgaggtggca gctctgacca cctctggccc caggccctca 1500
gggagaaagg agcaacacac tgcccctagg cgtgcgtgtg gcccagtttc tctctgctct 1560
ccattaagtg cattcactct gcttgccttg ggcccagccc ctggtgatca cagggttcaa 1620
acagtgtcct cctagaaaga gtgggagagc agctcacttc tctgtgttct gcctcccctc 1680
tggtctccag agttttcctg tcctctagag gcaagccagg ccagggagct gggagcgagc 1740
aagctgaggc cacgtccaca aggagctttt catgcccctg tgccgcatag cctcacctct 1800
ttcctccaga gtggctctct gcggccctgt gttcctgcta cagagtgttc ttttctggag 1860
tcaggatgtt ctcggtcacc ctcctggttc tgccctgtcc cattccaccc caccccaggg 1920
ggaacagtag cttcaccttg ttattcccat tgctctcctg gctcactctt acggtcggtc 1980
tccagtgact gaagcattcc ccacccttgg aatttctcat cttctgcctc ccttcctact 2040
ccttttggtt ttgtggggag aggggaagga tcagggggcc aggccagcag ctcgggggcc 2100

acaaggagat ggataatgtg cctgtttttt aacacaacaa aaaagcctac ctccaaaatc 2160

ccctttttgt tcttcctgga cctgggcatt cagcctcctg ctcttaactg aattgggagc 2220

ctctgccacc tgccccgtgt atcctggctc tcagctcatg gggaagccac atagacatcc 2280

ctttcttccc ttgcacgctc gctagcagct ggtaaggtct tcacaccctg attcctcaag 2340

ttttctgctt agtggcactg acattaagta gtggggggac agtccatgcc aggacaccct 2400

ggagtagcct tcccccttgg ccgtgggcag gccctaactc actgtcgctt tggagttgag 2460

gtgtcttttt tttttctttc tttagttcct gtattctaaa cattagtaaa aataaatgtt 2520

tttacacaga aaaaaaaaa aaaaa 2545

<210> 76
<211> 4077
<212> DNA
<213> Homo sapiens

<300>
<308> GenBank/NM_012074.4
<309> 2016-02-12
<313> (1)..(4077)

<400> 76
agacaatatt ctgttacatt gtagcaaaat ggcgactgtc attcacaacc ccctgaaagc 60

gctcggggac cagttctaca aggaagccat tgagcactgc cggagttaca actcacggct 120

gtgtgcagag cgcagcgtgc gtcttccctt cctggactca cagactgggg tggcccagaa 180

caactgctac atctggatgg agaagaggca ccgaggccca ggccttgccc cgggccagct 240

gtatacatac cctgcccgct gctggcgcaa gaagagacga ttgcacccac ctgaagatcc 300

aaaactgcgg ctgctggaga taaaacctga agtggagctt cccctgaaga aggatgggtt 360

cacctcagag agcaccacgc tggaagcctt gctccgtggc gagggggttg agaagaaggt 420

ggatgccagg gaggaggaaa gcatccagga aatacagagg gttttggaaa atgatgaaaa 480

tgtagaagaa gggaatgaag aagaggattt ggaagaggat attcccaagc gaaagaacag 540

gactagagga cgggctcgcg gctctgcagg gggcaggagg aggcacgacg ccgcctctca 600

ggaagaccac gacaaacctt acgtctgtga catctgtggc aagcgctaca agaaccgacc 660

ggggctcagc taccactatg ctcacactca cctggccagc gaggaggggg atgaagctca 720

agaccaggag actcggtccc cacccaacca cagaaatgag aaccacaggc cccagaaagg 780

accggatgga acagtcattc ccaataacta ctgtgacttc tgcttggggg gctccaacat 840

gaacaagaag agtgggcggc ctgaagagct ggtgtcctgc gcagactgtg gacgctctgc 900

tcatttggga ggagaaggca ggaaggagaa ggaggcagcg gccgcagcac gtaccacgga 960

ggacttattc ggttccacgt cagaaagtga cacgtcaact ttccacggct ttgatgagga 1020

cgatttggaa gagcctcgct cctgtcgagg acgccgcagt ggccggggtt cgcccacagc 1080

agataaaaag ggcagttgct aaacccacgg aacagactct ctgggcaatt agccatcccc 1140

ctctgacttt ggtcattgtg ctggttctga tatatatttt ttttaatgaa aggcaacttt 1200

agattttccc tctatccttg ctttttttcc cttcacctcc cacgtgtccc tccatccctc 1260

cccccacccc tctgttttgg gtatgtacaa cagaagcaca aactactgaa acaaaacaaa 1320

acagcagaat gagcgttctt ccgagagatg gcatcgtgat gcgctattta ttttccatag 1380

aaataggaag ttagacggat tgtctctttt ctgaggggag gggtcttttt tgacaggagc 1440

agagttgatg tcctcaattt tcatatttat tggcaaaagg aagagaagag gaactttggg 1500

ttggaaacaa agaaccaata acattaaaac attattattt atatattcta gctgttatta 1560

gaatcagact ttttttgcga gagagagaga gagagagaga gaagggaaat caaagaaatc 1620

gaagcaatat cctgtttaga ggcaagccgc ccggtgggga gaatttcctc aatgggagac 1680

ggttgcacta ttctgtgccc cacggagttt gcggctcccc gcggcagacc cctccctcat 1740

tctcctccct gacctttcca tcttcctctc tgcttgcgag aaaatgtcag tagttccaga 1800

gaagtcgggg tgcctatgcc tggcctccct ccacacctgg gccctgacca gccgcctcct 1860

gggctcctcc tcctccgtca gtagagctgc tgttttgtta ttgctggttt ttcctcactt 1920

tcctcctggc aaagaacgac ttccaaatgc agggatggaa tataagcaga acgtcatggg 1980

ctcagcagtg actccaccac ccgaggccga ggccgtgctt ctggaagata gaaggagaca 2040

tcatcgtgtg tttcccctcc ccttgcccct gttaagaaac gtatcaatac ccattggatg 2100

atcaaggcta ccgtatttct tctatttttt tttatagtgc ctgccaggca ctttgtttta 2160

tgtttccaat agcacttcct gaaataaacc aaagcaacac tgctcaaggc ccctggggcg 2220

atggagaagg ccacccacct cactgacagt cccaagaatg accggctgcg aggtcctagt 2280

caaaagtcaa cattatgacc tggggactcc agcatccttc aagcaagcca tttccgaaga 2340

aggtgaaaag aagccaggat gattggcacc tcctcctcct cctcctcttc ttcctcttcc 2400

cttgcccagc cccctcctgt gcgtgtgttt cagacaacac aggagccagc acaggagtgg 2460

aaaatcctgc agcgcaactc agctcagccc acagaagcct tgggaatggc ctcagtttgt 2520

gcaataagaa gatttttttt ttctttttaa atcttcatta tattttcttt gattgtctgt 2580

gagaaagtac ccaggtccgc ctggaattac tctacagtag aaataactga acacaaacaa 2640

actgatggaa aaaaagagtt aactatttta tttatttcaa tatttaaaag gaaaaaagtg 2700

ctgacatggc acagtatttt tgtttaaagt acctcctact tcaaaagtta agcgcaattt 2760

tgtgaagaca tgaaatcata agagtactta atgtaaaata aaagactgca tattaactct 2820

aaagaaaaat gccccacatt ttaaataaga aaataaagat caactctgct ctctcaggct 2880

ttttaaaaag ccattcatgt atgtgcttta ggtattttta tttctgcgag ttggatgtgg 2940

taagtgagga gtgctcagtt tttttttcct ccttcaaaag tctattgaaa gtgttggtga 3000

tgttaaatga ttgtgtgtta agatttgact gaaataactt agccacaaat cagcagtttc 3060

ccccaccctc attgccccct caccccaggc aagccccttt tatctgaatg tcagaagcag 3120

cctgcctcct agttatcatg tctgatgagg tctagctcag gaaggaattc catctattga 3180

tggaatatat cccctcaagt tcaatagatt cgaacacaga gagctttgtt taaaataatg 3240

cagcaaaaaa aaaaaaaaaa aaaaagcaaa aataaaagca tcagctgagg tgatattagt 3300

tcagtcacct aacaactcct agaagagatg aggaaaggga accttctgct gagctggctt 3360

ctggggcctg agcttccaga gctgtcccca agggctagga aggccgacct gaaggatgag 3420

aacctcaaat tcagttgctg gtgggagcca aggaagacgg cgggtgttct aacatggccc 3480

tttctggctg agctggcgga agtgggcgtt ttggccgatg ggatgtatct cggcgctgtg 3540

tctgtggccc agcaaaggtg cagggctgac tggctgagcc actgggttct acccgcaggc 3600

tccccactgc actgggcttt cacacagcca tgctcttggg tttccctccc ttgtaagcag 3660

agtcataata acacacgaat agtctaaggc tgggtattct ggtcagcaga ggtccttgag 3720

tcacagtgtt actgaaatgg ttctgagcct gagaatctct ttggcctctg aaagggcagg 3780

gcaggtgggc accgacttcc tgccagtcct ttcaggtttc ctgttcaaag ccagtcctgt 3840

tggtggaggg gatcaccgag agtgtctgta tcattttgta gccctttctc ctgacgtttt 3900

ctggtagaaa atgtcccttg tcaaaatgct aataattatc ataataatct gctttccaac 3960

caactcccac aagtgacaac ctgtgtagaa ctgtgataaa ggtttgcata atgtagggtt 4020

tgtaccaagt gtgtgtaagt ttctgttaaa taaaaagtct gtttccaatg ctcctat 4077

<210> 77
<211> 1166
<212> DNA
<213> Homo sapiens

<300>
<308> GenBank/NM_001280542.1
<309> 2016-06-12
<313> (1)..(1166)

<400> 77
agacaatatt ctgttacatt gtagcaaaat ggcgactgtc attcacaacc ccctgaaagc 60

gctcggggac cagttctaca aggaagccat tgagcactgc cggagttaca actcacggct 120

gtgtgcagag cgcagcgtgc gtcttccctt cctggactca cagactgggg tggcccagaa 180

caactgctac atctggatgg agaagaggca ccgaggccca ggccttgccc cgggccagct 240

gtatacatac cctgcccgct gctggcgcaa gaagagacga ttgcacccac ctgaagatcc 300

aaaactgcgg ctgctggaga taaaacctga agtggagctt cccctgaaga aggatgggtt 360

cacctcagag agcaccacgc tggaagcctt gctccgtggc gagggggttg agaagaaggt 420

ggatgccagg gaggaggaaa gcatccagga aatacagagg gttttggaaa atgatgaaaa 480

tgtagaagaa gggaatgaag aagaggattt ggaagaggat attcccaagc gaaagaacag 540

gactagagga cgggctcgcg gctctgcagg gggcaggagg aggcacgacg ccgcctctca 600

ggaagaccac gacaaacctt acgtctgtga catctgtggc aagcgctaca agaaccgacc 660

ggggctcagc taccactatg ctcacactca cctggccagc gaggaggggg atgaagctca 720

agaccaggag actcggtccc cacccaacca cagaaatgag aaccacaggc cccagaaagg 780

accggatgga acagtcattc ccaataacta ctgtgacttc tgcttggggg gctccaacat 840

gaacaagaag agtgggcggc ctgaagagct ggtgtcctgc gcagactgtg gacgctctgg 900

tcacccaacc tgcctgcagt ttaccctgaa catgaccgag gctgtcaaga cctacaagtg 960

gcagtgcata gagtgcaaat cctgtatcct ctgtgggacc tcagagaatg atgaccagct 1020

actcttctgc gatgactgtg accgaggcta tcacatgtac tgtttaaatc ccccggtggc 1080

tgagccccca gaaggaagct ggagctgcca cttatgctgg gaactgctca aagagaaagc 1140

ctcagccttt ggctgccagg cctagg 1166

<210> 78
<211> 24
<212> PRT
<213> Artificial Sequence

<220>
<223> DPF3-CT1 53-76aar

<400> 78

Cys Tyr Ile Trp Met Glu Lys Arg His Arg Gly Pro Gly Leu Ala Pro
1               5                   10                  15

Gly Gln Leu Tyr Thr Tyr Pro Ala
            20

<210> 79
<211> 36
<212> PRT
<213> Artificial Sequence

<220>
<223> DPF3-CT1 40-76aar

<400> 79

Phe Leu Asp Ser Gln Thr Gly Val Ala Gln Asn Asn Cys Tyr Ile Trp
1               5                   10                  15

Met Glu Lys Arg His Arg Gly Pro Gly Leu Ala Pro Gly Gln Leu Tyr
            20                  25                  30

Thr Tyr Pro Ala
        35